(19) Europäisches Patentamt
European Patent Office
Office européen des brevets

(11) EP 4 736 861 A1

(12) **EUROPEAN PATENT APPLICATION**
published in accordance with Art. 153(4) EPC

(43) Date of publication:
**06.05.2026 Bulletin 2026/19**

(21) Application number: **24832008.7**

(22) Date of filing: **26.06.2024**

(51) International Patent Classification (IPC):
***A61K 33/06*** (2006.01) ***A61P 1/04*** (2006.01)
***A61P 1/16*** (2006.01) ***A61P 3/04*** (2006.01)
***A61P 3/10*** (2006.01) ***A61P 9/00*** (2006.01)
***A61P 9/04*** (2006.01) ***A61P 13/12*** (2006.01)
***A61P 15/00*** (2006.01) ***A61P 25/16*** (2006.01)
***A61P 25/28*** (2006.01) ***A61P 27/06*** (2006.01)
***A61P 35/00*** (2006.01)

(52) Cooperative Patent Classification (CPC):
**A61K 33/06; A61P 1/04; A61P 1/16; A61P 3/04; A61P 3/10; A61P 9/00; A61P 9/04; A61P 13/12; A61P 15/00; A61P 25/16; A61P 25/28; A61P 27/06; A61P 35/00**

(86) International application number:
**PCT/JP2024/023237**

(87) International publication number:
**WO 2025/005149 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.06.2023 JP 2023105129**

(71) Applicants:
- **Tanabe Pharma Corporation**
  **Osaka-shi, Osaka 541-8505 (JP)**
- **Mitsubishi Chemical Corporation**
  **Tokyo 100-8251 (JP)**

(72) Inventors:
- **KOHYAMA, Yuh**
  **Osaka-shi, Osaka 541-8505 (JP)**
- **MATSUMOTO, Takumi**
  **Osaka-shi, Osaka 541-8505 (JP)**
- **KODAIRA, Ayako**
  **Osaka-shi, Osaka 541-8505 (JP)**
- **TOGO, Yuka**
  **Osaka-shi, Osaka 541-8505 (JP)**
- **HIKIMA, Shu**
  **Tokyo 100-8251 (JP)**
- **OHNISHI, Ryohji**
  **Tokyo 100-8251 (JP)**
- **TAKEWAKI, Takahiko**
  **Tokyo 100-8251 (JP)**
- **KATAGIRI, Noriko**
  **Tokyo 100-8251 (JP)**

(74) Representative: **Ter Meer Steinmeister & Partner Patentanwälte mbB**
**Nymphenburger Straße 4**
**80335 München (DE)**

# (54) ORAL COMPOSITION CONTAINING INORGANIC POROUS BODY

(57) The oral composition of the present disclosure includes an inorganic porous material, and with respect to choline and a primary, secondary, or tertiary amine or ammonia present as an equimolar mixture in simulated intestinal fluid, when 11.8 g of the inorganic porous material per 1 mmol of choline is mixed at a concentration of 2 g/L for 1 hour, the adsorption ratio of the primary, secondary, or tertiary amine or ammonia is 60.0% or more, and the adsorption ratio of choline is 40.0% or less. Alternatively, the oral composition of the present disclosure includes a zeolite, wherein the zeolite has an MFI structure and a molar ratio of $SiO_2$ to $Al_2O_3$ of 30.0 or more and 130,000.0 or less, or the zeolite has a FER structure and a molar ratio of $SiO_2$ to $Al_2O_3$ of 15.0 or more and 130,000.0 or less.

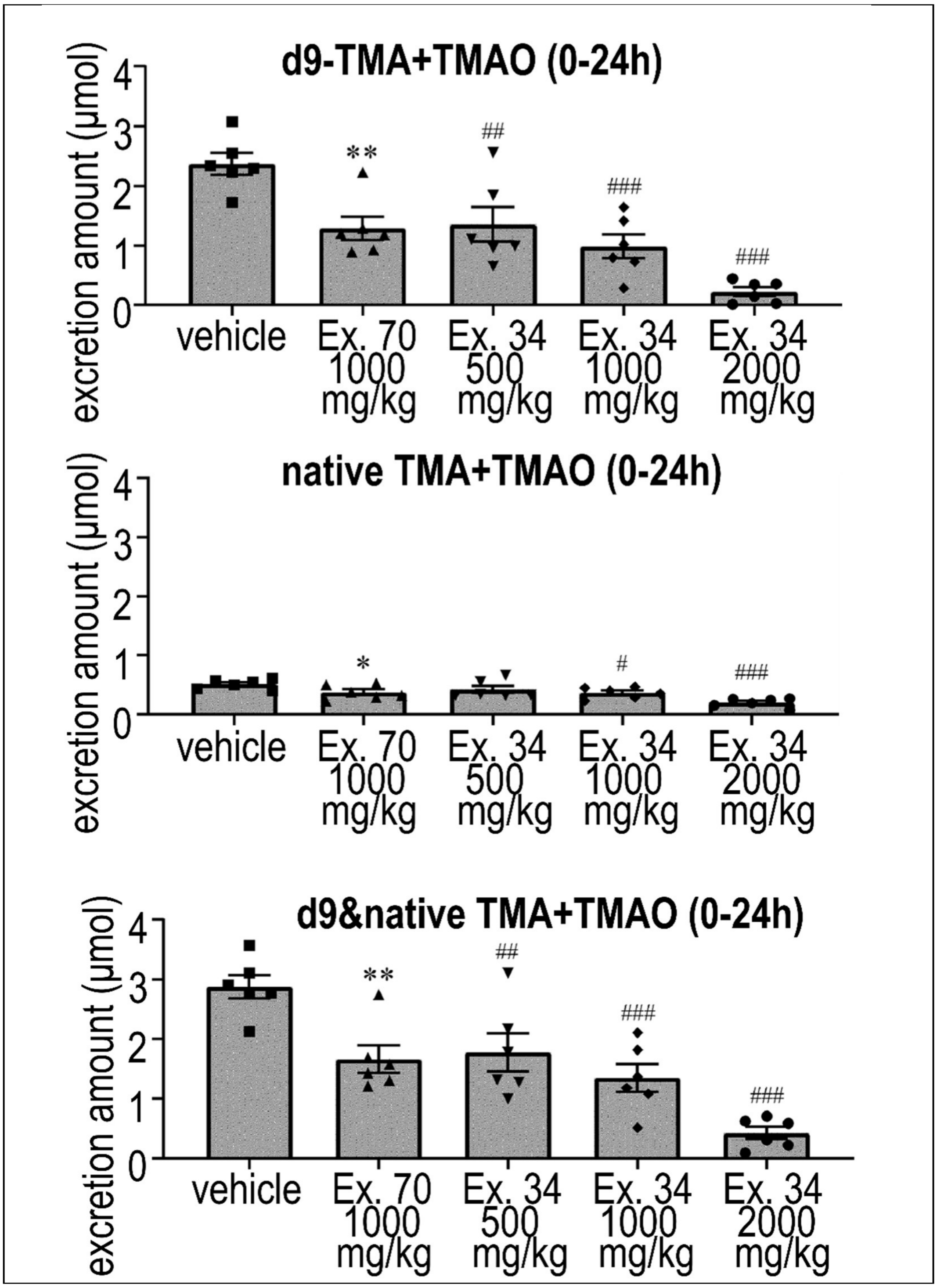
d9-TMA+TMAO (0-24h)
excretion amount (µmol)
0
1
2
3
4
**
##
###
###
vehicle
Ex. 70 1000 mg/kg
Ex. 34 500 mg/kg
Ex. 34 1000 mg/kg
Ex. 34 2000 mg/kg
native TMA+TMAO (0-24h)
excretion amount (µmol)
*
#
###
d9&native TMA+TMAO (0-24h)
excretion amount (µmol)
**
##
###
###

Fig. 13

## Description

### TECHNICAL FIELD

The present disclosure relates to an oral composition containing an inorganic porous material, which is formulated to selectively adsorb primary, secondary, or tertiary amines or ammonia over choline.

### BACKGROUND ART

Some primary, secondary, and tertiary amines or ammonia exist as toxins in the body, and their excessive presence can cause various diseases.

For example, in trimethylaminuria (fish odor syndrome), due to a genetic defect in the trimethylamine-metabolizing enzyme FMO3 (flavin-containing monooxygenase 3) or similar conditions, the concentration of trimethylamine (TMA) in bodily fluids becomes higher than in healthy individuals. The volatile component, trimethylamine, is excreted in sweat, breath, urine, and other bodily fluids, causing a body odor like rotting fish, which diminishes the quality of life and social activities of patients with trimethylaminuria, and can also lead to psychiatric symptoms such as depression. There is no fundamental cure, and symptomatic treatments are mainly performed, such as dietary restrictions of foods rich in trimethylamine precursors like choline, lecithin (phosphatidylcholine), carnitine, betaine (N,N,N-trimethylglycine), and trimethylamine-N-oxide (TMAO), and washing the body with acidic soap that can efficiently wash away the basic substance trimethylamine.

Furthermore, TMAO, a metabolite of trimethylamine, has been reported to be a pathogenic molecule of atherosclerosis. In addition, its involvement has been reported in coronary artery heart disease, heart failure with preserved ejection fraction, ST-elevation myocardial infarction, atrial fibrillation, abdominal aortic aneurysm, ischemic stroke, post-stroke cognitive impairment, mild cognitive impairment, Alzheimer's disease, obesity, progressive chronic kidney disease with type 2 diabetes, cardiovascular complications in chronic kidney disease, diabetic retinopathy, non-alcoholic steatohepatitis, polycystic ovary syndrome, Parkinson's disease, and colorectal cancer.

There are also reports that histamine excessively produced by intestinal bacteria increases histamine concentration in the gastrointestinal tract, activating H4 receptors and inducing abdominal pain in patients with irritable bowel syndrome.

Moreover, when detoxification of ammonia to urea in the liver becomes insufficient due to liver failure, cirrhosis, portosystemic shunt, urea cycle disorders, organic acidemia, etc., an excess of ammonia in the circulating blood, i.e., hyperammonemia, occurs, leading to hepatic encephalopathy.

As in the examples above, it is thought that detoxification of a poisoned state or treatment of a disease can be achieved by adsorbing and removing excess primary, secondary, or tertiary amines or ammonia that act as toxins or pathogenic substances. However, there is a problem of selectivity against choline, which is a basic organic molecule with similar properties and also a nutrient. Choline is an essential nutrient for the body, especially indispensable for growth and development. Choline deficiency has been reported to cause various diseases.

Materials (adsorbents) for adsorbing and removing basic compounds from bodily fluids, and more broadly from water, include activated carbon, cation exchange resins, clay minerals, inorganic porous bodies, and metal organic frameworks (MOF). Choline, primary, secondary, tertiary amines, and ammonia are all basic compounds. When they exist as the same cationic species in water, it is difficult to distinguish and selectively adsorb primary, secondary, or tertiary amines or ammonia over choline, and no such adsorbent is known.

Patent Document 1 discloses the treatment of trimethylaminuria using zeolite.

Some primary, secondary, tertiary amines, ammonia, or their metabolites exist as toxins in the body, and their excessive presence causes various diseases. It is believed that by adsorbing and removing excess primary, secondary, or tertiary amines or ammonia that act as toxins or pathogenic substances, it could be used for the treatment of a poisoned state or disease. However, the simultaneous removal of choline, a molecule with similar properties that is also a nutrient, becomes a problem in terms of the adsorbent's toxicity. Therefore, there is a need to selectively adsorb and remove primary, secondary, or tertiary amines or ammonia over choline. Patent Document 1 does not disclose any specific examples of adsorbing trimethylamine using zeolite, nor does it disclose the selective adsorption of trimethylamine over choline by zeolite or the specific characteristics of zeolite for that purpose, and it does not focus on the aforementioned adsorption selectivity at all.

### PRIOR ART DOCUMENTS

### PATENT DOCUMENTS

Patent Document 1: U.S. Patent Application Publication No. 2018/0200291

SUMMARY OF INVENTION

TECHNICAL PROBLEM

The present disclosure has been made in view of the above circumstances, and an object thereof is to provide an oral composition that has good safety and can selectively adsorb and remove primary, secondary, tertiary amines, ammonia, or their metabolites.

SOLUTION TO PROBLEM

As a result of intensive studies on adsorbents to solve the above problems, the inventors have found an oral composition containing an inorganic porous material that selectively adsorbs primary, secondary, or tertiary amines or ammonia over choline.
The present disclosure has been completed based on the above findings.
That is, the gist of the present disclosure includes the following.
[1] An oral composition comprising;

an inorganic porous material,
wherein the oral composition has an adsorption ratio of primary, secondary, or tertiary amine or ammonia of 60.0% or more, and has an adsorption ratio of choline of 40.0% or less, when each adsorption ratio is measured by the following method:
to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.

[1-1] The oral composition according to [1], wherein the oral composition has the adsorption ratio of the primary, secondary, or tertiary amine or ammonia of 70.0% or more, and has the adsorption ratio of choline of 40.0% or less, when each adsorption ratio is measured by the following method:
to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each adsorption ratio is measured.
[1-2] The oral composition according to [1] or [1-1], wherein the oral composition has the adsorption ratio of the primary, secondary, or tertiary amine or ammonia of 80.0% or more, and has the adsorption ratio of choline of 40.0% or less, when each adsorption ratio is measured by the following method:
to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.
[1-3] The oral composition according to any one of [1] to [1-2] wherein the oral composition has the adsorption ratio of the primary, secondary, or tertiary amine or ammonia of 90.0% or more, and has the adsorption ratio of choline of 40.0% or less, when each adsorption ratio is measured by the following method:
to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.
[1-4] The oral composition according to [1] or [1-1] wherein the oral composition has the adsorption ratio of the primary, secondary, or tertiary amine or ammonia of 70.0% or more, and has the adsorption ratio of choline of 30.0% or less, when each adsorption ratio is measured by the following method:
to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.
[1-5] The oral composition according to any one of [1] to [1-2], wherein the oral composition has the adsorption ratio of the primary, secondary, or tertiary amine or ammonia of 80.0% or more, and has the adsorption ratio of choline of 20.0% or less, when each adsorption ratio is measured by the following method:

to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.

[1-6] The oral composition according to any one of [1] to [1-3], wherein the oral composition has the adsorption ratio of the primary, secondary, or tertiary amine or ammonia of 90.0% or more, and has the adsorption ratio of choline of 10.0% or less, when each adsorption ratio is measured by the following method:

to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.

[2] The oral composition according to any one of [1] to [1-6], wherein the adsorption ratio of the primary, secondary, or tertiary amine or ammonia to the inorganic porous material is 2.00 times or more than the adsorption ratio of choline.

[2-1] The oral composition according to [2], wherein the adsorption ratio of the primary, secondary, or tertiary amine or ammonia to the inorganic porous material is 2.50 times or more than the adsorption ratio of choline.

[3] The oral composition according to any one of [1] to [2-1], wherein the inorganic porous material is an aluminosilicate or a silicate.

[4] The oral composition according to [3], wherein the aluminosilicate is a zeolite.

[5] The oral composition according to [4], wherein the zeolite has a maximum ring number of 10 and a largest free sphere diameter of 3.68 Å or more.

[5-1] The oral composition according to [5], wherein the zeolite has a maximum ring number of 10 and a largest free sphere diameter of 3.70 Å or more.

[5-2] The oral composition according to [5] or [5-1], wherein the zeolite has a maximum ring number of 10 and a largest free sphere diameter of 3.75 Å or more.

[5-3] The oral composition according to any one of [5] to [5-2], wherein the zeolite has a maximum ring number of 10 and a largest free sphere diameter of 4.22 Å or more.

[5-4] The oral composition according to any one of [5] to [5-3], wherein the zeolite has a maximum ring number of 10 and a largest free sphere diameter of 4.50 Å or more.

[5-5] The oral composition according to any one of [5] to [5-4], wherein the zeolite has a maximum ring number of 10 and a largest free sphere diameter of 4.65 Å or more.

[5-6] The oral composition according to any one of [5] to [5-5], wherein the zeolite has a maximum ring number of 10 and a largest free sphere diameter of 4.69 Å or more.

[5-7] The oral composition according to any one of [5] to [5-6], wherein the zeolite has a maximum ring number of 10 and a largest free sphere diameter of 4.91 Å or less.

[5-8] The oral composition according to any one of [5] to [5-7], wherein the zeolite has a maximum ring number of 10 and a largest free sphere diameter of 4.90 Å or less.

[5-9] The oral composition according to any one of [5] to [5-8], wherein the zeolite has a maximum ring number of 10 and a largest free sphere diameter of 4.85 Å or less.

[5-10] The oral composition according to any one of [5] to [5-9], wherein the zeolite has a maximum ring number of 10 and a largest free sphere diameter of 4.80 Å or less.

[5-11] The oral composition according to any one of [5] to [5-10], wherein the zeolite has a maximum ring number of 10 and a largest free sphere diameter of 4.75 Å or less.

[5-12] The oral composition according to any one of [5] to [5-11], wherein the zeolite has a maximum ring number of 10 and a largest free sphere diameter of 4.73 Å or less.

[5-13] The oral composition according to any one of [5] to [5-12], wherein the zeolite has a maximum ring number of 10 and a largest free sphere diameter of 4.71 Å or less.

[6] The oral composition according to any one of [4] to [5-13], wherein the zeolite has an MFI structure or a FER structure.

[6-1] The oral composition according to [6], wherein the zeolite has an MFI structure.

[7] The oral composition according to any one of [4] to [6-1], wherein the zeolite has a molar ratio of $SiO_2$ to $Al_2O_3$ of 15.0 or more and 130,000.0 or less.

[7-1] The oral composition according to [7], wherein the zeolite has a molar ratio of $SiO_2$ to $Al_2O_3$ of 18.0 or more and 129,000.0 or less.

[7-2] The oral composition according to [7] or [7-1], wherein the zeolite has a molar ratio of $SiO_2$ to $Al_2O_3$ of 30.0 or more and 129,000.0 or less.

[8] The oral composition according to any one of [7] to [7-2], wherein the zeolite has a molar ratio of $SiO_2$ to $Al_2O_3$ of 30.0 or more and 80.0 or less.

[9] The oral composition according to any one of [4] to [8], wherein the external surface area of the zeolite is less than

33.0 $m^2$/g.

[9-1] The oral composition according to any one of [4] to [9], wherein the external surface area of the zeolite is less than 30.0 $m^2$/g.

[9-2] The oral composition according to any one of [4] to [9-1], wherein the external surface area of the zeolite is less than 26.0 $m^2$/g.

[9-3] The oral composition according to any one of [4] to [9-2], wherein the external surface area of the zeolite is 20.0 $m^2$/g or less.

[9-4] The oral composition according to any one of [4] to [9-3], wherein the external surface area of the zeolite is 15.0 $m^2$/g or less.

[9-5] The oral composition according to any one of [4] to [9-4], wherein the external surface area of the zeolite is 13.3 $m^2$/g or less.

[9-6] The oral composition according to any one of [4] to [9-5], wherein the zeolite has an MFI structure, and in an X-ray powder diffraction pattern measured with Cu-Kα radiation, the full width at half maximum of the largest peak existing in the range of $2\theta$ = 7.9000° ± 0.5000° is 0.0100° or more and 0.1800° or less.

[9-7] The oral composition according to any one of [4] to [9-6], wherein the zeolite has an MFI structure, and in an X-ray powder diffraction pattern measured with Cu-Kα radiation, the full width at half maximum of the largest peak existing in the range of 20 = 7.9000° ± 0.5000° is 0.0100° or more and 0.1500° or less.

[9-8] The oral composition according to any one of [4] to [9-7], wherein the zeolite has an MFI structure, and in an X-ray powder diffraction pattern measured with Cu-Kα radiation, the full width at half maximum of the largest peak existing in the range of 20 = 7.9000° ± 0.5000° is 0.0100° or more and 0.1300° or less.

[9-9] The oral composition according to any one of [4] to [9-8], wherein the median diameter of the zeolite is 5.5 μm or more.

[9-10] The oral composition according to any one of [4] to [9-9], wherein the median diameter of the zeolite is 10.0 μm or more.

[9-11] The oral composition according to any one of [4] to [9-10], wherein the median diameter of the zeolite is 11.7 μm or more.

[9-12] The oral composition according to any one of [4] to [9-11], wherein the D10 of the zeolite is 2.5 μm or more.

[9-13] The oral composition according to any one of [4] to [9-12], wherein the D10 of the zeolite is 5.0 μm or more.

[9-14] The oral composition according to any one of [4] to [9-13], wherein the D10 of the zeolite is 5.3 μm or more.

[9-15] The oral composition according to any one of [4] to [9-13], wherein the crystal shape of the zeolite is coffin-shaped.

[9-16] The oral composition according to any one of [1] to [9-15], wherein the oral composition is a pharmaceutical composition, a supplement, a food, or a food additive.

[10] The oral composition according to any one of [1] to [9-16], wherein the oral composition is a pharmaceutical composition.

[11] The oral composition according to [10], for use in preventing, treating, or alleviating symptoms of a disease caused by a primary, secondary, or tertiary amine, ammonia, or a metabolite thereof.

[12] The oral composition according to [11], wherein the primary, secondary, or tertiary amine, ammonia, or the metabolite comprises one or more selected from the group consisting of trimethylamine, dimethylamine, methylamine, histamine, ammonia, and trimethylamine-N-oxide.

[13] The oral composition according to [12], wherein the primary, secondary, or tertiary amine, ammonia, or the metabolite is trimethylamine or trimethylamine-N-oxide.

[14] The oral composition according to any one of [11] to [13], wherein the disease caused by a primary, secondary, or tertiary amine, ammonia, or the metabolite is selected from the group consisting of trimethylaminuria, cardiovascular disease, glaucoma, atherosclerosis, coronary artery heart disease, heart failure with preserved ejection fraction, ST-elevation myocardial infarction, atrial fibrillation, abdominal aortic aneurysm, ischemic stroke, post-stroke cognitive impairment, mild cognitive impairment, Alzheimer's disease, obesity, progressive chronic kidney disease with type 2 diabetes, cardiovascular complications in chronic kidney disease, diabetic retinopathy, non-alcoholic steatohepatitis, polycystic ovary syndrome, Parkinson's disease, colorectal cancer, irritable bowel syndrome, hyperammonemia, urea cycle disorder, organic acidemia, hepatic encephalopathy, and portosystemic shunt.

[15] The oral composition according to [14], wherein the disease is caused by trimethylamine, and the disease is trimethylaminuria, cardiovascular disease, or glaucoma.

[15-1] The oral composition according to [15], wherein the disease is caused by trimethylamine, and the disease is trimethylaminuria.

[16] The oral composition according to [14], wherein the disease is caused by trimethylamine-N-oxide, and the disease is atherosclerosis, coronary artery heart disease, heart failure with preserved ejection fraction, ST-elevation myocardial infarction, atrial fibrillation, abdominal aortic aneurysm, ischemic stroke, post-stroke cognitive impairment, mild cognitive impairment, Alzheimer's disease, obesity, progressive chronic kidney disease with type 2 diabetes, cardiovascular complications in chronic kidney disease, diabetic retinopathy, non-alcoholic steatohepatitis, polycystic ovary syndrome, Parkinson's disease, or colorectal cancer.

[16-1] The oral composition according to [16], wherein the disease is caused by trimethylamine-N-oxide, and the disease is atherosclerosis.

[17] The oral composition according to [14], wherein the disease is caused by histamine, and the disease is irritable bowel syndrome.

[18] The oral composition according to [14], wherein the disease is caused by ammonia, and the disease is hyperammonemia, urea cycle disorder, organic acidemia, hepatic encephalopathy, or portosystemic shunt.

[19] The oral composition according to any one of [10] to [18], wherein the oral composition comprises zeolite as an active ingredient, is administered 1 to 5 times per day, and the dose of the zeolite is 200 mg to 42,000 mg per administration.

[20] The oral composition according to [19], wherein the dose of the zeolite is 500 mg to 2000 mg per administration.

[21] A method for producing the oral composition according to any one of [4] to

[20], comprising obtaining the zeolite by hydrothermal synthesis without using an organic structure-directing agent.

[22] The method according to [21], wherein sodium carbonate and/or sodium sulfate is used as a reagent.

[23] The method according to [21] or [22], comprising converting the zeolite to a proton form with an acid.

[24] The method according to [23], wherein the acid is sulfuric acid or nitric acid.

[25] The method according to any one of [20] to [24], further comprising performing a surface treatment on the zeolite after the hydrothermal synthesis in a solution containing a Si element source.

[26] An oral composition comprising;

a zeolite,
wherein the zeolite has an MFI structure and a molar ratio of $SiO_2$ to $Al_2O_3$ of 30.0 or more and 130,000.0 or less, or
the zeolite has a FER structure and a molar ratio of $SiO_2$ to $Al_2O_3$ of 15.0 or more and 130,000.0 or less,
for use in the treatment, prevention, or symptom alleviation of a disease selected from the group consisting of trimethylaminuria, atherosclerosis, irritable bowel syndrome, and hyperammonemia.

[26-1] The oral composition according to [26], wherein the zeolite has an MFI structure and a molar ratio of $SiO_2$ to $Al_2O_3$ of 30.0 or more and 80.0 or less, and
the zeolite has a FER structure and a molar ratio of $SiO_2$ to $Al_2O_3$ of 15.0 or more and 130,000.0 or less.

[27] The oral composition according to [26] or [26-1], wherein the zeolite has a molar ratio of $SiO_2$ to $Al_2O_3$ of 30.0 or more and 80.0 or less.

[28] The oral composition according to any one of [26] to [27], wherein the external surface area of the zeolite is less than 33.0 $m^2/g$.

[28-1] The oral composition according to any one of [26] to [28], wherein the external surface area of the zeolite is less than 30.0 $m^2/g$.

[28-2] The oral composition according to any one of [26] to [28-1], wherein the external surface area of the zeolite is less than 26.0 $m^2/g$.

[28-3] The oral composition according to any one of [26] to [28-2], wherein the external surface area of the zeolite is 20.0 $m^2/g$ or less.

[28-4] The oral composition according to any one of [26] to [28-3], wherein the external surface area of the zeolite is 15.0 $m^2/g$ or less.

[28-5] The oral composition according to any one of [26] to [28-4], wherein the external surface area of the zeolite is 13.3 $m^2/g$ or less.

[28-6] The oral composition according to any one of [26] to [28-5], wherein the zeolite has an MFI structure, and in an X-ray powder diffraction pattern measured with Cu-K$\alpha$ radiation, the full width at half maximum of the largest peak existing in the range of 20 = 7.9000° $\pm$ 0.5000° is 0.0100° or more and 0.1800° or less.

[28-7] The oral composition according to any one of [26] to [28-6], wherein the zeolite has an MFI structure, and in an X-ray powder diffraction pattern measured with Cu-K$\alpha$ radiation, the full width at half maximum of the largest peak existing in the range of 20 = 7.9000° $\pm$ 0.5000° is 0.0100° or more and 0.1500° or less.

[28-8] The oral composition according to any one of [26] to [28-7], wherein the zeolite has an MFI structure, and in an X-ray powder diffraction pattern measured with Cu-K$\alpha$ radiation, the full width at half maximum of the largest peak existing in the range of $2\theta$ = 7.9000° $\pm$ 0.5000° is 0.0100° or more and 0.1300° or less.

[28-9] The oral composition according to any one of [26] to [28-8], wherein the median diameter of the zeolite is 5.5 $\mu$m or more.

[28-10] The oral composition according to any one of [26] to [28-9], wherein the median diameter of the zeolite is 10.0 $\mu$m or more.

[28-11] The oral composition according to any one of [26] to [28-10], wherein the median diameter of the zeolite is 11.7 μm or more.

[28-12] The oral composition according to any one of [26] to [28-11], wherein the D10 of the zeolite is 2.5 μm or more.

[28-13] The oral composition according to any one of [26] to [28-12], wherein the D10 of the zeolite is 5.0 μm or more.

[28-14] The oral composition according to any one of [26] to [28-13], wherein the D10 of the zeolite is 5.3 μm or more.

[28-15] The oral composition according to any one of [26] to [28-14], wherein the crystal shape of the zeolite is coffin-shaped.

[29] The oral composition according to any one of [26] to [28-15], wherein the oral composition is a pharmaceutical composition.

[30] The oral composition according to [29], wherein the oral composition is administered 1 to 5 times per day, and the dose of the zeolite is 200 mg to 42,000 mg per administration.

[31] The oral composition according to [30], wherein the dose of the zeolite is 500 mg to 2000 mg per administration.

[32] A method for producing the oral composition according to any one of [26] to

[31], comprising obtaining the zeolite by hydrothermal synthesis without using an organic structure-directing agent.

[33] The method according to [32], wherein sodium carbonate and/or sodium sulfate is used as a reagent.

[34] The method according to [32] or [33], comprising converting the zeolite to a proton form with an acid.

[35] The method according to [34], wherein the acid is sulfuric acid or nitric acid.

[36] The method according to any one of [32] to [35], further comprising performing a surface treatment on the zeolite after the hydrothermal synthesis in a solution containing a Si element source.

[37] A method for treating, preventing, or alleviating the symptoms of a disease selected from the group consisting of trimethylaminuria, atherosclerosis, irritable bowel syndrome, and hyperammonemia, comprising administering a therapeutically or prophylactically effective amount of an oral composition containing zeolite to a subject in need thereof,

wherein the oral composition is the oral composition according to any one of [26] to [31].

[38] Use of a zeolite for the manufacture of an oral composition for treating, preventing, or alleviating the symptoms of a disease selected from the group consisting of trimethylaminuria, atherosclerosis, irritable bowel syndrome, and hyperammonemia,

wherein the oral composition is the oral composition according to any one of [26] to [31].

[39] An oral composition comprising a zeolite for use in the treatment, prevention, or symptom alleviation of a disease selected from the group consisting of trimethylaminuria, atherosclerosis, irritable bowel syndrome, and hyperammonemia,

wherein the oral composition is the oral composition according to any one of [26] to [31].

## ADVANTAGEOUS EFFECTS OF INVENTION

According to the present disclosure, by using the oral composition containing an inorganic porous material, it is possible to selectively remove primary, secondary, or tertiary amines or ammonia that act as toxins or pathogenic substances, with almost no removal of the essential nutrient choline. This is useful for the prevention, treatment, or symptom alleviation of diseases caused by primary, secondary, tertiary amines, ammonia, or their metabolites.

## BRIEF DESCRIPTION OF THE DRAWINGS

[FIG. 1] FIG. 1 is a histogram showing the distribution of the number of compounds with respect to the full width at half maximum of the largest peak existing in the range of $2\theta = 7.9000° \pm 0.5000°$ in XRPD (X-ray powder diffraction) for MFI-type zeolites with a TMA adsorption ratio of less than 60% or a choline adsorption ratio of more than 40%, and for MFI-type zeolites with a TMA adsorption ratio of 60% or more and a choline adsorption ratio of 40% or less.

[FIG. 2] FIG. 2 is a graph showing the results of the particle size distribution for the zeolites of Example 26, Example 34, Comparative Example 6, and Comparative Example 22.

[FIG. 3] FIG. 3 is a graph showing the time course of the adsorption amount of TMA (top), choline (middle), and arginine (bottom) for Example 26, Example 34, and Comparative Example 6.

[FIG. 4] FIG. 4 is a graph showing the time course of the release amount of choline for Comparative Example 6, Comparative Example 22, Example 26, and Example 34.

[FIG. 5] FIG. 5 is a graph showing the energy profile when the process of TMA or choline adsorption onto the (010) and (100) surfaces in the thickness direction (z-axis direction) of the zeolite layer was analyzed by first-principles calculation based on density functional theory.

[FIG. 6-1] FIG. 6-1 shows the cross-sectional structure of the zeolite layer at the energy reference point (z=11Å), the highest point of the energy barrier (z=7Å), and during internal adsorption (z=0Å), when the process of TMA adsorption onto the (010) surface in the thickness direction (z-axis direction) of the zeolite layer was analyzed by first-principles calculation based on density functional theory. The zeolite framework is displayed at twice the size of the unit cell along the [001] axis.

[FIG. 6-2] FIG. 6-2 shows the cross-sectional structure of the zeolite layer at the energy reference point (z=8Å), the highest point of the energy barrier (z=5Å), and during internal adsorption (z=0Å), when the process of choline adsorption onto the (010) surface in the thickness direction (z-axis direction) of the zeolite layer was analyzed by first-principles calculation based on density functional theory. The zeolite framework is displayed at twice the size of the unit cell along the [001] axis.

[FIG. 6-3] FIG. 6-3 shows the cross-sectional structure of the zeolite layer at the energy reference point (z=9Å), the highest point of the energy barrier (z=5Å), and during internal adsorption (z=0Å), when the process of TMA adsorption onto the (100) surface in the thickness direction (z-axis direction) of the zeolite layer was analyzed by first-principles calculation based on density functional theory. The zeolite framework is displayed at twice the size of the unit cell along the [001] axis.

[FIG. 6-4] FIG. 6-4 shows the cross-sectional structure of the zeolite layer at the energy reference point (z=9Å), the highest point of the energy barrier (z=7Å), and during internal adsorption (z=0Å), when the process of choline adsorption onto the (100) surface in the thickness direction (z-axis direction) of the zeolite layer was analyzed by first-principles calculation based on density functional theory. The zeolite framework is displayed at twice the size of the unit cell along the [001] axis.

[FIG. 7] FIG. 7 shows the structure at the highest point of the energy barrier (z=5-7Å) from a z-axis perspective, when the process of TMA or choline adsorption onto the (010) surface in the thickness direction (z-axis direction) of the zeolite layer was analyzed by first-principles calculation based on density functional theory. The zeolite framework is displayed at twice the size of the unit cell along the [001] axis.

[FIG. 8] FIG. 8 is a graph showing the time courses of plasma concentration of d9-TMA (top) and their AUCs (bottom) when different doses of the zeolite of Example 34 were administered to choline-load fasted mice. Results are shown as mean value ± standard error of the mean, and † indicates a significant difference from the vehicle group (Shirley-Williams test, $P < 0.05$).

[FIG. 9] FIG. 9 is a graph showing the time courses of plasma concentration of d9-choline (top) and d9-TMA (bottom) when different doses of the zeolite of Comparative Example 6 were administered to choline-load fasted mice. Results are shown as mean value ± standard error of the mean.

[FIG. 10] FIG. 10 shows the XRPD patterns of Example 34 (top) and Example 52 (bottom).

[FIG. 11] FIG. 11 is a conceptual diagram of the physiologically based pharmacokinetic (PBPK) model for d9-choline, d9-TMA, and d9-TMAO when d9-choline was orally administered to mice.

[FIG. 12-1] FIG. 12-1 is a graph comparing the individual measured values (dashed lines) and the predicted values from the PBPK model (solid lines) for the time course of plasma concentrations of d9-choline (top), d9-TMA (middle), and d9-TMAO (bottom) in the vehicle group of choline-load fasted mice.

[FIG. 12-2] FIG. 12-2 is a graph comparing the individual measured values (dashed lines) and the predicted values from the PBPK model (solid lines) for the time course of plasma concentrations of d9-choline (top), d9-TMA (middle), and d9-TMAO (bottom) when the zeolite of Example 34 was administered at 500 mg/kg dose to choline-load fasted mice.

[FIG. 12-3] FIG. 12-3 is a graph comparing the individual measured values (dashed lines) and the predicted values from the PBPK model (solid lines) for the time course of plasma concentrations of d9-choline (top), d9-TMA (middle), and d9-TMAO (bottom) when the zeolite of Example 34 was administered at 1000 mg/kg dose to choline-load fasted mice.

[FIG. 12-4] FIG. 12-4 is a graph comparing the individual measured values (dashed lines) and the predicted values from the PBPK model (solid lines) for the time course of plasma concentrations of d9-choline (top), d9-TMA (middle), and d9-TMAO (bottom) when the zeolite of Example 34 was administered at 2000 mg/kg dose to choline-load fasted mice.

[FIG. 13] FIG. 13 is a graph showing the urinary excretion amounts of d9-TMA and TMAO (top), native TMA and TMAO (middle), and d9-TMA, d9-TMAO, native TMA, and native TMAO (bottom) when different doses of the zeolite of Example 34 were administered to choline-load non-fasted mice. Results are shown as mean value ± standard error of the mean, and # indicates a significant difference from the vehicle group (Williams' multiple comparison test, #: $P < 0.025$, ##: $P < 0.005$, ###: $P < 0.0005$). The urinary excretion amount when the zeolite of Example 70 was administered at 1000 mg/kg dose is also included in the graph. Results are shown as mean value ± standard error of the mean, and * indicates a significant difference from the vehicle group (Student's t-test, *: $P < 0.05$, **: $P < 0.01$).

DESCRIPTION OF EMBODIMENTS

Hereinafter, the present disclosure will be described in detail.

The oral composition of the present disclosure includes an inorganic porous material, and the oral composition has the adsorption ratio of the primary, secondary, or tertiary amine or ammonia of 60.0% or more, and has the adsorption ratio of choline of 40.0% or less, when each adsorption ratio is measured by the following method: to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.

<Inorganic Porous Material>

An inorganic porous material is an inorganic material having a plurality of pores therein, and preferably means an inorganic material having a large number of fine pores with a pore diameter of 100 μm or less.

The inorganic material is preferably a silicate. A silicate may be any compound containing at least silicon (Si) and oxygen (O), and some of these atoms may be substituted with other atoms.

For example, the silicate may be an aluminosilicate in which some silicon atoms are substituted with aluminum atoms. That is, the inorganic material is preferably a silicate or an aluminosilicate.

An aluminosilicate is a compound containing at least aluminum (Al), silicon (Si), and oxygen (O), but some of these atoms may be further substituted with other atoms. The aluminosilicates in the present disclosure include compounds labeled with isotopes (e.g., $^{2}H$, $^{3}H$, $^{17}O$, $^{18}O$, $^{29}Si$, $^{30}Si$, etc.).

Examples of aluminosilicates include zeolite, mullite, kaolinite, illite, etc., with zeolite being preferred.

As for the zeolite, zeolites defined by the International Zeolite Association (IZA) can be used.

The molar ratio of $SiO_2$ to $Al_2O_3$, namely, the $SiO_2/Al_2O_3$ ratio, is usually 15.0 or more, preferably 18.0 or more, more preferably 20.0 or more, still more preferably 30.0 or more, and particularly preferably 31.0 or more. On the other hand, it is usually 130,000.0 or less, preferably 129,000.0 or less, more preferably 50,000.0 or less, still more preferably 10,000.0 or less, still more preferably 1,000.0 or less, yet more preferably 500.0 or less, especially preferably 100.0 or less, and particularly preferably 80.0 or less.

By using a zeolite with a $SiO_2/Al_2O_3$ molar ratio within such a specific range, the polarity of the zeolite can be controlled within a certain range, and high selectivity of adsorption for primary, secondary, or tertiary amines or ammonia over choline can be maintained.

The $SiO_2/Al_2O_3$ ratio is the ratio of $SiO_2$ to $Al_2O_3$ when the molar amounts of Si and Al contained in the zeolite are converted to the molar amounts of $SiO_2$ and $Al_2O_3$, respectively. The method for measuring the respective contents is not particularly limited, but examples include compositional analysis by ICP (Inductively Coupled Plasma) or XRF (X-ray Fluorescence) analysis.

The $SiO_2/Al_2O_3$ ratio of the zeolite can be adjusted by the reaction conditions of zeolite synthesis, etc.

The structure of the zeolite, represented by the codes defined by the IZA, includes, for example, ABW, ACO, AEI, AEN, AFI, AFT, AFX, ANA, ATN, ATT, ATV, AWO, AWW, BIK, CHA, DDR, DFT, EAB, EPI, ERI, ESV, GIS, GOO, ITE, JBW, KFI, LEV, LTA, MER, MON, MTF, OWE, PAU, PHI, RHO, RTE, RWR, SAS, SAT, SAV, SIV, TSC, UFI, VNI, YUG, AEL, AFO, AHT, DAC, FER, HEU, IMF, ITH, MEL, MFS, MWW, OBW, RRO, SFG, STI, SZR, TER, TON, TUN, WEI, MFI, MON, PAU, PHI, MOR, FAU, and the like.

The structure of a zeolite refers to the crystal structure of a crystal composed of tetrahedra in which four oxygens are coordinated to a cation, connected by sharing their vertex oxygens with adjacent cations. Substances with determined crystal structures are defined by the IZA with a structure code for each framework topology, and the type of structure is expressed using that notation.

The zeolite particularly preferably contains a zeolite having a pore structure with a maximum ring number of 10. This allows for control of the pore size and enhances the adsorption selectivity for primary, secondary, or tertiary amines or ammonia over choline.

The term "ring number" refers to the number of oxygen atoms contained in the ring structure that constitutes the cross-section of the zeolite pore, and the "maximum ring number" indicates the largest number of oxygens among the pores composed of oxygen and T-atoms (elements other than oxygen that constitute the framework) forming the zeolite framework. For example, if there are pores of 10-membered oxygen rings and 8-membered oxygen rings, as in FER-type zeolite, it is considered as a 10-membered oxygen ring zeolite.

The zeolite preferably has a largest free sphere diameter of 3.68 Å or more, more preferably 3.70 Å or more, still more preferably 3.75 Å or more, still more preferably 4.22 Å or more, yet more preferably 4.50 Å or more, particularly preferably 4.65 Å or more, and most preferably 4.69 Å or more. On the other hand, the upper limit of the largest free sphere diameter is preferably 4.91 Å or less, more preferably 4.90 Å or less, still more preferably 4.85 Å or less, still more preferably 4.80 Å or less, yet more preferably 4.75 Å or less, particularly preferably 4.73 Å or less, and most preferably 4.71 Å or less.

When the largest free sphere diameter is within the above range, the adsorption selectivity for primary, secondary, or tertiary amines or ammonia over choline can be further enhanced.

For the largest free sphere diameter (Å), one can refer to the value of "that can diffuse along" in the "Maximum diameter of a sphere" part on each framework code page of the Zeolite Framework Types (https://asia.iza-structure.org/IZA-SC/ftc_table.php) by the Structure Commission of the International Zeolite Association (IZA-SC). If there are multiple numerical values for the a, b, and c axes, the largest one should be referenced.

Examples of zeolites having a pore structure with a maximum ring number of 10 include AEL, AFO, AHT, CGF, CGS, DAC, EUO, FER, HEU, IMF, ITH, LAU, MEL, MFI, MFS, MTT, MWW, NES, OBW, PON, RRO, SFF, SFG, STF, STI, SZR, TER, TON, TUN, WEI, and the like. Among these, zeolites that have a pore structure with a maximum ring number of 10 and a largest free sphere diameter of 3.68 Å or more are preferable. Such zeolites are preferably FER or MFI, and more preferably MFI.

Examples of the counter cation of the zeolite include $H^+$ (proton), $NH_4^+$, alkali metal ions ($Li^+$, $Na^+$, $K^+$, $Rb^+$, $Cs^+$, and the like), group 2 element ions ($Ca^{2+}$, $Mg^{2+}$, $Sr^{2+}$, $Ba^{2+}$, and the like), transition metal ions ($Fe^{2+}$, $Cu^{2+}$, $Zn^{2+}$, and the like), and the like. Among these, ions such as proton, $NH_4^+$, $Li^+$, $Na^+$, and $K^+$ are preferred. As an ion suitable for oral administration, a proton is preferred because it can be used safely in the body, preventing excessive intake of various ions.

Furthermore, it is preferable to use a zeolite having an external surface area of less than 33.0 $m^2/g$. It is more preferably less than 30.0 $m^2/g$, still more preferably less than 26.0 $m^2/g$, still more preferably less than 25.8 $m^2/g$, yet more preferably 25.6 $m^2/g$ or less, especially preferably 20.0 $m^2/g$ or less, particularly preferably 15.0 $m^2/g$ or less, and most preferably 13.3 $m^2/g$ or less. The lower limit is not particularly restricted but is, for example, 0.1 $m^2/g$ or more, 1.0 $m^2/g$ or more, 3.0 $m^2/g$ or more, 5.0 $m^2/g$ or more, or 6.5 $m^2/g$ or more.

By keeping the external surface area within a certain range, the selectivity of adsorption for primary, secondary, or tertiary amines or ammonia over choline can be made even better.

Here, the external surface area is the value obtained by subtracting the internal surface area value per unit mass from the total surface area value per unit mass of a solid material. This value can be calculated from the slope and intercept values in the plateau portion of a t-plot (standard isotherm: de-Bore equation) obtained from the results of measuring the nitrogen adsorption isotherm at liquid nitrogen temperature for a powder that has undergone heating and degassing as a pretreatment.

Moreover, for MFI-type zeolite, it is preferable to use one in which the full width at half maximum of the largest peak existing in the range of $2\theta = 7.9000° \pm 0.5000°$ in an X-ray powder diffraction pattern measured with Cu-K$\alpha$ radiation is 0.1800° or less. It is more preferably 0.1500° or less, and still more preferably 0.1300° or less. The lower limit is not particularly restricted but is, for example, 0.0100° or more, 0.0300° or more, 0.0500° or more, 0.0700° or more, or 0.0900° or more.

By keeping the full width at half maximum of the largest peak existing in the range of $2\theta = 7.9000° \pm 0.5000°$ in an X-ray powder diffraction pattern measured with Cu-K$\alpha$ radiation within a certain range, the selectivity of adsorption for primary, secondary, or tertiary amines or ammonia over choline can be made even better.

Here, the full width at half maximum is the spectral width at half the value of the peak maximum.

Furthermore, it is preferable to use a zeolite with a median diameter in its particle size distribution of 5.5 $\mu$m or more. It is more preferably 10.0 $\mu$m or more, and still more preferably 11.7 $\mu$m or more. The upper limit is not particularly restricted but is, for example, 10.0 mm or less.

By keeping the median diameter of the particle size distribution within a certain range, the selectivity of adsorption for primary, secondary, or tertiary amines or ammonia over choline can be made even better.

Furthermore, it is preferable to use a zeolite with a D10 of 2.5 $\mu$m or more. It is more preferably 5.0 $\mu$m or more, and still more preferably 5.3 $\mu$m or more. The upper limit is not particularly restricted but is, for example, 5.0 mm or less.

By keeping the D10 within a certain range, the selectivity of adsorption for primary, secondary, or tertiary amines or ammonia over choline can be made even better.

Here, the median diameter and the D10 are the particle sizes at which the cumulative values in the particle size distribution obtained by the laser diffraction/scattering method are 50% and 10%, respectively, and are the values measured while circulating in a flow cell after applying ultrasonic waves, using pure water as a dispersion medium.

Moreover, the crystal shape of the MFI-type zeolite is preferably coffin-shaped.

By having a specific crystal shape for the MFI-type zeolite, the selectivity of adsorption for primary, secondary, or tertiary amines or ammonia over choline can be made even better.

Here, coffin-shaped refers to a shape where the (010) surface is dominant when observed with a scanning electron microscope (SEM).

Furthermore, by selectively surface-treating surfaces other than the (010) surface, the selectivity of adsorption for primary, secondary, or tertiary amines or ammonia over choline can be made even better.

The zeolite may be a commercially available product, or it may be synthesized by hydrothermal synthesis as described later or other synthesis methods.

The oral composition of the present disclosure has the adsorption ratio of the primary, secondary, or tertiary amine or ammonia of 60.0% or more, and has the adsorption ratio of choline of 40.0% or less, when each adsorption ratio is measured by the following method:
to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.

The adsorption ratio of the primary, secondary, or tertiary amine or ammonia is usually 60.0% or more, preferably 60.4%, more preferably 70.0% or more, still more preferably 80.0%, and particularly preferably 90.0% or more. On the other hand, the adsorption ratio of the primary, secondary, or tertiary amine or ammonia can be, for example, 100.0% or less.

Furthermore, the adsorption ratio of choline is usually 40.0% or less, preferably 30.0% or less, more preferably 20.0% or less, and still more preferably 10.0% or less. On the other hand, the adsorption ratio of choline can be, for example, 0.0% or more, 0.1% or more, or 0.2% or more.

Furthermore, it is preferable that the adsorption ratio of the primary, secondary, or tertiary amine or ammonia is 2.00 times or more that of choline, more preferably 2.49 times or more, and still more preferably 2.50 times or more. On the other hand, while the upper limit is not particularly specified, for example, the adsorption ratio of the primary, secondary, or tertiary amine or ammonia can be 350.00 times or less, 342.00 times or less, or 341.70 times or less that of choline.

Here, choline is the compound identified by CAS Registry Number 62-49-7, which is a quaternary ammonium cation represented by the structural formula $(CH_3)_3N^+(CH_2)_2OH$.

Ammonia is the compound identified by CAS Registry Number 7664-41-7, represented by the structural formula $NH_3$. The ammonium cation ($NH_4^+$) is also included.

The primary, secondary, and tertiary amines are compounds in which hydrogen atoms of ammonia are substituted with alkyl or aromatic groups. If one is substituted, it is a primary amine; if two, a secondary amine; if three, a tertiary amine. For example, heterocyclic aromatics such as imidazole and pyridine are also included in amines. Primary, secondary, and tertiary amines also include their conjugate acids.

As the simulated intestinal fluid, Fasted-state Simulated Small Intestine Fluid (FaSSIF) is used.
Reference: Dissolution Technologies 20(3):44-50 "Comparison of the Solubility and Dissolution of Drugs in Fasted-State Biorelevant Media (FaSSIF and FaSSIF-V2)"

Specifically, FaSSIF (pH 6.5) having the following composition can be used. 3.5 mM Sodium taurocholate, 0.75 mM Lecithin, 105.85 mM Sodium chloride, 10.5 mM Sodium hydroxide, 28.65 mM Sodium dihydrogen phosphate.

The adsorption ratio means the value obtained by subtracting from 1 the value obtained by dividing the concentration of the substance in the solution after the adsorption operation by the concentration in the solution before the adsorption operation.

For example, an equimolar amount of choline and a primary, secondary, or tertiary amine or ammonia are placed in simulated intestinal fluid, the inorganic porous material is added to the simulated intestinal fluid at a concentration of 2 g/L in an amount of 11.8 g of the inorganic porous material per 1 mmol of choline, and an adsorption reaction is carried out at room temperature for 1 hour. After that, the respective adsorption ratios of choline and the primary, secondary, or tertiary amine or ammonia are measured, and it can be confirmed that the adsorption ratio of the primary, secondary, or tertiary amine or ammonia is 60.0% or more, and the adsorption ratio of choline is 40.0% or less.

Room temperature is a temperature of 20°C $\pm$ 10°C.

Also, for example, an equimolar amount of choline and TMA are placed in simulated intestinal fluid, the inorganic porous material is added to the simulated intestinal fluid at a concentration of 2 g/L in an amount of 11.8 g of the inorganic porous material per 1 mmol of choline, and an adsorption reaction is carried out for 1 hour. After that, the respective adsorption ratios of choline and TMA are measured, and it can be confirmed that the adsorption ratio of TMA is 60.0% or more, and the adsorption ratio of choline is 40.0% or less.

<Oral Composition>

As used herein, an oral composition refers to a mixture of substances suitable for oral administration to an individual. For example, the oral composition may contain one or more inorganic porous bodies and an orally acceptable carrier (such as an excipient, binder, disintegrant, flavoring agent, odor improving agent, emulsifier, diluent, solubilizer, etc.).

The orally acceptable carrier is appropriately selected depending on the dosage form, and examples include excipients, lubricants, binders, and disintegrants in solid preparations, or solvents, solubilizers, emulsifiers, diluents, suspending agents, isotonic agents, buffers, and soothing agents in liquid preparations. Furthermore, if necessary, conventional additives such as preservatives, antioxidants, coloring agents, flavoring agents, odor improving agents, and wetting agents can be used in appropriate amounts.

The dosage form of the oral composition may be in the form of solid or liquid dosage forms, specifically tablets, coated tablets, pills, fine granules, granules, powders, capsules, syrups, emulsions, suspensions, injections, troches, enteric capsules, and the like.

The oral composition of the present disclosure may further contain other therapeutically effective agents, for example, inhibitors of TMA-producing enzymes such as cutC and cntAB, TMA-degrading enzymes such as trimethylamine dehydrogenase; riboflavin; and antibacterial agents. Also, if necessary, components such as anti-inflammatory agents, vitamins, and amino acids can be blended.

The oral composition containing the inorganic porous material of the present disclosure can be used, for example, as a pharmaceutical composition, a supplement, a food, or a food additive.

The supplement, food, and food additive may be those that have functions or uses such as for adsorbing primary, secondary, or tertiary amines or ammonia; for adsorbing and removing primary, secondary, or tertiary amines or ammonia; or for preventing, treating, or alleviating symptoms of diseases caused by primary, secondary, or tertiary amines, ammonia, or their metabolites.

The oral composition containing the inorganic porous material of the present disclosure can be preferably used as a pharmaceutical composition, and specifically, can be used to treat, prevent, or alleviate symptoms of diseases caused by primary, secondary, or tertiary amines, ammonia, or their metabolites.

The inorganic porous material contained in the oral composition of the present disclosure is insoluble in water and exceeds the particle size suitable for endocytosis (100 nm), so its absorption into the body is negligible. Furthermore, the zeolite contained in the oral composition of the present disclosure is stable in the stomach and other parts of the digestive tract, and the elution of its constituent components, including silicon and aluminum, is very small, making it highly safe when taken.

One embodiment of the present disclosure is a pharmaceutical composition for the treatment, prevention, or symptom alleviation of a disease caused by a primary, secondary, or tertiary amine, ammonia, or their metabolites, including the inorganic porous material or the oral composition containing it according to the embodiments of the present disclosure described above.

One embodiment of the present disclosure is a method for treating, preventing, or alleviating the symptoms of a disease caused by a primary, secondary, or tertiary amine, ammonia, or their metabolites, including the step of administering an effective amount of the inorganic porous material or the oral composition containing it according to the embodiments of the present disclosure described above to a subject.

One embodiment of the present disclosure is the use of the inorganic porous material or the oral composition containing it according to the embodiments of the present disclosure described above for the manufacture of a medicament for treating, preventing, or alleviating the symptoms of a disease caused by a primary, secondary, or tertiary amine, ammonia, or their metabolites.

One embodiment of the present disclosure is the inorganic porous material or the oral composition containing it according to the embodiments of the present disclosure described above for use in the treatment, prevention, or symptom alleviation of a disease caused by a primary, secondary, or tertiary amine, ammonia, or their metabolites.

The primary, secondary, or tertiary amine, ammonia, or their metabolite is preferably one or more selected from the group consisting of trimethylamine, dimethylamine, methylamine, histamine, ammonia, and trimethylamine-N-oxide, and more preferably trimethylamine or trimethylamine-N-oxide.

The disease caused by a primary, secondary, or tertiary amine, ammonia, or their metabolites is not particularly limited as long as it is a disease or symptom that can be caused by these substances, but includes diseases that can be treated, prevented, or have their symptoms alleviated by reducing or removing these substances.

More specifically, diseases caused by primary, secondary, or tertiary amines, ammonia, or their metabolites include trimethylaminuria, cardiovascular disease, glaucoma, atherosclerosis, coronary artery heart disease, heart failure with preserved ejection fraction, ST-elevation myocardial infarction, atrial fibrillation, abdominal aortic aneurysm, ischemic stroke, post-stroke cognitive impairment, mild cognitive impairment, Alzheimer's disease, obesity, progressive chronic kidney disease with type 2 diabetes, cardiovascular complications in chronic kidney disease, diabetic retinopathy, non-alcoholic steatohepatitis, polycystic ovary syndrome, Parkinson's disease, colorectal cancer, irritable bowel syndrome, hyperammonemia, urea cycle disorder, organic acidemia, hepatic encephalopathy, portosystemic shunt, and the like.

For example, when the causative substance of the disease is trimethylamine, the diseases that can be treated, prevented, or have their symptoms alleviated by the oral composition containing the inorganic porous material of the present disclosure can include trimethylaminuria, cardiovascular disease, or glaucoma, preferably trimethylaminuria.

Here, trimethylaminuria is a disease in which trimethylamine, generated during the digestion and decomposition of ingested food in the body, is not metabolized and is excreted in sweat, urine, and breath. Because trimethylamine has the odor of rotting fish, it is also called fish odor syndrome. It is broadly classified into type 1 (primary trimethylaminuria), which is a genetic or congenital deficiency or low activity of flavin-containing monooxygenase 3 (FMO3), the enzyme that oxidatively metabolizes trimethylamine, and type 2 (secondary trimethylaminuria), which is an acquired condition due to renal/hepatic dysfunction, reduced renal/hepatic function, portosystemic shunt, imbalance of intestinal flora (dysbiosis), excessive intake of trimethylamine precursors, or a combination of these factors. In addition, there are an intermittent form, in which FMO3 expression is affected by steroid hormones and fluctuates in correlation with the menstrual cycle, and a transient form, in which FMO3 expression is reduced in infants and young children. Different types may also coexist.

The relationship between trimethylamine and each disease is described, for example, in the following literature:

Trimethylaminuria: European Journal of Human Genetics volume 23, page 1269 (2015)
Cardiovascular disease: Toxins 2019, 11(9), 490
Glaucoma: International Ophthalmology volume 41, pages 341-347 (2021)

For example, when the causative substance of the disease is trimethylamine-N-oxide, the diseases that can be treated, prevented, or have their symptoms alleviated by the oral composition containing the inorganic porous material of the present disclosure can include atherosclerosis, coronary artery heart disease, heart failure with preserved ejection fraction, ST-elevation myocardial infarction, atrial fibrillation, abdominal aortic aneurysm, ischemic stroke, post-stroke cognitive impairment, mild cognitive impairment, Alzheimer's disease, obesity, progressive chronic kidney disease with type 2 diabetes, cardiovascular complications in chronic kidney disease, diabetic retinopathy, non-alcoholic steatohepatitis, polycystic ovary syndrome, Parkinson's disease, or colorectal cancer, preferably atherosclerosis.

Here, atherosclerosis is a disease characterized by patchy intimal plaques (atheromata) that encroach on the lumen of medium-sized and large arteries, thereby reducing or blocking blood flow.

The relationship between trimethylamine-N-oxide and each disease is described, for example, in the following literature:

Atherosclerosis: Nature Medicine volume 19, pages 576-585 (2013)
Coronary artery heart disease: BMC Cardiovascular Disorders (2020) 20:7
Heart failure with preserved ejection fraction: BMC Cardiovascular Disorders (2020) 20:394
ST-elevation myocardial infarction: American Journal of Cardiology. 2019 Mar 15;123(6):894-898.
Abdominal aortic aneurysm: Circulation Volume 147, Issue 14, 4 April 2023; Pages 1079-1096
Atrial fibrillation: International Journal of Cardiology Volume 267, 15 September 2018, Pages 100-106
Ischemic stroke: Journal of Biochemical and Molecular Toxicology 2019 Feb;33(2):e22246
Post-stroke cognitive impairment: Neurological Sciences volume 41, pages 57-63 (2020)
Mild cognitive impairment and Alzheimer's disease: Alzheimer's Research & Therapy (2018) 10:124
Obesity: Obesity Reviews. 2020;21(5):e12993.
Progressive chronic kidney disease with type 2 diabetes: Journal of Clinical Medicine 2017, 6(9), 86
Cardiovascular complications in chronic kidney disease: Kidney International Volume 92, Issue 4, October 2017, Pages 809-815
Diabetic retinopathy: Acta Diabetologica 58, 221-229 (2021)
Non-alcoholic steatohepatitis: Diabetes & Metabolism 47 (2021) 101183
Polycystic ovary syndrome: BMC Endocr Disord. 2020; 20: 3.
Parkinson's disease: Clinica Chimica Acta Volume 501, February 2020, Pages 165-173 Colorectal cancer: International Journal of Molecular Sciences 2020, 21, 6782

For example, when the causative substance of the disease is histamine, the disease that can be treated, prevented, or have its symptoms alleviated by the oral composition containing the inorganic porous material of the present disclosure can be irritable bowel syndrome.

Here, irritable bowel syndrome refers to a syndrome in which abnormal bowel movements such as chronic diarrhea or constipation, abdominal pain, and abdominal bloating occur, despite the absence of structural abnormalities such as ulcers or tumors in the large and small intestines.

The relationship between histamine and irritable bowel syndrome is described, for example, in the following literature:

Science Translational Medicine 14, eabj1895 (2022)

For example, when the causative substance of the disease is ammonia, the diseases that can be treated, prevented, or have their symptoms alleviated by the oral composition containing the inorganic porous material of the present disclosure can include hyperammonemia, urea cycle disorder, organic acidemia, hepatic encephalopathy, or portosystemic shunt, preferably hyperammonemia.

Here, hyperammonemia is a disease in which ammonia in the body cannot be metabolized, and ammonia accumulates in the blood. Causes include decreased function of the urea cycle due to cirrhosis, fulminant hepatitis, decreased liver function, etc., portosystemic shunt, urea cycle disorders, organic acidemia, ammonia production associated with infection by urease-producing bacteria, and drug-induced causes (sodium valproate, pivalic acid-containing antibiotics). Hyperammonemia can also lead to symptoms such as hepatic encephalopathy and hepatic coma.

The relationship between ammonia and hyperammonemia and its related diseases is described in the following literature:

Pediatric Nephrology volume 27, pages 207-222 (2012)

Furthermore, the inventors have found that among inorganic porous materials, zeolites, particularly one or more zeolites selected from the group consisting of zeolites with a specific range of molar ratio of $SiO_2$ to $Al_2O_3$ and zeolites with a maximum ring number of 10 and a largest free sphere diameter of 3.68 Å or more, have excellent performance in selectively adsorbing primary, secondary, or tertiary amines or ammonia in comparison with choline, and are useful for the treatment, prevention, or symptom alleviation of diseases caused by primary, secondary, or tertiary amines, ammonia, or their metabolites.

Based on the above findings, one aspect of the oral composition of the present disclosure includes:

An oral composition, including a zeolite, wherein
the zeolite has an MFI structure and a molar ratio of $SiO_2$ to $Al_2O_3$ of 30.0 or more and 130,000.0 or less, or
the zeolite has a FER structure and a molar ratio of $SiO_2$ to $Al_2O_3$ of 15.0 or more and 130,000.0 or less,
for use in the treatment, prevention, or symptom alleviation of one or more diseases selected from the group consisting of trimethylaminuria, atherosclerosis, irritable bowel syndrome, and hyperammonemia.

Alternatively, a preferred aspect of the oral composition of the present disclosure includes:

An oral composition, including a zeolite, wherein
the zeolite has an MFI structure and a molar ratio of $SiO_2$ to $Al_2O_3$ of 30.0 or more and 80.0 or less, or
the zeolite has a FER structure and a molar ratio of $SiO_2$ to $Al_2O_3$ of 15.0 or more and 130,000.0 or less,
for use in the treatment, prevention, or symptom alleviation of one or more diseases selected from the group consisting of trimethylaminuria, atherosclerosis, irritable bowel syndrome, and hyperammonemia.

The oral composition or pharmaceutical composition containing the inorganic porous material of the present disclosure can be administered to a subject by oral administration.

The "subject" to which the oral composition or pharmaceutical composition of the present disclosure is administered includes humans or non-human mammals (e.g., one or more of mice, guinea pigs, hamsters, rats, mice, rabbits, pigs, sheep, goats, cattle, horses, cats, dogs, marmosets, monkeys, or chimpanzees). The "subject" may be a patient diagnosed with a disease caused by a primary, secondary, or tertiary amine, ammonia, or their metabolites, or a subject at risk of developing a disease caused by a primary, secondary, or tertiary amine, ammonia, or their metabolites.

"Treatment" includes any treatment of a disease in a mammal, particularly a human, and includes inhibiting the symptoms of the disease, i.e., arresting its progression or eliminating the disease or symptoms, causing regression of the disease or symptoms, or delaying the progression of the symptoms.

"Symptom alleviation" includes reducing or relieving the symptoms of the above diseases and reducing the frequency of symptoms in a mammal, particularly a human.

"Prevention" includes preventing the onset of the above diseases in a mammal, particularly a human.

The dosage of the oral composition or pharmaceutical composition containing the inorganic porous material of the present disclosure is determined by a physician based on various factors such as, for example, the type of disease, the severity of symptoms, the patient's age, sex, weight, the severity of the disease, pharmacological knowledge such as pharmacokinetics and toxicological characteristics, the presence or absence of the use of a drug delivery system, and whether it is administered as part of a combination of other drugs. Usually, for an adult (body weight 60 kg), the oral dose can be 200 mg/dose to 42,000 mg/dose, preferably 500 mg/dose to 2,000 mg/dose. Administration may be performed once or multiple times per day, for example, 1 to 5 times per day.

<Method for Producing Zeolite>

One aspect of the present disclosure relates to a method for producing zeolite. However, the zeolite contained in the oral composition of the present disclosure is not limited to that obtained by the following production method.

Hydrothermal synthesis is a method for synthesizing substances and growing crystals in the presence of high-temperature water at 50°C or higher.

In the method for producing zeolite, hydrothermal synthesis is performed in a reaction system containing a Si element source (a substance containing at least silicon (Si)), an Al element source (a substance containing at least aluminum (Al)), an alkali metal salt, and water, and, if necessary, further containing a structure-directing agent. Seed crystals of zeolite may be added to the reaction system.

As the Al element source, aluminum sulfate, aluminum nitrate, aluminum hydroxide, sodium aluminate, pseudo-boehmite, alumina sol, aluminum alkoxides such as aluminum isopropoxide, and the like are usually used. Also, aluminum-containing zeolites such as FAU-type zeolite (e.g., Y-type zeolite) and CHA-type zeolite may be used as the aluminum source. Among these aluminum sources, from the viewpoint of reactivity, aluminum sulfate, aluminum nitrate, aluminum hydroxide, or aluminum-containing zeolite is preferable, and aluminum hydroxide or aluminum-containing zeolite is more preferable.

The usage amount of Al element source, from the viewpoint of ease of preparation of the pre-reaction mixture or the aqueous gel obtained by aging it and production efficiency, is usually 0.001 or more, preferably 0.005 or more, more preferably 0.01 or more, and still more preferably 0.02 or more, in terms of the molar ratio of aluminum (Al) in the Al element source to silicon (Si) contained in the ingredient mixture other than the seed crystals added as necessary. Further, the upper limit is not particularly limited, but from the viewpoint of uniformly dissolving the Al element source in the aqueous gel, it is usually 1 or less, preferably 0.5 or less, more preferably 0.25 or less, still more preferably 0.1 or less, particularly preferably 0.08 or less, and most preferably 0.06 or less.

The Si element source is not particularly limited, and various known substances can be used. Colloidal silica, amorphous silica, sodium silicate, trimethylethoxysilane, tetraethyl orthosilicate, aluminosilicate gel, and the like can be used, and zeolites containing Si atoms can also be used. These may be used alone or in combination of two or more. Among these, those in a form that can be mixed sufficiently uniformly with other components, and, particularly, ingredients that are easily soluble in water, are preferable, and colloidal silica, trimethylethoxysilane, tetraethyl orthosilicate, or aluminosilicate gel is preferable.

The alkali metal cation exists in the vicinity of the aluminum anion to perform charge compensation and has the effect of assisting the crystal growth of the desired structure in the same way as the structure-directing agent.

The alkali metal salt contains a sodium salt and/or a potassium salt and can also contain other alkali metal atoms. The other alkali metal atoms are not particularly limited, and known ones used for zeolite synthesis can be used, for example, lithium, rubidium, and cesium can be mentioned, and these may be used in combination of two or more.

As the alkali metal salt, inorganic acid salts such as hydroxides, oxides, carbonates, sulfates, nitrates, phosphates, chlorides, and bromides of the above alkali metal atoms, and organic acid salts such as acetates, oxalates, and citrates can be used. The alkali metal salt may be one kind or two or more kinds.

The usage amount of water, from the viewpoint of ease of crystal formation, is usually 5 or more, preferably 10 or more, more preferably 12 or more, still more preferably 15 or more, and particularly preferably 17 or more, in terms of the molar ratio to silicon (Si) contained in the ingredient mixture other than the seed crystals. This range is preferable because crystals are more easily formed. Also, in order to sufficiently obtain the effect of cost reduction for wastewater treatment, it is usually 100 or less, preferably 90 or less, more preferably 75 or less, and still more preferably 50 or less, in terms of the molar ratio to silicon (Si) contained in the ingredient mixture other than the seed crystals.

A structure-directing agent is a substance that may be added as a reagent during the synthesis of zeolite and is a material added to control the structure of the zeolite obtained by synthesis. The type is not limited as long as it can synthesize the desired zeolite, and any type may be used. Also, one kind of structure-directing agent may be used, or two or more kinds may be used in combination, or these structure-directing agents may not be used.

Structure-directing agents are broadly classified into organic structure-directing agents and inorganic structure-directing agents. Examples of organic structure-directing agents usually include amines, quaternary ammonium salts, and primary alcohols. Also, examples of inorganic structure-directing agents include salts of inorganic cations with nitrates, sulfates, carbonates, and the like.

When an organic structure-directing agent is used, a calcination step is required to remove the structure-directing agent from the zeolite, but it is considered that there are no approved pharmaceuticals that require a calcination step, and there are no GMP-compliant calcination facilities for pharmaceutical manufacturing. Therefore, in order to use the oral composition of the present disclosure for pharmaceutical applications, it is preferable not to use an organic structure-directing agent in the hydrothermal synthesis. That is, in a preferred embodiment, the method for producing the oral pharmaceutical composition of the present disclosure is a method including obtaining the zeolite by hydrothermal synthesis without using an organic structure-directing agent. Also, from the viewpoint of obtaining a good quality zeolite, it is more preferable to use an inorganic structure-directing agent in the hydrothermal synthesis. Furthermore, when the target zeolite is MFI-type, it is preferable to use an inorganic salt with Na as the cation as the inorganic structure-directing agent from the viewpoint that it is easy to synthesize MFI-type zeolite, and it is still more preferable to use sodium carbonate and/or sodium sulfate from the viewpoint that the effect of increasing the particle size is strong.

In the method for producing zeolite, a Si element source, an Al element source, an alkali metal salt, and water, and if necessary, a structure-directing agent are mixed to obtain a mixture, and if necessary, seed crystals are further added to obtain a pre-reaction mixture, which is then hydrothermally synthesized.

The order of mixing these ingredients is not particularly limited, but it is preferable to mix water and an alkali metal salt to prepare an alkaline solution, and then add the Al element source, the Si element source, and if necessary, seed crystals to this alkaline solution in this order and mix them, since it is preferable to add the Si element source and the Al element source after preparing the alkaline solution from the viewpoint that the ingredients dissolve more uniformly.

As the seed crystals, zeolite is usually used. If the zeolite to be produced is an aluminosilicate, the seed crystal is preferably also an aluminosilicate zeolite. The seed crystals may contain an amorphous component as part of them. Also, the zeolite used as seed crystals may or may not contain an organic structure-directing agent. The method for producing the zeolite to be used as seed crystals is not particularly limited, and it may be one produced by the method for producing zeolite exemplified as the method for producing the oral composition of the present disclosure, or it may be one produced by another method, for example, a general batch method using an autoclave or the like. The amount of seed crystals is preferably large from the viewpoint that the effect of promoting crystallization as seed crystals is easily expressed. On the other hand, it is preferably small from the viewpoint that the seed crystals are easily dissolved and function easily as seed crystals. Therefore, it is preferably 0.1% by mass or more, more preferably 0.5% by mass or more, and still more preferably 1.0% by mass or more, with respect to the silica ($SiO_2$) contained in the ingredient mixture other than the seed crystals contained in the ingredient composition. On the other hand, it is preferably 30% by mass or less, more preferably 20% by mass or less, still more preferably 15% by mass or less, and particularly preferably 10% by mass or less.

In addition to the above Al element source, Si element source, alkali metal salt, water, and if necessary, seed crystals, other additives such as an auxiliary agent that is a component to assist the synthesis of zeolite, for example, an acid component that promotes the reaction, and a metal stabilizer such as a polyamine may be added and mixed in any step as necessary to prepare the pre-reaction mixture.

The pre-reaction mixture prepared as described above may be hydrothermally synthesized immediately after preparation, but in order to obtain a zeolite with high crystallinity, it is preferable to age it for a certain period of time under a predetermined temperature condition. In particular, when scaling up, the stirring efficiency tends to decrease, and the mixed state of the ingredients tends to be insufficient. Therefore, it is preferable to improve the ingredients to a more uniform state by aging them while stirring for a certain period of time. The aging temperature is usually 100°C or less, preferably 95°C or less, more preferably 90°C or less, and the lower limit is not particularly set, but is usually 0°C or more, preferably 10°C or more. The aging temperature may be constant during aging, or it may be changed stepwise or continuously. The aging time is not particularly limited, but is usually 2 hours or more, preferably 3 hours or more, more preferably 5 hours or more, and usually 30 days or less, preferably 10 days or less, and still more preferably 4 days or less.

The hydrothermal synthesis is performed, for example, by placing the pre-reaction mixture prepared as described above or the aqueous gel obtained by aging it in a pressure vessel and maintaining a predetermined temperature under self-generated pressure or under gas pressure to the extent that it does not inhibit crystallization, while stirring, or while rotating or shaking the vessel, or in a stationary state.

The reaction temperature during hydrothermal synthesis is usually 100°C or higher, and usually 230°C or lower, preferably 220°C or lower, more preferably 200°C or lower, and still more preferably 190°C or lower. The reaction time is not particularly limited, but is usually 2 hours or more, preferably 3 hours or more, more preferably 5 hours or more, and particularly preferably 1 day or more. The upper limit is not particularly limited, and is usually 30 days or less, preferably 10 days or less, more preferably 7 days or less, and still more preferably 5 days or less. The reaction temperature may be constant during the reaction, or it may be changed stepwise or continuously.

After the above hydrothermal synthesis, the produced zeolite is separated from the hydrothermal synthesis reaction solution. The method for separating the zeolite from the hydrothermal synthesis reaction solution is not particularly limited, but usually includes methods such as filtration, decantation, or direct drying.

The zeolite separated and recovered from the hydrothermal synthesis reaction solution is washed with water and dried as necessary.

The temperature during drying is usually 50°C or higher, preferably 80°C or higher, and more preferably 100°C or higher. On the other hand, the temperature during drying is usually 200°C or lower, and preferably 150°C or lower.

The time for drying is not particularly limited as long as it is a time for the zeolite to be sufficiently dried, and is preferably 0.5 hours or more, and more preferably 1 hour or more. The upper limit is not particularly limited, and is usually 200 hours or less, preferably 150 hours or less, and more preferably 100 hours or less.

When an organic structure-directing agent is used in hydrothermal synthesis, the organic structure-directing agent is removed by calcination after drying.

The temperature during calcination is usually 350°C or higher, preferably 400°C or higher, more preferably 430°C or higher, and still more preferably 480°C or higher. On the other hand, the temperature during calcination is usually 900°C or lower, preferably 850°C or lower, more preferably 800°C or lower, and still more preferably 750°C or lower.

During calcination, when the calcination temperature is the above lower limit value or higher, it is possible to prevent the proportion of the remaining organic structure-directing agent from increasing and to suppress the inhibition of adsorption by the organic structure-directing agent remaining in the zeolite pores. On the other hand, when the calcination temperature is the above upper limit value or lower, it is possible to prevent the framework structure of the zeolite from being broken and the adsorption selectivity from being lost due to the loss of the uniform pore structure of the framework.

The heating time during calcination is not particularly limited as long as it is a time for the organic structure-directing agent to decompose, and is preferably 0.5 hours or more, and more preferably 1 hour or more. The upper limit is not particularly limited, and is usually 200 hours or less, preferably 150 hours or less, and more preferably 100 hours or less. The calcination may be performed in an air atmosphere, but may also be performed in an atmosphere to which an inert gas such as $N_2$ or oxygen is added.

The method for producing zeolite according to one aspect of the present disclosure may further include exchanging cations by ion exchange. When synthesized using an organic structure-directing agent, ion exchange is usually performed after removing the organic structure-directing agent. Examples of ions to be ion-exchanged include $H^+$ (proton), $NH_4^+$, alkali metal ions ($Li^+$, $Na^+$, $K^+$, $Rb^+$, $Cs^+$, etc.), group 2 element ions ($Ca^{2+}$, $Mg^{2+}$, $Sr^{2+}$, $Ba^{2+}$, etc.), transition metal ions ($Fe^{2+}$, $Cu^+$, $Cu^{2+}$, $Zn^{2+}$, etc.), and the like. Among these, ions such as proton, $NH_4^+$, $Li^+$, $Na^+$, and $K^+$ are preferable. As an ion suitable for oral administration, a proton is preferable because it can be used safely in the body, preventing excessive intake of various ions.

Ion exchange may be performed by a method such as treating the zeolite with an aqueous solution containing a nitrate such as $NH_4NO_3$ or $KNO_3$ or an ion to be exchanged, and in some cases, an acid such as hydrochloric acid, usually at a temperature from room temperature to 100°C, and then washing with water. Furthermore, it may be calcined at 100°C to 750°C as necessary.

As a method for synthesizing a proton-type zeolite in which 50% or more of the cations existing inside the zeolite as charge compensation for the atoms constituting the framework structure of the zeolite are protons ($H^+$), there are a method of calcination after ion-exchanging with ammonium ions, a method of treating by bringing it into contact with an acid, and the like. In particular, it is preferable to convert to the proton type by acid treatment from the viewpoint that the calcination step during synthesis can be avoided. Also, as the acid to be used, organic acids such as carboxylic acids and sulfonic acids, and inorganic acids such as sulfuric acid, nitric acid, phosphoric acid, and hydrochloric acid can be mentioned, and these may be used alone or in combination of two or more. Among these, sulfuric acid or nitric acid is more preferable because it is a strong acid and ion exchange is efficiently promoted.

The method for producing zeolite according to one aspect of the present disclosure may further include performing a surface treatment on the zeolite after the hydrothermal synthesis in a solution containing a Si element source.

The solution used for this surface treatment is not particularly limited as long as it is in a state where the zeolite can be treated under the surface treatment conditions, and may be a solution obtained by adding a solvent to a Si compound, may be a liquid without adding a solvent, or may be a sol or a gel. Here, the solvent may be water or an organic solvent. Also, at a temperature higher than the boiling point, a substance that is in a liquid state under pressure is also included in the solvent. The pressure in this case may be autogenous pressure or applied pressure. Furthermore, the liquid for surface treatment may contain at least a Si compound and may also contain, for example, an Al compound as another element source (compound).

As the Si compound, for example, alkylhalosilanes such as trichloroethylsilane, dichlorohexylmethylsilane, and chlorotrimethylsilane, alkylalkoxysilanes such as methyltriethoxysilane, 3-aminopropyltriethoxysilane, and 1,1,3,3-tetramethoxy-1,3-dimethylpropanedisiloxane, and siloxane-containing organosilicon compounds such as hexamethyldisiloxane, silazane-containing organosilicon compounds such as hexamethyldisilazane, silicates such as tetramethoxysilane and tetraethoxysilane, silicate oligomers such as methyl silicate oligomer and ethyl silicate oligomer, amorphous silica, fumed silica, colloidal silica, silica gel, sodium silicate, silica sol, and the like can be used. These Si compounds may be used alone or in combination of two or more.

As the treatment method, a chemical bond with the Si compound may be formed by immersing the zeolite in a liquid, a chemical bond may be formed after immersion, or a chemical bond may be formed both during and after immersion. Also, a chemical bond may be formed during and/or after exposure to the zeolite with the evaporated gas of the liquid by a method such as heating.

## EXAMPLES

Hereinafter, the present disclosure will be described more specifically by giving examples, but these are not intended to limit the scope of the present disclosure.

### <In Vitro Adsorption Test>

The adsorption capacity of various zeolites for TMA, choline, etc. was evaluated in vitro according to the following procedure. The zeolites used were synthesized or obtained according to the Reference Examples described later.

### Test Method

[Adsorption Test]

A reaction solvent was prepared by adding glycine, lysine, and histidine to Fasted-state Simulated Small Intestine Fluid (hereinafter sometimes referred to as FaSSIF or simulated intestinal fluid) prepared according to the preparation method described in the literature (Dissolution Technologies 20(3):44-50). The concentrations of glycine, lysine, and histidine in the reaction solvent were 0.30, 0.42, and 0.21 mg/mL, respectively. A standard solution was prepared by accurately weighing 2050 mg of arginine, 120 mg of choline chloride, and 80 mg of TMA hydrochloride into 50 mL of a 0.1N hydrochloric acid aqueous solution prepared by diluting 1N hydrochloric acid with ultrapure water, and shaking well for dissolution. 10 mg of zeolite was accurately weighed into a 25 mL tube, and 5 mL of the reaction solvent was added. A stir bar and 50 μL of the standard solution were added to this solution. This solution was stirred for 1 hour to prepare a reaction solution. Separately, 5 mL of the reaction solvent and 50 μL of the standard solution were added to a 25 mL tube to prepare a blank stock solution. Two blank stock solutions were prepared to confirm the variation in preparation.

[Sample Preparation]

The stir bar of the reaction solution was removed, and the solution was centrifuged at 3000 rpm for 5 minutes (hereinafter, unless otherwise specified, centrifugation was performed using a himac CF 9RX manufactured by Hitachi Koki at 3000 rpm for 5 minutes). 1.5 mL of the supernatant of this solution was taken into a 2 mL tube, and 100 μL of 1N hydrochloric acid was added (pH=1-2). After centrifuging this solution at 10000 rpm for 10 minutes, 1 mL of this solution was taken into a 50 mL tube, and 9 mL of ultrapure water was added thereto to prepare a sample solution.

Separately, 1.5 mL of the blank stock solution was taken into a 2 mL tube, and 100 μL of 1N hydrochloric acid was added (pH=1-2). After centrifuging this solution at 10000 rpm for 10 minutes, 1 mL of this solution was taken into a 50 mL tube, and 9 mL of ultrapure water was added thereto to prepare a blank solution. Two blank solutions were prepared to confirm the variation in preparation.

Table 1 shows the reagents used in the study, and Table 2 shows the containers and equipment used in the study.

[Table 1]

Table 1. The reagents used in the study

| reagent name | supplier | grade | note |
|---|---|---|---|
| FaSSIF buffer concentrate | Bio relevant | - | FaSSIF preparation buffer |
| FaSSIF/FeSSIF /FaSSGF | Bio relevant | - | Lecithin and sodium taurocholate powder (to be added to the above) |
| TMA hydrochloride | NACALAI TESQUE, INC. | guaranteed reagent (GR) | - |
| choline chloride | FUJIFILM Wako Pure Chemical Corporation | for biochemistry | - |
| L(+)-arginine | FUJIFILM Wako Pure Chemical Corporation | special grade | - |
| glycine | Tokyo Chemical Industry Co., Ltd. | for electrophoresis | - |
| L-(+)-lysine | Tokyo Chemical Industry Co., Ltd. | extra pure reagent (EP) | - |
| L-histidine | Tokyo Chemical Industry Co., Ltd. | guaranteed reagent (GR) | - |
| 1N hydrochloric acid | FUJIFILM Wako Pure Chemical Corporation | for volumetric Analysis | - |
| cation mixed standard solution (II) | KANTO CHEMICAL CO.,INC. | for ion chromatography | - |

[Table 2]

Table 2. The container and the equipment used in the study

| container/equipment | supplier | model No. | note |
|---|---|---|---|
| 25 mL Tube | IWAKI | 2362-025 | - |
| 2 mL Tube | eppendorf | 0030120.094 | - |
| 50 mL Tube | CORNING | 430921 | - |

(continued)

| Table 2. The container and the equipment used in the study | | | |
|---|---|---|---|
| container/equipment | supplier | model No. | note |
| Stirrer | MASUDA | SM-60N | - |
| Centrifuge | Hitachi Koki Co., Ltd. | Himac CF 9RX | used at 3000 rpm |
| | TOMY SEIKO Co., Ltd. | MX-200 | used at 10000 rpm |

[Measurement Conditions]

The analysis was conducted using an Integrion RFIC system with an electrochemical detector manufactured by Thermo Fischer Scientific under the following ion chromatography conditions.

Column: CS17 (4×250 mm)
Guard Column: CG17 (4×250 mm)
Detector: Conductivity Detector
Flow Rate: 1.0 mL/min
Eluent: Methanesulfonic acid aq. (Eluent Generator)
Gradient Conditions: Table 3

[Table 3]

Table 3

| time (min) | eluent concentration (mmol/L) |
|---|---|
| 0.000 | 3.00 |
| 9.000 | 3.00 |
| 9.100 | 7.00 |
| 21.900 | 7.00 |

Column Oven: 30°C
Injection Volume: 50 μL
Suppressor: CERS500e (4 mm)
Measurement Time: 22 minutes

[Evaluation Method]

Based on a separately prepared calibration curve (linearity) and the calculated detection/quantitation limits, the concentrations of TMA and choline in the sample solutions were calculated, and the adsorption ratio of each substance adsorbed to the zeolite, as well as the ratio of TMA adsorption ratio to choline adsorption ratio, were determined.

[Calibration Curve (Linearity) and Detection/Quantitation Limits]

2050 mg of arginine, 120 mg of choline chloride, and 80 mg of TMA hydrochloride were accurately weighed, and about 25 mL of ultrapure water was added thereto and stirred well for dissolution. Then, ultrapure water was added thereto to make a final volume of 50 mL. Using this solution, five points of solutions bracketing the concentration of each substance added to the sample solution were prepared and used as the calibration curve (linearity). Furthermore, for a solution diluted to a concentration at which TMA and choline could be detected and quantified, the relative standard deviation and linearity of each substance measured 6 times were confirmed, and the detection and quantitation limit values were calculated.

The calibration curve (linearity) and detection/quantitation limits were confirmed once every three months, and the most recent results were used for the evaluation.

Method of the Kinetic Study

[TMA/Choline/Arginine Three-Component Mixed Adsorption Kinetic Study]

A standard solution was prepared by accurately weighing 13.53 mg of arginine, 10.84 mg of choline chloride, and 7.42 mg of TMA hydrochloride into 2.0 mL of a 0.1N hydrochloric acid aqueous solution prepared by diluting 1N hydrochloric acid with ultrapure water, and shaking well for dissolution.

10 mg of zeolite was accurately weighed into a 25 mL tube, and 5 mL of FaSSIF was added. Then, a stir bar and 50 μL of the standard solution were added to this solution. Sampling was performed only once per solution, and the same number of reaction solutions as the number of samplings was prepared using the same procedure. After stirring for the desired time (3 min, 10 min, 30 min, 1 hr, 3 hrs, 6 hrs, 24 hrs, 48 hrs), sampling was performed according to the same procedure as in [Sample Preparation] of the in vitro adsorption study, and the time course of the adsorption amount of each component was determined from the concentrations of TMA, choline, and arginine in the supernatant at each time point. The adsorption amount (μg/mL) on the vertical axis of FIG. 3 is the value obtained by subtracting the concentration of each component in the sample solution from the concentration of each component in the blank solution.

[Choline Release Study]

12.38 g of sodium chloride, 8.94 g of sodium dihydrogen phosphate dihydrate, and 0.84 g of sodium hydroxide were weighed, and about 1.8 L of ultrapure water was added thereto and stirred well for dissolution. The pH was adjusted to 6.5 with 1 mol/L sodium hydroxide aqueous solution or 1 mol/L hydrochloric acid aqueous solution. Ultrapure water was added thereto to make a final volume of 2 L to obtain a phosphate buffer. 991.2 mg of choline chloride was added to 2 mL of the phosphate buffer to prepare a choline solution. 70 mg of zeolite was accurately weighed, and 5 mL of phosphate buffer and 50 μL of the above choline solution were added. The mixture was stirred overnight at room temperature and filtered to collect the choline-adsorbed zeolite, which was then dried at 40°C under reduced pressure for 30 min. The filtrate was sampled according to the same procedure as in [Sample Preparation] of the in vitro adsorption study, then further diluted 50-fold with ultrapure water, and the blank solution and sample solution were measured.

10 mg of the choline-adsorbed zeolite powder was accurately weighed, and 5 mL of phosphate buffer was added. Sampling was performed only once per solution, and the same number of reaction solutions as the number of samplings was prepared using the same procedure. After stirring for the desired time (30 min, 1 hr, 3 hrs, 8 hrs, 24 hrs), sampling was performed according to the same procedure as in [Sample Preparation] of the in vitro adsorption study, and the time course of the choline release amount was determined from the choline concentration in the supernatant at each time point. For the adsorption amount (μg/mL) on the vertical axis of FIG. 4, the value obtained by subtracting the choline concentration in the sample solution during overnight choline adsorption from the choline concentration in the blank solution was multiplied by 50/7 to obtain the adsorption amount at time 0, and the value obtained by subtracting the choline concentration in the sample solution at each time point of the release study from this was taken as the adsorption amount at each time point.

Hereinafter, each item in the tables indicates the following, respectively.

Maximum ring number: The maximum ring number of the zeolite used in the example.

Framework code: The framework code of the zeolite used in the example, which conforms to the code defining the framework structure of zeolites established by the IZA (International Zeolite Association). If the compound is not a zeolite, the name of the compound is stated.

Df (Å): The largest free sphere diameter of the zeolite used in the example. The numerical value of the largest free sphere diameter was referenced from the value of "that can diffuse along" in the "Maximum diameter of a sphere" part on each framework code page of the Zeolite Framework Types (https://asia.iza-structure.org/IZA-SC/ftc_table.php) by the Structure Commission of the International Zeolite Association (IZA-SC). If there are multiple numerical values for the a, b, and c axes, the largest one is referenced.

Channel dimensionality: The dimensionality of the channel of the zeolite used in the example.

Counter cation: The counter cation of the zeolite used in the example.

SAR: The molar ratio of $SiO_2$ to $Al_2O_3$ of the zeolite used in the example; for Examples 70 and 78, it is the molar ratio of $SiO_2$ to $Al_2O_3$ of the zeolite before surface treatment.

(In vitro) TMA adsorption ratio: The TMA adsorption ratio from the adsorption study conducted in the example.

(In vitro) Choline adsorption ratio: The choline adsorption ratio from the adsorption study conducted in the example.

(In vitro) TMA/Choline: The value obtained by dividing the TMA adsorption ratio by the choline adsorption ratio from the adsorption study conducted in the example.

FWHM in XRPD: The full width at half maximum (unit: ° (2θ)) of the largest peak existing in the range of 2θ = 7.9000° ± 0.5000° (hereinafter referred to as the 2θ=7.9° peak) in the X-ray powder diffraction (hereinafter referred to as XRPD) of the MFI-type zeolite used in the example.

Zeolite before acid treatment: The example number of the zeolite before the acid treatment conducted in the example.

Acid: The reagent name of the acid used in the example.

Acid treatment temperature: The temperature of the acid treatment conducted in the example.

Acid treatment time: The time of the acid treatment conducted in the example.

Na residual ratio: The residual ratio of Na in the zeolite after the acid treatment conducted in the example. The Na residual ratio was calculated from the Na/Al calibration curve in XRF, with the zeolite of Example 48 before ion exchange being 100%.

Zeolite used: The example number of the zeolite used for the surface treatment reaction in the example.

SAR of zeolite used: The molar ratio of $SiO_2$ to $Al_2O_3$ of the zeolite used for the surface treatment reaction in the example.

Surface treatment reactant: The surface treatment reactant used in the example.

Reaction condition: The reaction condition of the surface treatment reaction conducted in the example.

TMA AUC change ratio: The d9-TMA AUC change ratio of the zeolite-administered group from the vehicle group in the in vivo study using a choline-load fasted mouse model conducted in the example.

Choline AUC change ratio: The d9-choline AUC change ratio of the zeolite-administered group from the vehicle group in the in vivo study using a choline-load fasted mouse model conducted in the example.

Post-treatment after hydrothermal synthesis: The post-treatment method after hydrothermal synthesis conducted in the example.

Characteristics of XRPD pattern: The characteristics of the XRPD pattern of the zeolite used in the example.

External surface area: The external surface area of the zeolite used in the example.

SEM image: The SEM image of the zeolite used in the example.

Concomitant drug: The classification by physicochemical properties and name of the concomitant drug used in the example.

Molecular weight of the free form: The molecular weight of the free form of the concomitant drug used in the example.

pKa: The pKa of the concomitant drug used in the example. Described for acidic compounds.

pKa of conjugate acid: The pKa of the conjugate acid of the concomitant drug used in the example. Described for basic compounds.

Adsorption ratio to zeolite (%): The adsorption ratio (%) of the concomitant drug adsorbed by the zeolite used in the example.

Results

In Vitro Adsorption Study

For Examples 1, 26, and 34, and Comparative Examples 1-5 and 7-21, the characteristics of the zeolites such as maximum ring number and framework, and the TMA and choline adsorption ratios from the in vitro adsorption study are summarized in Table 4.

For zeolites with a maximum ring number of 8, it is considered that the pore diameter is smaller than the molecular diameter of TMA or choline, and thus adsorption of TMA or choline into the pores did not occur. Although choline adsorption was observed in Comparative Example 16, it is thought to be due to external surface adsorption.

For zeolites with a maximum ring number of 10, efficient adsorption of both choline and TMA occurred, with the exception of HEU-type zeolite. In particular, some MFI-type zeolite compounds completely adsorbed the TMA in the system (Examples 26 and 34), and these preferentially adsorbed TMA over choline. For HEU-type zeolite, the 10-membered ring pores are distorted, and the largest free sphere diameter is only 3.67 Å, so it is considered that TMA adsorption into the pores did not occur.

For zeolites with a maximum ring number of 12, both choline and TMA are adsorbed, but it is not TMA-selective. This is thought to be because the pore diameter is larger than TMA or choline, making it impossible to distinguish between TMA and choline.

For common adsorbents such as activated carbon (Comparative Example 1) and molecular sieves 4A (Comparative Examples 4 and 5), as well as the potassium adsorbent Lokelma® (Comparative Example 2) used as a drug for hyperkalemia, and clinoptilolite (Comparative Example 3) used in zeolite supplements in the United States, almost no TMA adsorption occurred.

[Table 4]

Table 4. Maximum ring number, framework, and adsorption ratio from the in vitro adsorption study

| | maximum ring number | framework code | Df (Å) | channel dimensionality | counter cation | SAR | TMA adsorption ratio | choline adsorption ratio | TMA/ choline |
|---|---|---|---|---|---|---|---|---|---|
| Comp. Ex. 1 | - | activated carbon | - | - | - | - | 2.7% | 2.3% | 1.20 |
| Comp. Ex. 2 | - | Lokelma® | - | - | - | - | 0.7% | 0.0% | - |
| Comp. Ex. 3 | 10 | HEU | 3.67 | 2-dimension | Na, K, Ca | 6.0 | 3.9% | 1.1% | 3.45 |
| Comp. Ex. 15 | 8 | CHA | 3.72 | 3-dimension | H | 20.0 | 2.4% | 1.9% | 1.21 |
| Comp. Ex. 16 | | AFX | 3.73 | 3-dimension | H | 7.0 | 0.9% | 22.8% | 0.04 |
| Comp. Ex. 17 | | AEI | 3.84 | 3-dimension | H | 10.0 | 3.0% | 4.9% | 0.60 |
| Comp. Ex. 4 | | LTA | 4.21 | 3-dimension | Na | 2.0 | 0.4% | 1.2% | 0.34 |
| Comp. Ex. 5 | | | | | Na | 2.6 | 1.8% | 1.2% | 1.49 |
| Ex. 1 | 10 | FER | 4.69 | 2-dimension | K | 18.0 | 70.8% | 0.2% | 341.61 |
| Ex. 26 | | MFI | 4.70 | 3-dimension | H | 41.1 | 100.0% | 33.1% | 3.02 |
| Ex. 34 | | | | | H | 59.6 | 100.0% | 33.1% | 3.02 |
| Comp. Ex. 18 | | MWW | 4.92 | 2-dimension | H | 20.9 | 84.9% | 52.8% | 1.61 |
| Comp. Ex. 19 | | | | | H | 31.4 | 82.2% | 60.0% | 1.37 |
| Comp. Ex. 20 | | MEL | 5.19 | 3-dimension | H | 26.5 | 86.9% | 83.2% | 1.04 |
| Comp. Ex. 21 | | | | | H | 43.0 | 88.4% | 90.2% | 0.98 |

(continued)

| Table 4. Maximum ring number, framework, and adsorption ratio from the in vitro adsorption study | | | | | | | | | |
|---|---|---|---|---|---|---|---|---|---|
| | maximum ring number | framework code | Df (Å) | channel dimensionality | counter cation | SAR | TMA adsorption ratio | choline adsorption ratio | TMA/ choline |
| Comp. Ex. 7 | 12 | Beta | 5.94 | 3-dimension | $NH_4$ | 25.0 | 85.6% | 92.0% | 0.93 |
| Comp. Ex. 8 | | | | | H | 40.0 | 67.5% | 87.0% | 0.78 |
| Comp. Ex. 9 | | MOR | 6.45 | 2-dimension | $NH_4$ | 20.0 | 72.4% | 92.0% | 0.79 |
| Comp. Ex. 10 | | | | | H | 30.0 | 92.5% | 94.1% | 0.98 |
| Comp. Ex. 11 | | FAU | 7.35 | 3-dimension | Na | 6.0 | 3.5% | 4.5% | 0.78 |
| Comp. Ex. 12 | | | | | H | 30.0 | 23.8% | 41.8% | 0.57 |
| Comp. Ex. 13 | | | | | H | 60.0 | 22.4% | 39.1% | 0.57 |
| Comp. Ex. 14 | | | | | H | 2000.0 | 24.0% | 20.6% | 1.17 |

For the MFI-type zeolites (maximum ring number: 10) of Examples 2-35 and 37-64, and Comparative Examples 6 and 22, the Silical/Alumina Ratio (hereinafter referred to as SAR), counter cation, the full width at half maximum of the 2θ=7.9° peak in XRPD, and the results of the in vitro adsorption study are summarized in Tables 5-1 and 5-2.

When the SAR was less than 33.0, the TMA/choline selectivity ranged from about 1 to 3. When the SAR was 33.0 or more and 79.4 or less, the TMA/choline selectivity was generally high, and the TMA adsorption ratio was also high. When the SAR exceeded 79.4, the TMA/choline selectivity was generally high, but the TMA adsorption ratio showed a tendency to decrease compared to the range of 33.0 to 79.4.

[Table 5-1]

| Table 5-1.SAR, counter cation, FWHM in XRPD, and adsorption ratio from the in vitro adsorption study of MFI-type zeolite | | | | | | |
|---|---|---|---|---|---|---|
| | SAR | counter cation | FWHM in XRPD | TMA adsorption ratio | choline adsorption ratio | TMA/ choline |
| Comp. Ex. 6 | 24.0 | H | 0.1774 | 86.6% | 91.9% | 0.95 |
| Comp. Ex. 22 | 26.0 | H | 0.1545 | 89.7% | 88.9% | 1.01 |
| Ex. 12 | 31.0 | H | 0.1239 | 100.0% | 36.6% | 2.73 |
| Ex. 24 | 32.0 | H | 0.1047 | 96.6% | 38.1% | 2.54 |
| Ex. 13 | 33.0 | H | 0.1272 | 100.0% | 22.0% | 4.55 |
| Ex. 56 | 33.9 | H | 0.1141 | 100.0% | 32.1% | 3.12 |
| Ex. 8 | 35.0 | H | 0.1110 | 100.0% | 27.9% | 3.59 |
| Ex. 23 | 35.0 | H | 0.1260 | 100.0% | 36.4% | 2.75 |
| Ex. 25 | 35.0 | H | 0.1135 | 100.0% | 19.6% | 5.10 |
| Ex. 14 | 40.0 | H | 0.1112 | 100.0% | 36.7% | 2.72 |
| Ex. 58 | 40.8 | H | 0.1099 | 100.0% | 23.8% | 4.20 |
| Ex. 20 | 41.0 | H | 0.1093 | 97.0% | 24.6% | 3.95 |

(continued)

| Table 5-1.SAR, counter cation, FWHM in XRPD, and adsorption ratio from the in vitro adsorption study of MFI-type zeolite | | | | | | |
|---|---|---|---|---|---|---|
| | SAR | counter cation | FWHM in XRPD | TMA adsorption ratio | choline adsorption ratio | TMA/ choline |
| Ex. 26 | 41.1 | H | 0.1269 | 100.0% | 33.1% | 3.02 |
| Ex. 15 | 41.3 | H | 0.1180 | 100.0% | 32.0% | 3.13 |
| Ex. 57 | 41.4 | H | 0.1068 | 100.0% | 33.7% | 2.97 |
| Ex. 11 | 42.1 | H | 0.1087 | 100.0% | 30.3% | 3.30 |
| Ex. 10 | 42.2 | H | 0.1178 | 100.0% | 30.3% | 3.30 |
| Ex. 27 | 42.3 | H | 0.1079 | 100.0% | 28.9% | 3.46 |
| Ex. 9 | 42.4 | H | 0.1088 | 100.0% | 39.2% | 2.55 |
| Ex. 16 | 48.2 | H | 0.1124 | 100.0% | 16.3% | 6.15 |
| Ex. 60 | 48.7 | H | 0.1044 | 96.9% | 33.5% | 2.89 |
| Ex. 39 | 49.0 | H | 0.0908 | 100.0% | 20.2% | 4.95 |
| Ex. 28 | 50.2 | H | 0.1014 | 90.0% | 36.1% | 2.49 |
| Ex. 41 | 55.2 | H | 0.1130 | 91.5% | 7.3% | 12.59 |
| Ex. 61 | 56.2 | H | 0.1028 | 96.9% | 28.4% | 3.41 |
| Ex. 21 | 56.7 | H | 0.1092 | 100.0% | 31.8% | 3.15 |
| Ex. 38 | 57.2 | H | 0.1094 | 89.8% | 31.1% | 2.89 |
| Ex. 44 | 57.2 | H | 0.0983 | 92.1% | 10.9% | 8.47 |
| Ex. 43 | 57.2 | H | 0.1046 | 100.0% | 28.6% | 3.49 |
| Ex. 17 | 57.6 | H | 0.1070 | 100.0% | 19.5% | 5.12 |
| Ex. 51 | 58.6 | H | 0.1053 | 100.0% | 29.2% | 3.43 |
| Ex. 34 | 59.6 | H | 0.1027 | 100.0% | 33.1% | 3.02 |
| Ex. 37 | 60.6 | H | 0.0945 | 94.6% | 13.0% | 7.29 |
| Ex. 49 | 60.9 | H | 0.1054 | 100.0% | 34.0% | 2.94 |

[Table 5-2]

| Table 5-2. SAR, counter cation, FWHM in XRPD, and adsorption ratio from the in vitro adsorption study of MFI-type zeolite ~continued | | | | | | |
|---|---|---|---|---|---|---|
| | SAR | counter cation | FWHM in XRPD | TMA adsorption ratio | choline adsorption ratio | TMA/ choline |
| Ex. 33 | 60.9 | H | 0.1056 | 91.7% | 23.5% | 3.90 |
| Ex. 32 | 61.2 | H | 0.1124 | 100.0% | 39.4% | 2.54 |
| Ex. 50 | 61.7 | H | 0.1108 | 100.0% | 18.5% | 5.40 |
| Ex. 29 | 61.9 | H | 0.1018 | 100.0% | 25.7% | 3.88 |
| Ex. 30 | 62.1 | H | 0.1037 | 96.5% | 20.1% | 4.80 |
| Ex. 46 | 62.6 | H | 0.1081 | 100.0% | 26.8% | 3.73 |
| Ex. 53 | 62.7 | H | 0.1383 | 92.0% | 36.1% | 2.55 |
| Ex. 48 | 63.2 | Na | 0.1095 | 91.4% | 15.1% | 6.04 |
| Ex. 52 | 63.4 | H | 0.1113 | 100.0% | 22.5% | 4.45 |
| Ex. 42 | 63.7 | H | 0.1045 | 92.0% | 21.5% | 4.27 |

(continued)

Table 5-2. SAR, counter cation, FWHM in XRPD, and adsorption ratio from the in vitro adsorption study of MFI-type zeolite ~continued

| | SAR | counter cation | FWHM in XRPD | TMA adsorption ratio | choline adsorption ratio | TMA/ choline |
|---|---|---|---|---|---|---|
| Ex. 47 | 64.4 | H | 0.0979 | 100.0% | 20.7% | 4.82 |
| Ex. 18 | 64.8 | H | 0.1010 | 96.8% | 15.1% | 6.40 |
| Ex. 59 | 65.3 | H | 0.1060 | 92.2% | 28.0% | 3.30 |
| Ex. 45 | 65.8 | H | 0.1094 | 87.1% | 20.3% | 4.30 |
| Ex. 35 | 65.4 | Na | 0.1098 | 83.0% | 24.7% | 3.36 |
| Ex. 62 | 66.5 | H | 0.1122 | 96.6% | 31.3% | 3.08 |
| Ex. 54 | 67.2 | H | 0.1104 | 88.7% | 21.2% | 4.19 |
| Ex. 40 | 67.6 | H | 0.1004 | 100.0% | 17.0% | 5.87 |
| Ex. 31 | 67.8 | H | 0.0994 | 100.0% | 25.6% | 3.91 |
| Ex. 22 | 71.8 | H | 0.1208 | 100.0% | 23.4% | 4.28 |
| Ex. 63 | 72.8 | H | 0.1043 | 96.5% | 32.2% | 2.99 |
| Ex. 64 | 78.3 | H | 0.1180 | 96.3% | 23.3% | 4.14 |
| Ex. 19 | 78.5 | H | 0.0984 | 96.7% | 17.6% | 5.49 |
| Ex. 55 | 79.4 | H | 0.1096 | 100.0% | 26.7% | 3.74 |
| Ex. 6 | 578.0 | H | 0.1280 | 74.7% | 3.4% | 22.01 |
| Ex. 4 | 874.0 | H | 0.1114 | 80.0% | 15.3% | 5.23 |
| Ex. 2 | 1000.0 | H | 0.1428 | 62.8% | 10.7% | 5.86 |
| Ex. 3 | 1600.0 | H | 0.1162 | 60.4% | 12.4% | 4.86 |
| Ex. 7 | 57300.0 | H | 0.1246 | 64.9% | 3.3% | 19.83 |
| Ex. 5 | 129000.0 | H | 0.1169 | 80.7% | 0.9% | 88.51 |

FIG. 1 is a histogram showing the distribution of the number of compounds with respect to the full width at half maximum of the peak existing in the range of the 2θ=7.9° in XRPD for MFI-type zeolites with a TMA adsorption ratio of less than 60.0% or a choline adsorption ratio of more than 40.0%, and for MFI-type zeolites with a TMA adsorption ratio of 60.0% or more and a choline adsorption ratio of 40.0% or less.

In MFI-type zeolites, it is considered that not only SAR correlates with TMA/choline selectivity, but also the crystal quality, i.e., the full width at half maximum of the peak in XRPD, correlates with TMA/choline selectivity. For compounds with a TMA adsorption ratio of 60.0% or more and a choline adsorption ratio of 40.0% or less, the frequency of smaller full width at half maximum values was higher, and all were 0.15° or less. On the other hand, for compounds with a TMA adsorption ratio of less than 60.0% or a choline adsorption ratio of more than 40.0%, the peak full width at half maximum exceeded 0.15°.

From the Scherrer equation, the smaller the full width at half maximum of the peak, the larger the crystallite size of the zeolite. It is assumed that zeolites with larger crystallite sizes are more significantly affected by the intracrystalline pore diffusion rate, and it is considered that TMA adsorption is preferred over choline.

Acid Treatment

Using the zeolite of Example 50 (maximum ring number: 10, framework code: MFI), the stability of the framework under acidic conditions was confirmed.

The results are summarized in Table 6. Under all conditions, the full width at half maximum of XRPD did not change significantly, suggesting that the MFI framework structure was maintained. The TMA adsorption ratio did not decrease due to acid treatment, and there was no significant change in the choline adsorption ratio either.

[Table 6]

| Table 6. Acid Treatment to Confirm the Framework Stability | | | | | | | |
|---|---|---|---|---|---|---|---|
| | zeolite before acid treatment | acid | acid treatment temperature | FWHM in XRPD | TMA adsorption ratio | choline adsorption ratio | TMA/ choline |
| Ex. 50 | - | - | - | 0.1108 | 100.0% | 18.5 | 5.40 |
| Ex. 65 | Ex. 50 | 1 mol/L nitric acid aq. solu-tion | room tem-perature | 0.1094 | 100.0% | 36.5 | 2.74 |
| Ex. 66 | Ex. 50 | conc. nitric acid | room temperature | 0.1048 | 100.0% | 31.4 | 3.19 |
| Ex. 67 | Ex. 50 | conc. nitric acid | 100°C | 0.1145 | 100.0% | 18.7 | 5.34 |

The zeolite of Example 48 (maximum ring number: 10, framework code: MFI), which had only dried after the hydrothermal synthesis, was subjected to different acid treatment procedures to investigate the effect on the Na/H exchange efficiency (Table 7).

From the Na residual ratios of the zeolites of Examples 68 and 69, it is considered that the Na/H cation exchange reaction almost reached equilibrium state with about 1 hour of stirring in a 1 mol/L nitric acid aqueous solution at room temperature. The Na residual ratio is comparable to the proton-type zeolite prepared by ammonium ion exchange and calcination (Example 49).

[Table 7]

Table 7. Consideration of condition of Na/H exchange by acid treatment

| | zeolite before acid treatment | acid | acid treatment temperature | acid treatment time | Na residual ratio | SAR | FWHM in XRPD | TMA adsorption ratio | choline adsorption ratio | TMA/ choline |
|---|---|---|---|---|---|---|---|---|---|---|
| Ex. 48 | - | - | - | - | 100% | 63.2 | 0.1095 | 91.4% | 15.1% | 6.04 |
| Ex. 68 | Ex. 48 | 1 mol/L nitric acid aq. solution | room temperature | 1 h | 8.2% | 59.1 | 0.1001 | 100.0% | 36.8% | 2.72 |
| Ex. 69 | Ex. 48 | 1 mol/L nitric acid aq. solution | room temperature | 2 h | 8.2% | 60.1 | 0.1036 | 100.0% | 39.6% | 2.52 |
| Ex. 49 | Ex. 48 | - | - | - | 6.3% | 60.8 | 0.1054 | 100.0% | 34.0% | 2.94 |

Surface Treatment

The change in adsorption capacity due to surface treatment on the zeolite was confirmed.

The results are summarized in Table 8.

For all compounds, the in vitro TMA/choline adsorption selectivity was improved by surface treatment. Regarding small molecule-based surface treatment reagents (Comparative Example 23, Examples 70 and 72-76), the TMA adsorption ratio decreased only for the zeolites of Examples 74 and 76, which were treated with a surface treatment reagent containing an octadecyl group, a long-chain alkyl chain. One possible reason is that the long alkyl chains may fill the zeolite pores. Regarding polymer-based surface treatment reagents (Examples 77-88), the change in choline adsorption ratio was small when the reaction was carried out by the vapor method (Examples 77 and 80), suggesting that the surface treatment reaction was insufficient with the vapor method. It depended on the polymer surface treatment reagent whether the toluene-2 condition or the neat condition was better.

[Table 8]

Table 8. Surface treatment of MFI-type zeolite

| | zeolite used | SAR of zeolite used | surface treatment reagent | reaction condition | TMA adsorption ratio | choline adsorption ratio | TMA/ choline |
|---|---|---|---|---|---|---|---|
| Comp. Ex. 6 | - | - | - | - | 86.6% | 91.1% | 0.95 |
| Comp. Ex. 23 | Comp. Ex. 6 | 24.0 | HMDS | neat | 96.7% | 72.6% | 1.33 |
| Ex. 34 | - | - | - | - | 100.0% | 33.1% | 3.02 |
| Ex. 70 | Ex. 34 | 59.6 | HMDS | neat | 100.0% | 11.5% | 8.68 |
| Ex. 50 | - | - | - | - | 100.0% | 18.5% | 5.40 |
| Ex. 72 | Ex. 50 | 61.7 | chlorotrimethylsilane | toluene-1 | 100.0% | 28.4% | 3.52 |
| Ex. 73 | | | dichlorohexylmethylsilane | toluene-1 | 100.0% | 15.2% | 6.60 |
| Ex. 74 | | | dichloromethyloctadecylsilane | toluene-1 | 86.2% | 8.9% | 9.67 |
| Ex. 75 | | | Trichloroethylsilane | toluene-1 | 100.0% | 10.0% | 10.04 |
| Ex. 76 | | | trichlorooctadecylsilane | toluene-1 | 78.7% | 10.4% | 7.55 |
| Ex. 77 | | | MS-51 | vapor | 100.0% | 31.3% | 3.20 |
| Ex. 78 | | | MS-51 | toluene-2 | 100.0% | 3.2% | 31.36 |
| Ex. 79 | | | MS-51 | neat | 100.0% | 8.2% | 12.13 |
| Ex. 80 | | | MS-56 | vapor | 100.0% | 33.7% | 2.97 |
| Ex. 81 | | | MS-56 | toluene-2 | 100.0% | 9.0% | 11.08 |
| Ex. 82 | | | MS-56 | neat | 100.0% | 2.6% | 38.14 |
| Ex. 83 | | | MS-57 | toluene-2 | 100.0% | 13.1% | 7.62 |
| Ex. 84 | | | MS-57 | neat | 100.0% | 5.8% | 17.34 |
| Ex. 85 | | | MS-58B15 | toluene-2 | 100.0% | 17.3% | 5.77 |
| Ex. 86 | | | MS-58B15 | neat | 100.0% | 8.5% | 11.73 |
| Ex.87 | | | MS-58B30 | toluene-2 | 100.0% | 15.4% | 6.48 |
| Ex. 88 | | | MS-58B30 | neat | 100.0% | 5.9% | 16.99 |

Counter cation

Using the zeolites of Examples 34-36, 71, and 89 (maximum ring number: 10, framework code: MFI), which are produced under the same hydrothermal synthesis conditions and have different counter cations, the effect of the difference in counter cations on the adsorption capacity was investigated (Table 9).

When the counter cation was changed from $H^+$ to $Li^+$, $Na^+$, $K^+$, or $NH_4^+$, the in vitro TMA/choline adsorption selectivity did not decrease significantly in any case. For oral use, the proton form is preferable as it is less likely to affect the body due to excessive ion intake.

[Table 9]

| Table 9. Counter cation | | | | |
|---|---|---|---|---|
| | counter cation | TMA adsorption ratio | choline adsorption ratio | TMA/ choline |
| Ex. 34 | H | 100.0% | 33.1% | 3.02 |
| Ex. 89 | $NH_4$ | 90.1% | 6.7% | 13.5 |
| Ex. 36 | Li | 88.0% | 37.1% | 2.37 |
| Ex. 35 | Na | 83.0% | 24.7% | 3.36 |
| Ex. 71 | K | 89.3% | 24.1% | 3.69 |

BET Specific Surface Area, Particle Size Distribution

For the zeolites of Examples 5, 11, 19, 26, 34, 38, 49, 52, and 53, and Comparative Examples 6 and 22 (maximum ring number: 10, framework code: MFI), the results of the BET specific surface area and external surface area obtained from the BET surface adsorption study are shown in Table 10.

Compounds with an external surface area exceeding 30 $m^2/g$ showed less TMA/choline adsorption selectivity. In adsorption using the internal pores of the zeolite, the selectivity of TMA adsorption over choline is high because the difference in molecular size is distinguished. However, for compounds with a large external surface area, the contribution of adsorption on the external surface of the zeolite particles, which does not distinguish the difference in molecular size, becomes larger and the TMA/choline selectivity is considered to decrease.

[Table 10]

| Table 10. BET specific surface area | | | | | | |
|---|---|---|---|---|---|---|
| | SAR | BET specific surface area ($m^2/g$) | external surface area ($m^2/g$) | TMA adsorption ratio | choline adsorption ratio | TMA/ choline |
| Comp. Ex. 6 | 24.0 | 358 | 36.2 | 86.6% | 91.1% | 0.95 |
| Comp. Ex. 22 | 26.0 | 356 | 33.4 | 89.7% | 88.9% | 1.01 |
| Ex. 19 | 78.5 | 280 | 25.6 | 96.7% | 17.6% | 5.49 |
| Ex. 53 | 62.7 | 192 | 24.8 | 92.0% | 36.1% | 2.55 |
| Ex. 52 | 63.4 | 207 | 23.8 | 100.0% | 22.5% | 4.45 |
| Ex. 38 | 57.2 | 263 | 14.8 | 89.8% | 31.1% | 2.89 |
| Ex. 34 | 59.6 | 313 | 13.3 | 100.0% | 33.1% | 3.02 |
| Ex. 49 | 60.8 | 297 | 12.7 | 100.0% | 34.0% | 2.94 |
| Ex. 11 | 42.1 | 380 | 12.6 | 100.0% | 30.3% | 3.30 |
| Ex. 26 | 41.1 | 336 | 7.8 | 100.0% | 33.1% | 3.02 |
| Ex. 5 | 129000 | 418 | 6.5 | 80.7% | 0.9% | 88.51 |

Particle Size Distribution

For the zeolites of Examples 26 and 34, and Comparative Examples 6 and 22 (maximum ring number: 10, framework code: MFI), the results of the particle size distribution are shown in Table 11-1 and FIG. 2.

In Comparative Examples 6 and 22, there are two peaks in the frequency distribution, with the first peak being 1 $\mu m$ or less, which is reflected in the small D10 (< 2.5 $\mu m$). On the other hand, in Examples 26 and 34, there is one peak in the frequency distribution, and the D10 is large (> 2.5 $\mu m$). Similarly, the median diameters of Examples 26 and 34 are larger than the values of Comparative Examples 6 and 22. In Comparative Examples 6 and 22, the external surface area is larger than in Examples 26 and 34, which is thought to reflect the difference in particle size. Also, the small particle size is correlated with the low selectivity of TMA adsorption over choline.

[Table 11-1]

| Table 11-1. Particle size distribution | | | | | | |
|---|---|---|---|---|---|---|
| | SAR | D10 (μm) | median diameter (μm) | TMA adsorption ratio | choline adsorption ratio | TMA/ choline |
| Comp. Ex. 6 | 24.0 | 2.35 | 5.00 | 86.6% | 91.1% | 0.95 |
| Comp. Ex. 22 | 26.0 | 0.55 | 4.83 | 89.7% | 88.9% | 1.01 |
| Ex. 26 | 41.1 | 5.37 | 11.78 | 100.0% | 33.1% | 3.02 |
| Ex. 34 | 59.6 | 6.29 | 12.27 | 100.0% | 33.1% | 3.02 |

[In vitro adsorption study using a small amount of zeolite]

An in vitro adsorption study was conducted using a small amount of the zeolites from Examples 26 and 34. The in vitro adsorption study was performed using the same method as previously described in [In Vitro Adsorption Study], except that the amount of zeolite was changed from 10 mg to 3 mg or 1 mg. The results are shown in Table 11-2. For zeolites with a high TMA adsorption ratio and TMA/choline selectivity, it is considered that lowering the amount of zeolite in the in vitro adsorption study improves the TMA/choline adsorption selectivity.

[Table 11-2]

| Table 11-2. In vitro adsorption study using a small amount of zeolite | | | | |
|---|---|---|---|---|
| | amount of zeolite | TMA adsorption ratio | choline adsorption ratio | TMA/ choline |
| Ex. 26 | 3 mg | 71.2% | 11.7% | 6.09 |
| | 1 mg | 33.0% | 5.2% | 6.35 |
| Ex. 34 | 3 mg | 82.8% | 5.1% | 16.22 |
| | 1 mg | 43.3% | 3.2% | 13.54 |

Kinetic Study

For the zeolites of Example 26, Example 34, and Comparative Example 6 (maximum ring number: 10, framework code: MFI), the time course of the adsorption amount of TMA, choline, and arginine is shown in FIG. 3.

The TMA adsorption reached a plateau in a short time for all compounds. Arginine was adsorbed by Comparative Example 6, but not by Examples 26 and 34. The adsorption of choline reached a plateau within 3 minutes of the start of the adsorption study for Comparative Example 6, but did not reach a plateau for Examples 26 and 34.

The adsorption process of each component onto the zeolite is thought to proceed in the order of (1) surface adsorption, (2) adsorption into the pores of the surface layer part, and (3) intra-pore diffusion into the deep layer part. Even if (1) is fast, if the adsorption and diffusion into the pores of (2) and (3) are slow, that becomes the rate-determining step of adsorption. The difference in the adsorption rates of TMA and choline in Examples 26 and 34 is thought to reflect the difference in the rates of the intra-pore adsorption processes of (2) and (3). That is, the rate for choline, which has a larger molecular size than TMA, became slower than that for TMA in the intra-pore adsorption and diffusion process due to steric hindrance, etc. In Comparative Example 6, choline, TMA, and arginine were mainly adsorbed by external surface adsorption, and no difference in adsorption rates was observed. However, in Examples 26 and 34, which have larger particle sizes, the influence of the intra-pore diffusion rate became larger, and it is considered that the choline adsorption rate became significantly slower. Also, arginine, which is an even larger molecule than choline, was not adsorbed by Examples 26 and 34, where the external surface area is small and intra-pore adsorption is dominant. It has been confirmed from the particle size distribution and external surface area that the particle sizes of Examples 26 and 34 are larger than that of Comparative Example 6.

For Comparative Examples 6 and 22, and Examples 26 and 34, the time course of the release amount of choline is shown in FIG. 4. In the release study, Comparative Examples 6 and 22 had a large amount of released choline, and the release also reached a plateau within 1 hour. On the other hand, Examples 26 and 34 had a small amount of released choline, and it took about 8 hours for the release to reach a plateau. In Comparative Examples 6 and 22, which have large external surface areas, choline was mainly adsorbed on the external surface, so the contribution of the intra-pore diffusion process of choline was small, and it is considered that re-release proceeded rapidly.

<Calculation>

To support the reasons for the difference in adsorption rates of TMA and choline to MFI zeolite, as well as the TMA/choline selectivity, an analysis of the adsorption process was performed by first-principles calculation based on density functional theory.

Calculation Method

Application and Version Used: VASP (Vienna Ab initio Simulation Package) ver. 6.3.2 (Calculation performed by Masayoshi Mikami, Mitsubishi Chemical Corporation)

Density Functional Theory: Exchange-correlation energy was calculated using the generalized gradient approximation (GGA-PBE), with dispersion force correction by DFT+D3 (Grimm). The PAW method was used (plane-wave basis cutoff energy 400 eV). K-point sampling was at the $\Gamma$ point, the SCF calculation threshold was $1.0\times10^{-6}$ eV, and the force threshold for structure optimization was $1.0\times10^{-5}$ eV/Å.

Calculation Model

Periodic slab models corresponding to the (010) surface and the (100) surface of MFI zeolite were prepared in accordance with the data described in a paper (ACS Catalysis 2020 10, 3297-3312). When placing TMA and choline on the surface models, TMA was treated as a neutral molecule, and choline was treated as a monovalent cation molecule. For calculations including the monovalent cation state, a calculation with a uniformly distributed background charge of the opposite sign is necessary to maintain the overall charge neutrality of the system. In VASP, this calculation with a distributed background charge can be automatically performed by adjusting the number of electron occupations (NELECT) to correspond to the monovalent cation state. The central position in the thickness direction (z-axis direction) of the zeolite layer of each surface model was taken as the reference (defined as z=0), and the nitrogen atom of the TMA molecule or the nitrogen atom of the choline molecule was placed there. The stable configuration of the molecule was determined by optimizing the atomic positions other than the z-coordinate of the nitrogen atom (internal adsorption). Furthermore, the z-coordinate of the nitrogen was moved in 1Å increments toward the surface, and each time, the atomic positions other than the z-coordinate of the nitrogen were optimized. A point near the surface (z=8-11Å) where the total energy of each molecule was lowest was set to 0 kcal/mol and used as the energy reference point. The point where the energy barrier was highest when entering the zeolite interior from the surface (z=5-7Å) was defined as the highest point of the energy barrier.

Calculation Results

The energy profiles for the adsorption process of TMA or choline from the (010) and (100) surfaces are shown in FIG. 5. The cross-sectional structures of the zeolite layer at the energy reference point, the highest point of the energy barrier, and during internal adsorption are shown in FIG. 6-1, FIG. 6-2, FIG. 6-3, and FIG. 6-4. The structure at the highest point of the energy barrier of the (010) surface model from the z-axis perspective is shown in FIG. 7.

The energy barrier for entering the zeolite interior from the (100) surface is about 5 kcal/mol for both the TMA molecule and the choline molecule (energy difference within quantum chemical accuracy of 1 kcal/mol), and it is considered that there is no difference in adsorption rates. On the other hand, the energy barrier for entering the zeolite interior from the (010) surface is about 10 kcal/mol for the TMA molecule, whereas it is about 15 kcal/mol for the choline molecule, and it was predicted that the adsorption rate of the choline molecule would be slower than that of TMA. This calculation result supports the experimental result that the (010) surface is a crystal surface with high TMA/choline selectivity (results from "SEM image, external surface area and in vitro/vivo efficacy" described later). The tertiary amine TMA has a trigonal pyramidal molecular structure with nitrogen at the apex and can pass through the elliptical pore shape by aligning its minor and major axes. In contrast, the quaternary ammonium ion choline has a tetrahedral shape centered on nitrogen and cannot fit the elliptical shape in any orientations, resulting in steric hindrance during passage, as can be inferred from FIG. 7.

The 3D channel system of MFI zeolite is composed of straight [010] channels and sinusoidal [100] channels connected to each other, and molecules diffuse in the [001] direction by alternately using the [010] and [100] channels. Since the steric hindrance in the entry process of choline into (010) surface is assumed to occur similarly in the inter-pore movement across the (010) surface inside the crystal, the diffusion rate of choline is assumed to be slower than that of TMA in the [010] and [001] directions. This is consistent with the experimental result that coffin-shaped crystals, which are long in the [001] direction, have high TMA/choline selectivity (results from "SEM image, external surface area and in vitro/vivo efficacy" described later) and also with the result of the kinetic study that the choline adsorption rate is slow in Examples 26 and 34, which have large particle sizes (results from the previously described "Kinetic Study").

< In Vivo Study Using a Choline-Load Fasted Mouse Model>

Test Method

Seven-week-old male C57BL/6 mice were fasted for 12 hours. Then, d9-choline chloride (Toronto Research Chemicals Inc.) dissolved in distilled water and zeolite suspended in a 0.5 w/v% methylcellulose 400 solution, sterilized (FUJIFILM Wako Pure Chemical Corporation), were simultaneously administered orally at doses of 78.78 mg/kg (60 mg/kg as d9-choline) and 500-2000 mg/kg, respectively. Blood was collected from the tail vein at 1, 2, 4, 6, 8, and 10 hours after administration, and the concentrations of d9-choline, d9-TMA, and d9-TMAO in the plasma were measured by LC/MS/MS.

The time courses of the plasma concentrations of d9-choline, d9-TMA, and d9-TMAO were plotted with the plasma concentration on the vertical axis and the time after administration on the horizontal axis, and the Areas Under the Curves (hereinafter referred to as AUC) up to 10 hours after administration were calculated. The pharmacological effect of the zeolite was confirmed by comparing the AUC of the zeolite non-administered group (hereinafter referred to as the vehicle group) and that of the administered group,. In addition, the pharmacological effects of each zeolite were compared using the AUC change ratio, which was normalized by dividing the AUC change of zeolite administered group from the vehicle group by the vehicle group AUC. The AUC change ratio was calculated from the following formula.

[Eq. 1]

$$\text{AUC change ratio (\%)} = \left( \frac{\text{AUC (μmol·h/L) of zeolite-administered group} - \text{AUC (μmol·h/L) of vehicle group}}{\text{AUC (μmol·h/L) of vehicle group}} \right) \times 100$$

Results

In Vivo Study Using a Choline-Load Fasted Mouse Model

For the zeolites of Examples 1, 5, 9, 13, 15, 26, 34, and 55, and Comparative Examples 8, 6, and 22, the compound profiles and the results of the in vivo study using a choline-load fasted mouse model are summarized in Table 12.

All compounds were tested at 500 mg/kg dose (hereinafter, unless otherwise specified, the results of the in vivo study using a choline-load fasted mouse model refer to those conducted at 500 mg/kg dose).

All compounds decreased the AUC of d9-TMA compared to the vehicle group. For the zeolites of Comparative Examples 6, 8, and 22, which have low TMA/choline selectivity in the in vitro adsorption study, the AUC reduction ratio of d9-choline is comparable to or greater than the AUC reduction ratio of d9-TMA. For compounds with high TMA/choline selectivity and high TMA adsorption capacity in the in vitro adsorption study, the AUC reduction ratio of d9-TMA is high, and the d9-choline AUC reduction ratio is low. Choline is a nutrient, and compounds that inhibit the absorption of choline are undesirable. Note that the "AUC reduction ratio" refers to the value obtained by multiplying the "AUC change ratio" by minus one.

[Table 12]

Table 12. In vitro/vivo efficacy of various zeolites

| | maximum ring number | framework code | counter cation | SAR | In vitro TMA adsorption ratio | In vitro choline adsorption ratio | In vitro TMA/ choline | TMA AUC change ratio | choline AUC change ratio |
|---|---|---|---|---|---|---|---|---|---|
| Comp. Ex. 8 | 12 | Beta | H | 40.0 | 67.5% | 87.0% | 0.78 | -38.4% | -46.4% |
| Comp. Ex. 6 | 10 | MFI | H | 24.0 | 86.6% | 91.1% | 0.95 | -44.3% | -49.3% |

(continued)

| Table 12. In vitro/vivo efficacy of various zeolites | | | | | | | | | |
|---|---|---|---|---|---|---|---|---|---|
| | maximum ring number | framework code | counter cation | SAR | In vitro TMA adsorption ratio | In vitro choline adsorption ratio | In vitro TMA/ choline | TMA AUC change ratio | choline AUC change ratio |
| Comp. Ex. 22 | 10 | MFI | H | 26.0 | 89.7% | 88.9% | 1.01 | -43.6% | -42.4% |
| Ex. 9 | 10 | MFI | H | 42.4 | 100.0% | 39.2% | 2.55 | -39.4% | -10.0% |
| Ex. 34 | 10 | MFI | H | 59.6 | 100.0% | 33.1% | 3.02 | -54.2% | -27.2% |
| Ex. 26 | 10 | MFI | H | 41.1 | 100.0% | 33.1% | 3.02 | -54.6% | -7.3% |
| Ex. 15 | 10 | MFI | H | 41.3 | 100.0% | 32.0% | 3.13 | -37.4% | 5.8% |
| Ex. 55 | 10 | MFI | H | 79.4 | 100.0% | 26.7% | 3.74 | -30.1% | -0.1% |
| Ex. 13 | 10 | MFI | H | 33.0 | 100.0% | 22.0% | 4.55 | -26.0% | 5.5% |
| Ex. 5 | 10 | MFI | H | 129000.0 | 80.7% | 0.9% | 88.51 | -24.6% | 10.9% |
| Ex. 1 | 10 | FER | K | 18.0 | 70.8% | 0.2% | 341.61 | -29.2% | -2.0% |

The time courses of d9-TMA plasma concentration and their AUCs when different doses of the zeolite of Example 34 were administered to choline-load fasted mice are shown in FIG. 8.

The zeolite of Example 34 dose-dependently decreased the plasma d9-TMA concentration and AUC, and at the highest dose of 2000 mg/kg, it almost completely suppressed the increase in plasma d9-TMA. It is considered that the zeolite of Example 34 had adsorbed most of the d9-TMA in the gastrointestinal tract.

The time courses of plasma concentrations of d9-choline and d9-TMA when different doses of the zeolite of Comparative Example 6 were administered to choline-load fasted mice are shown in FIG. 9.

For the zeolite of Comparative Example 6, which has low in vitro TMA/choline selectivity, the time to reach the maximum plasma concentration ($T_{max}$) of d9-TMA was delayed in the 600 mg/kg dose group from the vehicle group, and the slope of the reduction phase of the 600 mg/kg group was gentler than that of the vehicle group. Also, in the 2000 mg/kg group, the d9-TMA plasma concentration gradually increased from 4 to 10 hours after administration. At all doses, the plasma concentration of choline was consistently suppressed at a low level.

The time courses of plasma concentrations of d9-choline and d9-TMA in the zeolite of Comparative Example 6 suggested that (1) choline absorption in the upper gastrointestinal tract and the small intestine was suppressed by adsorption to the zeolite, (2) choline was released from the zeolite in the colon and (3) degraded into TMA by bacteria, and (4) TMA absorption from the colon occurred. Briefly, the zeolite may temporarily adsorb choline and release it back into the colon, thereby increasing the amount of TMA generated in the colon. An in vitro TMA/choline adsorption selectivity of about 1-fold may be not sufficient to fully compensate for the effect of increased TMA absorption due to choline delivery to the colon (Table 12; zeolites of Comparative Examples 6, 8, and 22). The fast choline release rate of the zeolites of Comparative Examples 6 and 22 has been confirmed in FIG. 4.

Zeolite Counter cation/Post-treatment Method and In Vitro/Vivo Efficacy

For the zeolites of Examples 34, 35, and 68, the effect of the difference in counter cations and the difference in post-treatment methods on the in vivo study using a choline-load fasted mouse model was compared within the same run (Table 13). Example 35 is a Na-form zeolite that did not exchanged to proton, Example 34 is a proton-form zeolite obtained by ammonium ion exchange of the Na-form followed by calcination, and Example 68 is a proton-form zeolite obtained by nitric acid treatment of the Na-form.

Efficacy was confirmed in the in vivo study using a choline-load fasted mouse model for all zeolites. Compared to the proton-form zeolite calcined after ammonium ion exchange, the proton-form zeolite treated with nitric acid had a higher d9-TMA AUC reduction ratio.

[Table 13]

| Table 13. Zeolite Counter cation/Post-treatment Method and In vitro/vivo Efficacy | | | | | | | | |
|---|---|---|---|---|---|---|---|---|
| | counter cation | SAR | post-treatment after hydrothermal synthesis | In vitro TMA adsorption ratio | In vitro choline adsorption ratio | In vitro TMA/ choline | TMA AUC change ratio | Choline AUC change ratio |
| Ex. 34 | H | 59.6 | ion exchange, firing | 100.0% | 33.1% | 3.02 | -35.9% | -12.8% |
| Ex. 35 | Na | 65.4 | - | 83.0% | 24.7% | 3.36 | -41.2% | -13.0% |
| Ex. 68 | H | 59.1 | acid treatment | 100.0% | 36.8% | 2.72 | -49.7% | -19.3% |

Zeolite Surface Treatment and In Vivo Efficacy

To confirm the effect of zeolite surface treatment on pharmacological effect, the zeolites of Examples 34, 70, and 78 were administered to choline-load fasted mice, and their pharmacological effects were compared. Table 14 summarizes the compound profiles and the results of the in vivo study using a choline-load fasted mouse model.

The zeolites of Examples 70 and 78 are those that have been surface-treated on the zeolite of Example 34 or a zeolite obtained by a similar synthesis method. Due to the surface treatment, the d9-TMA AUC reduction ratio was comparable to or greater than that before the surface treatment, while the decrease in d9-choline AUC was prevented. The effect of preventing choline adsorption by surface treatment was confirmed in the in vivo study using a choline-load fasted mouse model as well as the in vitro adsorption study.

[Table 14]

| Table 14. Zeolite Surface Treatment and In vitro/vivo Efficacy | | | | | | | | |
|---|---|---|---|---|---|---|---|---|
| | study No. | SAR | surface treatment agent | In vitro TMA adsorption ratio | In vitro choline adsorption ratio | In vitro TMA/ choline | TMA AUC change ratio | Choline AUC change ratio |
| Ex. 34 | S5 | 59.6 | - | 100.0% | 33.1% | 3.02 | -49.0% | -16.5% |
| Ex. 70 | S5 | 59.6 | HMDS | 100.0% | 11.5% | 8.68 | -50.6% | 10.2% |
| Ex. 34 | S7 | 59.6 | - | 100.0% | 33.1% | 3.02 | -37.2% | -5.7% |
| Ex. 78 | S7 | 61.7 | MS-51 | 100.0% | 3.2% | 31.36 | -43.4% | 8.5% |

Quartz Contained in Zeolite and In Vivo Efficacy

For the zeolites of Examples 34 and 52, the compound profiles and the AUC changes in the in vivo study using a choline-load fasted mouse model are shown in Table 15. FIG. 10 shows the XRPD patterns of Example 34 (upper panel) and Example 52 (lower panel). The vertical axis is intensity (cps), and the horizontal axis is the diffraction angle 2θ (°).

Example 52 contains more characteristic quartz peaks near 2θ=21° and 27° compared to Example 34. For the zeolite of Example 52, the d9-TMA AUC reduction ratio in the in vivo study using a choline-load fasted mouse model was smaller than that of Example 34 (Table 15). Quartz does not have a pore structure and is considered to have no TMA adsorption action. That is, in Example 52, which contains a large amount of quartz, the content ratio of MFI-type zeolite per weight decreased, and it is considered that the in vivo pharmacological effect (d9-TMA AUC reduction ratio) became lower than that of Example 34.

[Table 15]

| Table 15. Quartz Contained in Zeolite and In vitro/vivo Efficacy | | | | | | | |
|---|---|---|---|---|---|---|---|
| | SAR | characteristics of XRPD pattern | In vitro TMA adsorption ratio | In vitro Choline adsorption ratio | In vitro TMA/ choline | TMA AUC change ratio | Choline AUC change ratio |
| Ex. 34 | 59.6 | many quarts peaks | 100.0% | 33.1% | 3.02 | -49.0% | -16.5% |

(continued)

| Table 15. Quartz Contained in Zeolite and In vitro/vivo Efficacy | | | | | | | |
|---|---|---|---|---|---|---|---|
| | SAR | characteristics of XRPD pattern | In vitro TMA adsorption ratio | In vitro Choline adsorption ratio | In vitro TMA/ choline | TMA AUC change ratio | Choline AUC change ratio |
| Ex. 52 | 63.4 | few quarts peaks | 100.0% | 22.5% | 4.45 | -37.1% | -24.5% |
| (Test No. of Ex 34 is S5) | | | | | | | |

SEM Image, External Surface Area and In Vitro/Vivo Efficacy

For the zeolites used in the in vitro adsorption study and the in vivo study using a choline-load fasted mouse model, the correlation among pharmacological efficacy, external surface area, and SEM images was confirmed (Tables 16-1, 16-2).

For the zeolites of Comparative Examples 6 and 22, where the d9-TMA AUC reduction ratio and the d9-choline AUC reduction ratio were comparable, they mostly consist of fine particles of 1-2 μm or less and their aggregates in the SEM images. On the other hand, for the zeolites where the d9-TMA AUC reduction ratio was larger than the d9-choline AUC reduction ratio (Examples 5, 26, 34, and 52), it was confirmed that the proportion of fine particles of 1-2 μm or less and their aggregates was small compared to Comparative Examples 6 and 22, and the crystals had grown larger. For Examples 5, 26, and 34, which had high in vitro adsorption selectivity and a particularly large difference between the d9-TMA AUC reduction ratio and the d9-choline AUC reduction ratio, it was confirmed by particle observation using SEM that the crystal shape was coffin-shaped, and the (010) crystal surface was large. The adsorption rates of choline and TMA are governed by the rate of entry of molecules from the medium into the zeolite pores and the rate of diffusion of molecules through the crystal pores. In the case of MFI-type zeolite having a three-dimensional channel structure, it is further considered that there are differences in the rate of penetration by the crystal planes and in the rate of diffusion along the three-dimensional coordinate axes of the crystal. In MFI-type zeolite, since the TMA/choline selectivity was high when the (010) crystal surface was large, it is presumed that the (010) surface is a crystal surface with particularly high TMA/choline selectivity. It has been confirmed in SCIENCE, 2003, Vol 300, Issue 5618, pp. 456-460 that the largest crystal surface of coffin-shaped MFI-type zeolite is the (010) surface. Furthermore, as reported in J. Am. Chem. Soc. 2023, 145, 9021-9028, further improvement in selectivity can be expected by selectively treating surfaces other than the (010) surface, which is difficult for choline to enter. Also, since the length of the crystal axes of a coffin-shaped crystal is in the order of [001] axis > [100] axis > [010] axis, it is similarly presumed that TMA/choline selectivity is obtained in the intracrystalline pore diffusion process in the [001] axis direction.

A tendency was confirmed that the more fine particles of 1-2 μm or less and their aggregates (Comparative Examples 6 and 22) or scale-like structures (Examples 19 and 53) were present in the SEM image, the larger the external surface area became. Furthermore, a correlation was also confirmed that the larger the external surface area, the higher the d9-choline AUC reduction ratio in the in vivo study.

For Example 52, which was confirmed to contain a large amount of quartz from XRPD data, the SEM image also shows many particles different from MFI-type zeolite, which are considered to be quartz, compared to Example 34.

[Table 16-1]

| Table 16-1. SEM image, external surface area and in vitro/vivo efficacy | | | | | | | | |
|---|---|---|---|---|---|---|---|---|
| | SAR | External surface area | SEM image | In vitro TMA adsorption ratio | In vitro Choline adsorption ratio | In vitro TMA/ choline | TMA AUC change ratio | Choline AUC change ratio |
| Comp. Ex.6 | 24.0 | 36.2 $m^2/g$ | 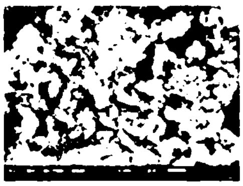 | 86.8% | 91.1% | 0.95 | -44.3% | -49.3% |

(continued)

Table 16-1. SEM image, external surface area and in vitro/vivo efficacy

| | SAR | External surface area | SEM image | In vitro TMA adsorption ratio | In vitro Choline adsorption ratio | In vitro TMA/ choline | TMA AUC change ratio | Choline AUC change ratio |
|---|---|---|---|---|---|---|---|---|
| Comp. Ex.22 | 26.0 | 33.4 $m^2/g$ | 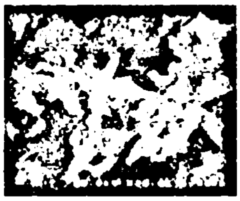 | 89.7% | 88.9% | 1.01 | -43.6% | -42.4% |
| Ex.19 | 78.5 | 25.6 $m^2/g$ | 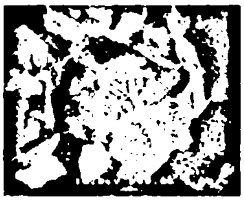 | 96.7% | 17.6% | 5.49 | - | - |
| Ex.53 | 62.7 | 24.8 $m^2/g$ | 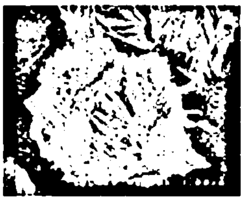 | 92.0% | 36.1% | 2.56 | - | - |
| Ex.52 | 63.4 | 23.8 $m^2/g$ | 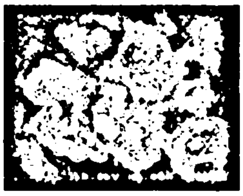 | 100.0% | 22.5% | 4.45 | -37.1% | -24.5% |
| Ex.38 | 57.2 | 14.8 $m^2/g$ | 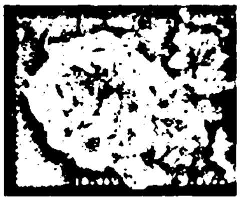 | 89.8% | 31.1% | 2.89 | - | |

[Table 16-2]

Table 16-2. SEM image, external surface area and in vitro/vivo efficacy ~continued

| | | | | | | | | |
|---|---|---|---|---|---|---|---|---|
| Ex.34 | 59.6 | 13.3 $m^2/g$ | 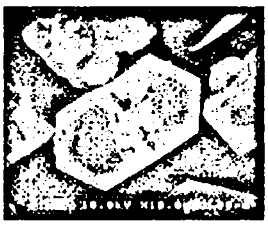 | 100.0% | 33.1% | 3.02 | -54.2% | -27.2% |

(continued)

| Table 16-2. SEM image, external surface area and in vitro/vivo efficacy ~continued | | | | | | | | | |
|---|---|---|---|---|---|---|---|---|---|
| Ex.49 | 60.8 | 12.7 $m^2/g$ | 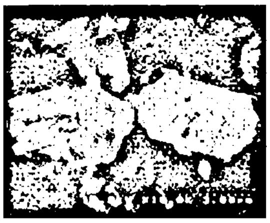 | 100.0% | 34.0% | 2.94 | - | - |
| Ex.11 | 42.1 | 12.6 $m^2/g$ | 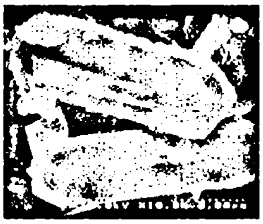 | 100.0% | 30.3% | 3.30 | - | - |
| Ex.26 | 41.1 | 7.8 $m^2/g$ | 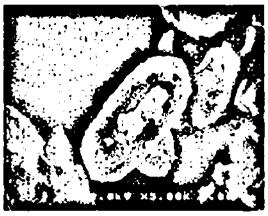 | 100.0% | 33.1% | 3.02 | -54.6% | -7.3% |
| Ex.5 | 129000.0 | 6.5 $m^2/g$ | 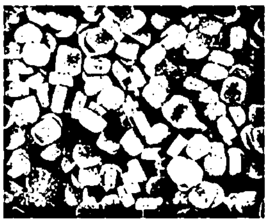 | 80.7% | 0.9% | 88.51 | -24.6% | 10.9% |

<Single-dose PK study and construction of a physiologically based pharmacokinetic (PBPK) model>

Single intravenous dose PK study of d9-TMA and d9-TMAO

d9-TMA or d9-TMAO was administered intravenously to 7-9 week-old male C57BL/6 mice at a dose of 11.5 mg/kg or 10 mg/kg, and blood samples were collected serially. In addition, d9-TMA was administered intravenously to 7-week-old male C57BL/6 mice at a dose of 10 mg/kg, and urine was collected for 24 hours. The concentrations of d9-TMA and d9-TMAO in plasma and urine were measured by LC-MS/MS.

Single oral dose PK study of d9-TMA

d9-TMA was administered orally to 7-week-old male C57BL/6 mice under fasted conditions at a dose of 34.6 mg/kg, and serial blood samples were collected. The concentrations of d9-TMA and d9-TMAO in plasma were measured by LC-MS/MS.

Construction of the PBPK model

With reference to the report by Shimizu et al.[1)], a PBPK model for d9-choline, d9-TMA, and d9-TMAO when d9-choline is orally administered to mice was constructed by adopting a transit model that takes into account the conversion process from d9-choline to d9-TMA by intestinal bacteria in the colon and the transit time from the upper gastrointestinal tract to the colon. A conceptual diagram of the PBPK model is shown in FIG. 11. For the construction, in vivo data from the vehicle group of the previously described "In Vivo Study Using a Choline-Load Fasted Mouse Model" and in vivo data from the single-dose PK study were used.

1) Shimizu, M. et al. Human plasma concentrations of trimethylamine N-oxide extrapolated using pharmacokinetic modeling based on metabolic profiles of deuterium-labeled trimethylamine in humanized-liver mice. J. Toxicol. Sci. 43, 387-393 (2018).

The following steps were applied for the construction of the PBPK model.

* The liver-to-plasma partition coefficient, blood cell-to-plasma partition coefficient, and plasma protein unbound fraction for d9-TMA and d9-TMAO were cited from the report by Shimizu et al.[1)], assuming no species difference.
* The liver volume and hepatic blood flow rate were cited from the report by Yamashita et al.[2)].
* Using the data from the single intravenous dose PK study of d9-TMA and d9-TMAO, the PK parameters for d9-TMA and d9-TMAO were estimated. The renal clearance was optimized to accurately reproduce the urinary excretion rate of d9-TMA.
* Using the data from the vehicle group of the in vivo study using a choline-load fasted mouse model, the absorption parameters for d9-TMA (including the rate constant for transit from the upper gastrointestinal tract to the colon and the conversion ratio from d9-choline to d9-TMA) were estimated. The conversion ratio was calculated by dividing the value obtained by normalizing the dose-corrected AUC of d9-TMA in the vehicle group of the in vivo study using a choline-load fasted mouse model by the dose-corrected AUC of d9-TMA in the d9-TMA administered group of the single intravenous dose PK study, by the absorption ratio of d9-TMA obtained from the PBPK model.
* Considering that the AUC reduction ratios of d9-choline and d9-TMA reflect the inhibition ratio of TMA absorption from gastrointestinal tract (hereinafter sometimes referred to as $IR_{pre}$ and IR, respectively) resulting from its adsorption to the zeolite, relational expressions for $IR_{pre}$ and IR with respect to the zeolite dose were constructed, respectively. For d9-choline, the relationship between the inhibition ratio of TMA absorption from gastrointestinal tract and the zeolite dose was fitted to an Emax model to calculate the 50% effective dose. For d9-TMA, assuming that adsorption equilibrium is reached in the gastrointestinal tract, it was considered to follow the formula: inhibition ratio of d9-TMA absorption from gastrointestinal tract = theoretical acid amount of unadsorbed zeolite / (water volume in the colon × dissociation constant + theoretical acid amount of unadsorbed zeolite), and the dissociation constant of zeolite and d9-TMA was calculated. The water volume in the colon was cited from the value in SimCYP (V23) manufactured by Certara.

2) Yamashita, M. et al. Human plasma concentrations of herbicidal carbamate molinate extrapolated from the pharmacokinetics established in in vivo experiments with chimeric mice with humanized liver and physiologically based pharmacokinetic modeling. Regulatory Toxicology and Pharmacology 70, 214-221 (2014).

The parameters used in the PBPK model are shown in Table 16-3.

[Table 16-3]

Table 16-3. Parameters used in PBPK model

| Parameter | Unit | Value | Explanation |
|---|---|---|---|
| $V_h$ | L/kg | 0.034 | liver volume |
| $Q_h$ | L/h/kg | 6.4 | hepatic blood flow rate |
| $K_{ph}$ | - | 0.752 | liver-to-plasma partition coefficient (d9-TMA) |
| $R_b$ | - | 0.719 | blood cell-to-plasma partition coefficient (d9-TMA) |
| $f_p$ | - | 0.9 | plasma protein unbound fraction (d9-TMA) |
| $K_{ph, m}$ | - | 0.675 | liver-to-plasma partition coefficient (d9-TMAO) |
| $R_{b, m}$ | - | 0.845 | blood cell-to-plasma partition coefficient (d9-TMAO) |
| $f_{p, m}$ | - | 0.714 | plasma protein unbound fraction (d9-TMAO) |
| $V_{1, pre}$ | Ukg | 79.55 | central compartment volume (d9-choline) |
| $k_e$ | /h | 0.05058 | elimination rate constant (d9-choline) |
| $k_{a, pre}$ | /h | 4.823 | absorption rate constant (d9-choline) |
| $k_{12, pre}$ | /h | 0.8397 | rate constant from central to peripheral compartment (d9-choline) |
| $k_{21, p21}$ | /h | 0.2395 | rate constant from peripheral to central compartment (d9-choline) |
| $V_{1, m}$ | Ukg | 0.3345 | central compartment volume (d9-TMAO) |
| $CL_{int, h, m}$ | L/h/kg | 0.05619 | hepatic intrinsic clearance (d9-TMAO) |

(continued)

Table 16-3. Parameters used in PBPK model

| Parameter | Unit | Value | Explanation |
|---|---|---|---|
| $CL_{r, m}$ | L/h/kg | 0.8955 | renal clearance (d9-TMAO) |
| $k_{12, m}$ | /h | 0.8907 | rate constant from central to peripheral compartment (d9-TMAO) |
| $k_{21, m}$ | /h | 0.6291 | rate constant from peripheral to central compartment (d9-TMAO) |
| $V_1$ | L/kg | 2.581 | central compartment volume (d9-TMA) |
| $CL_{int, h}$ | L/h/kg | 0.2383 | hepatic intrinsic clearance (d9-TMA) |
| $CL_p$ | L/h/kg | 3.217 | renal clearance (d9-TMA) |
| $k_{12}$ | /h | 1.093 | rate constant from central to peripheral compartment (d9-TMA) |
| $k_{21}$ | /h | 0.7807 | rate constant from peripheral to central compartment (d9-TMA) |
| BC | - | 0.9324 | conversion ratio from d9-choline to d9-TMA |
| $CL_{tot}$ | Uh/kg | 3.982 | total body clearance (d9-TMA) |
| $k_{deg}$ | /h | 0.7137 | conversion rate from d9-choline to d9-TMA |
| $k_a$ | /h | 0.5913 | absorption ratio constant (d9-TMA) |
| $F_aF_g$ | - | 0.7723 | intestinal wall permeability x fraction escaping intestinal metabolism (d9-TMA) |
| $F_{pre}$ | - | 0.3588 | absorption ratio (d9-choline) |
| $k_t$ | /h | 12.53 | rate constant for transit from upper gastrointestinal tract to colon (d9-TMA) |
| $ED_{50, pre}$ | mg/kg | 1257 | 50% effective dose (d9-choline) |
| $K_d$ | μmol/mL | 12.25 | dissociation constant of zeolite and d9-TMA |
| $V_{col}$ | mL/kg | 5.08 | water volume in the colon |

Simulation of plasma concentration-time profile and urinary excretion amount following administration of the zeolite of Example 34

FIG. 12 shows a graph comparing the measured plasma concentrations of d9-choline, d9-TMA, and d9-TMAO with the plasma concentration-time profile predicted from the PBPK model when different doses of the zeolite of Example 34 were administered to choline-load fasted mice. In addition, the predicted results of the 24-hour urinary excretion amount calculated from the same PBPK model are shown in Table 16-4. The plasma concentration-time profiles of d9-choline, d9-TMA, and d9-TMAO simulated by the PBPK model generally reproduced the measured plasma concentration-time profiles.

[Table 16-4]

Table 16-4. Predicted results of 24-hour urinary excretion amount calculated from PBPK model

| | | 24-hour urinary excretion amount (μmol) | | inhibition ratio of TMA absorption from gastrointestinal tract (%) |
|---|---|---|---|---|
| | | d9-TMA | d9-TMA + d9-TMAO | |
| Vehicle | | 6.20 | 7.01 | - |
| Ex. 34 | 500 mg/kg | 2.78 | 3.14 | 55.2 |
| | 1000 mg/kg | 0.89 | 1.01 | 85.6 |
| | 2000 mg/kg | 0.21 | 0.23 | 96.7 |

<In Vivo Study Using a Choline-Load Non-Fasted Mouse Model>

Method

Ten-week-old male C57BL/6 mice, acclimatized to a reversed light-dark cycle and restricted feeding, and fed a choline-free diet (A21122102) (EP Trading Co., Ltd.), were simultaneously administered orally with d9-choline chloride (Toronto Research Chemicals Inc.) dissolved in distilled water and the zeolite of Example 34 suspended in a 0.5 w/v% methylcellulose 400 solution, sterilized (FUJIFILM Wako Pure Chemical Corporation), at doses of 78.78 mg/kg and 500-2000 mg/kg, respectively. Similarly, d9-choline chloride dissolved in distilled water and the zeolite of Example 70 suspended in a 0.5 w/v% methylcellulose 400 solution, sterilized, were simultaneously administered orally at doses of 78.78 mg/kg and 1000 mg/kg, respectively. After oral administration, the animals were housed in metabolic cages, and urine was collected for 24 hours. The concentrations of d9-TMA, d9-TMAO, native TMA, and native TMAO in the obtained urine were measured by LC/MS/MS, and the respective 24-hour urinary excretion amounts were calculated from the urine volume. Here, "native" indicates a molecule composed of the most abundant isotope of each element.

The results are shown in FIG. 13. The administration of the zeolite of Example 34 caused a dose-dependent decrease in the combined value of the 24-hour urinary excretion amount (μmol) of d9-TMA and TMAO from the vehicle group. Similarly, the combined value of the 24-hour urinary excretion amount of native TMA and TMAO also decreased dose-dependently. The combined value of these d9 and native forms also decreased dose-dependently. Administration of the zeolite of Example 70 resulted in a decrease in the 24-hour urinary excretion amount of TMA and TMAO comparable to that of the zeolite of Example 34.

The administration of the zeolite of Example 34 reduced the urinary excretion of d9-TMA and TMAO under non-fasted conditions where d9-choline was loaded to mimic human meal conditions, not in the fasted conditions. This indicates that the zeolite of Example 34 specifically recognized and adsorbed/removed TMA even under non-fasted conditions where various interfering substances are present in the gastrointestinal tract. It was strongly suggested that using a zeolite with high selectivity for TMA can show pharmacological efficacy in TMAU patients.

<Clinical Dose Prediction>

The clinical effective dose of the zeolite of Example 34 was estimated by calculating the inhibition ratio of TMA absorption from gastrointestinal tract by the zeolite of Example 34 based on the total urinary excretion of d9-TMA and d9-TMAO in the in vivo study of a choline-load non-fasted mouse model, and correcting its effective dose by the ratio of TMA amounts in gastrointestinal tract of mice to that of humans.

Calculation of Inhibition ratios of TMA Absorption from Gastrointestinal tract

When $^{14}$C-TMA was orally administered to humans and rats, 95% and 80% or more of the radioactivity was excreted in the urine within 24 hours after administration, respectively, and the majority of it was TMA and TMAO[3, 4)]. From this, it was considered that the TMA amount in gastrointestinal tract of mice could be inferred from the total urinary excretion of TMA and TMAO.

3) Al-Waiz M, Mitchell SC, Idle JR, Smith RL. The metabolism of 14C-labelled trimethylamine and its N-oxide in man. Xenobiotica 1987; 17(5): 551-558
4) Al-Waiz M, Mitchell SC. The fate of trimethylamine in the rat. Drug Metab and Drug Interact 1991; 9(1): 41-48

The inhibition ratio of TMA absorption from gastrointestinal tract of the zeolite of Example 34 in this study was calculated from the following formula, using the total urinary excretion of d9-TMA and d9-TMAO when the vehicle or the zeolite of Example 34 was co-administered orally with d9-choline.

[Eq. 2]

$$\text{inhibition ratio (\%) of TMA absorption from gastrointestinal tract}$$
$$= \left(1 - \frac{\text{total urinary excretion (μmol) of d9-TMA and TMAO in Example 34 zeolite-administered group}}{\text{total urinary excretion (μmol) of d9-TMA and TMAO in vehicle group}}\right) \times 100$$

The daily TMA intake in a typical Western diet is reported to be 50 mg TMA/day[5)], and the threshold for urinary excretion at which the TMA odor arises from patients is reported to be 20 mg TMA/day[6]. Since the urinary TMAO prevalence (as % of TMA + TMAO) is reported to be 9.5% in the most severe TMAU patient reported in a paper[7] and the average value for severe patients (n=13) is 26.6%, the target inhibition ratio of TMA absorption from gastrointestinal tract as a therapeutic agent for TMAU in each patient group was calculated from the following formula.

[Eq. 3]

$$\text{the target value of inhibition ratio (\%) of TMA absorption from gastrointestinal tract for clinical practice}$$

$$= \left(1 - \frac{\text{the threshold of urinary excretion (20mg)} \div 0.95 \div \left(1 - \frac{\text{urinary TMAO prevalence (\%)}}{100}\right)}{\text{daily TMA intake in typical western diet}}\right) \times 100$$

5) Fennema D, Phillips IR, Shephard EA. Trimethylamine and Trimethylamine N-Oxide, a Flavin-Containing Monooxygenase 3 (FMO3)-Mediated Host-Microbiome Metabolic Axis Implicated in Health and Disease. Drug Metab Dis 2016; 44:1839-1850
6) Michell SC, Smith RL. Trimethylaminuria: The Fish Malodor Syndrome. Drug Metab Dis 2001; 29:517-521
7) Shimizu M, Allerston CK, Shephard EA, Yamazaki H, Phillips IR. Relationships between flavin-containing monooxygenase 3 (FMO3) genotype and trimethylaminuria phenotype in a Japanese population. Br J Clin Pharmacol 2013; 77(5): 839-851

Estimation of Clinical Dose

The adsorption ratio by a zeolite is determined by the abundance ratio of the substance to be adsorbed and the zeolite. Based on this idea, the clinical effective dose of the zeolite of Example 34 was estimated by correcting the mouse dose (mouse effective dose) at which the TMA adsorption ratio (inhibition ratio of TMA absorption from gastrointestinal tract) in mice is equivalent to the target TMA adsorption ratio in clinical practice, by the ratio of standard TMA amounts in gastrointestinal tract of mice and humans. The formula used for the calculation is shown below.

[Eq. 4]

$$\text{estimated clinical effective dose (mg/man/meal) of the zeolite of Example 34}$$

$$= \text{mouse effective dose (mg/head)} \times \frac{\text{daily TMA intake in typical western diet (50mg)} \div 3}{\text{TMA amount in gastrointestinal tract of mouse* (mg)}}$$

*Value obtained by dividing the total urinary excretion of d9-TMA and d9-TMAO (mol) in the vehicle group by the urine recovery ratio (0.9) and the bioavailability of TMA (assumed to be 0.8, the same as in rats), and converting to TMA equivalent weight. The urine recovery ratio (0.9) was calculated based on the amount of urine before dripping and after recovery, obtained by dripping and recovering mouse urine in a metabolic cage.

Calculation of Inhibition ratios of TMA Absorption from Gastrointestinal tract

The inhibition ratios of TMA absorption from gastrointestinal tract calculated from the above formula are shown in Table 17, and the target inhibition ratios of TMA absorption from gastrointestinal tract for clinical practice are shown in Table 18. The inhibition ratio of TMA absorption from gastrointestinal tract when the zeolite of Example 34 and 60 mg/kg of d9-choline were co-administered to mice was 42.8% at 500 mg/kg, 58.4% at 1000 mg/kg, and 90.4% at 2000 mg/kg.

Also, the target of inhibition ratio of absorption from gastrointestinal tract for clinical practice was calculated to be 42.6% in severe patients and 53.5% in patients considered to be the most severe in the paper. From these results, the inhibition ratio of TMA absorption from gastrointestinal tract when the zeolite of Example 34 was administered to mice at 500 mg/kg and 1000 mg/kg were considered to be equivalent to the target inhibition ratios assumed for the severe patient group and the most severe patient, respectively.

[Table 17]

Table 17. Inhibition ratio of TMA absorption from gastrointestinal tract in pharmacological test using TMAU model mouse

| | | 24-hour urinary excretion amount (μmol, n=6 mean) | | | inhibition ratio of TMA absorption from gastrointestinal tract (%) |
|---|---|---|---|---|---|
| | | d9-TMA | d9-TMAO | d9-TMA + d9-TMAO | |
| | Vehicle | 2.013 | 0.354 | 2.368 | - |
| Ex. 34 | 500 mg/kg | 1.159 | 0.197 | 1.355 | 42.8 |
| | 1000 mg/kg | 0.821 | 0.163 | 0.984 | 58.4 |
| | 2000 mg/kg | 0.180 | 0.049 | 0.228 | 90.4 |

[Table 18]

Table 18. Target of inhibition ratio of TMA absorption from gastrointestinal tract for clinical practice

| patient category | urinary TMAO prevalence (%)* | target of inhibition ratio of TMA absorption from gastrointestinal tract (%) |
|---|---|---|
| severe | 26.6 | 42.6 |
| most severe | 9.5 | 53.5 |
| * severe: average of 13 patients | | |

Results of Clinical Dose Estimation

The estimated clinical effective doses of the zeolite of Example 34 calculated from the above formula are shown in Table 19. The clinical effective dose expected to have a therapeutic effect on severe patients was estimated to be 793 mg/man/meal, equivalent to 500 mg/kg in the TMAU model mouse, and similarly, the clinical effective dose for the most severe patient was estimated to be 1595 mg/man/meal, equivalent to 1000 mg/kg in the same model mouse.

[Table 19]

Table 19. Estimated clinical effective dose of zeolite of Example 34

| effective dose to mouse | | | TMA amount in gastrointestinal tract of mouse | | clinical TMA intake | estimated clinical effective dose |
|---|---|---|---|---|---|---|
| (mg/kg) | body weight (kg mean) | (mg/head) | (μmol) | (mg) | (mg/meal) | (mg/man/meal) |
| 500 | 0.0185 | 9.25 | 3.289 | 0.194 | 16.7 | 793 |
| 1000 | 0.0186 | 18.6 | 3.289 | 0.194 | 16.7 | 1595 |
| Molecular weight of TMA: 59.11 | | | | | | |

<Leaching test of Si and Al>

Methods & Results

A leaching test for Si and Al was conducted for the zeolites of Examples 26 and 34. 250 mg of zeolite was accurately weighed, 5 mL of 1N hydrochloric acid was added, and the mixture was stirred at 37°C for 3 hours. This solution was centrifuged. 2 mL of the supernatant was centrifuged again to obtain a new supernatant (first eluate). The remaining zeolite-containing solution was filtered using filter paper (No. 5B) to collect the zeolite. This zeolite was added to a fresh 5 mL of 1N hydrochloric acid and stirred at 37°C for 3 hours. 2 mL of the supernatant of this solution was taken and centrifuged to obtain a new supernatant (second eluate). 1000 mg of each of the first and second eluates was weighed, 2 mL of 38% (12N) hydrochloric acid was added, and ultrapure water was added thereto to make a final volume of 25 mL. The Si and Al concentrations in these solutions were measured using ICP-AES (Inductively Coupled Plasma Atomic Emission Spectroscopy), and the concentrations and elution ratios of in the eluate Si and Al were calculated (Table 20). The elution ratio is the value obtained by calculating the weight of Si or Al in the zeolite used in the test, assuming that the counter cation of the zeolite is a proton and that the zeolite contains no water, and dividing this by the weight of Si or Al present in a 5 mL equivalent of eluate.

For the zeolite of Example 34, the elution ratio of Al was 10% or less, and for the zeolite of Example 26, it was about 0.1%. The elution of Si was 0.1% or less for both compounds. Also, the elution amounts of both Si and Al decreased during the second elution compared to the first elution. This is thought to be because trace impurities other than zeolite that were present during the first elution and the incomplete structure of the zeolite surface were gone by the second elution. It is considered that by adding an acid treatment as a post-treatment after zeolite synthesis, the amounts of Si and Al eluted in the gastrointestinal tract during in vivo administration can be reduced.

During in vivo administration, since a slight amount of Si and Al elution is expected in the gastric acid, it is necessary to consider the effect on the body. Even if a 70 kg human takes 3 g of the zeolites of Examples 26 and 34 per day, and if they elute to the same extent as in this study in the gastrointestinal tract, the amount of Al elution will not exceed the Provisional Tolerable Weekly Intake of Al (2 mg/kg body weight/week) set by the Joint FAO/WHO Expert Committee on Food Additives (JECFA), and it is considered that there are few safety concerns.

[Table 20]

Table 20. Leaching test of Si and Al

| zeolite | SAR | solution analyzed | concentration in the solution (μg/g) | | leaching ratio | |
|---|---|---|---|---|---|---|
| | | | Al | Si | Al | Si |
| - | - | 1N hydrochloric acid | <0.1 | <0.1 | - | - |
| Ex. 34 | 59.6 | first leaching | 65.1 | 18.9 | 9.1% | 0.085% |
| | | second leaching | 9.82 | 9.84 | 1.4% | 0.044% |
| Ex. 26 | 41.1 | first leaching | 1.16 | 3.23 | 0.11% | 0.015% |
| | | second leaching | 0.468 | 2.81 | 0.045% | 0.013% |

<Confirmation Study of Gastrointestinal Absorption>

For the zeolites of Examples 26 and 34, the fecal excretion ratio when each was administered orally as a single dose to rats was calculated from the Si and Al concentrations in the feces. It was shown that for both zeolites, almost the entire amount of the administered zeolite was excreted in the feces, and they were hardly absorbed from the gastrointestinal tract. Specific details are shown below.

Test Method

The zeolite of Example 26 or Example 34 was administered orally as a single dose to 6-week-old male Crl:CD(SD) rats at 2000 mg/kg dose, and feces were collected up to 48 hours after administration. After adding ultrapure water to the collected feces, a homogenate was prepared using a Polytron and lyophilized. An appropriate amount of the dried feces was weighed, incinerated, melted in alkaline salt, and dissolved in acid to prepare a sample solution. The Si and Al concentrations in the sample solutions were measured using ICP-AES. In addition, feces obtained from 24 hours before to immediately before the administration of each zeolite were used as blank feces, and after performing the same homogenization and lyophilization as above on the blank dried feces, a specified amount of each zeolite was added, and the Si and Al concentrations in the feces were measured by the same experimental procedure as above to create a calibration curve of the Si and Al concentrations in the feces against the added zeolite concentration, and the fecal excretion ratio of the zeolite was determined.

Results

The fecal excretion ratio of the zeolite of Example 26 was 99%-118% (based on Si concentration) and 98%-116% (based on Al concentration), and the fecal excretion ratio of the zeolite of Example 34 was 91%-104% (based on Si concentration) and 96%-109% (based on Al concentration).

<Nonclinical Safety Studies>

When cytotoxicity tests, a single-dose toxicity study, a one-week repeated dose toxicity study, and in vitro genotoxicity tests were conducted using various zeolites, high safety was confirmed in all studies. Details are shown below.

Cytotoxicity Test

For the zeolites of Comparative Examples 6, 10, 12, 15, and 16, and Examples 9, 13, 25, 26, 58, 70, and 79, cytotoxicity was evaluated using HepG2 cells based on the number of viable cells indicated by the ATP amount at a maximum dose of 500 μg/mL. It was confirmed that for all zeolites, the 50% inhibitory concentration ($IC_{50}$) was higher than 500 μg/mL.

Single-Dose Toxicity Study

The zeolite of Example 34 was administered orally as a single dose to 6 or 7-week-old Crl:CD(SD) male rats at a maximum dose of 6000 mg/kg/day (2000 mg/kg, 3 times per day), and general condition, body weight, food consumption, and gross pathology (external surface, thoracic and abdominal cavities and their internal organs and tissues) were examined. The zeolite of Example 34 was well-tolerated up to the maximum dose.

One-Week Repeated Dose Toxicity Study

The zeolite of Example 34 was administered orally for one week to 6 or 7-week-old Crl:CD(SD) male rats at a maximum dose of 6000 mg/kg/day (2000 mg/kg, 3 times per day). The following items were evaluated: general condition, body weight, food consumption, ophthalmological examination, hematological examination (red blood cell count, hemoglobin concentration, hematocrit, mean corpuscular volume, mean corpuscular hemoglobin, mean corpuscular hemoglobin concentration, red blood cell distribution width, reticulocytes, platelet count, mean platelet volume, white blood cell count, white blood cell differential, prothrombin time, activated partial thromboplastin time), blood chemistry examination (aspartate aminotransferase, alanine aminotransferase, alkaline phosphatase, glutamate dehydrogenase, glucose, total bilirubin, direct bilirubin, indirect bilirubin, total bile acids, urea nitrogen, creatinine, total cholesterol, triglycerides, inorganic phosphorus, calcium, sodium, potassium, chloride, total protein, protein fraction, albumin/globulin ratio), organ weights (heart, thymus, spleen, lung, liver, kidney, pituitary, adrenal, testis, epididymis, prostate, brain), gross pathology (external surface, thoracic and abdominal cavities and their internal organs and tissues), histopathological examination (heart, sternum, sternal bone marrow, femur, knee joint, femoral bone marrow, thymus, spleen, mesenteric lymph node, bronchus, lung, stomach, duodenum, jejunum, ileum, Peyer's patch, cecum, colon, rectum, liver, pancreas, kidney, adrenal, testis, brain, sciatic nerve, eye, optic nerve, skeletal muscle), and bone marrow micronucleus evaluation. In all dose groups, no toxicological changes were observed.

In Vitro Genotoxicity Test

The zeolite of Example 13 was evaluated for mutagenicity based on the number of revertant colonies under two conditions (in the absence of a metabolic activation system and in the presence of a metabolic activation system) using the strains Salmonella typhimurium TA100, TA98, TA1537, TA1535, and Escherichia coli WP2uvrA, with a maximum dose of 5000 μg/plate, the upper limit specified in the guidelines. It was judged to be negative. (Ames test)

The zeolite of Example 13 was evaluated for micronucleus-inducing potential under three conditions (6-hour treatment in the absence of a metabolic activation system, 6-hour treatment in the presence of a metabolic activation system, and 24-hour treatment in the absence of a metabolic activation system) using CHL/IU cells, with a maximum dose of 0.5 μg/mL, the minimum dose at which precipitation occurred. It was judged to be negative. (in vitro micronucleus test)

<Adsorption Selectivity Studies>

Adsorption selectivity studies were conducted for ions and compounds in the body that may be competitively adsorbed with the compound to be removed, or whose adsorption may cause problems, when administered to humans.

Alkali Metals, Alkaline Earth Metals, Ammonia

An adsorption study for alkali metals, alkaline earth metals, and ammonia was conducted for the zeolites of Examples 5, 34, and 70, and Comparative Example 6. 3 mg of zeolite was accurately weighed, 10 mL of a mixed cation standard solution II (Kanto Chemical Co., Inc., $Li^+$: 0.5 mg/mL, $Na^+$ and $NH_4^+$: 2 mg/mL, $K^+$, $Mg^{2+}$ and $Ca^{2+}$: 5 mg/mL) was added, and the mixture was stirred at room temperature for 1 hour. This solution was centrifuged, and the cations in the supernatant were measured by ion chromatography to calculate the adsorption ratio. The results are shown in Table 21-1.

Except for the zeolite of Example 5, which has a high SAR, the remaining three zeolites adsorbed $NH_4^+$ and $K^+$. The adsorption ratios for $Li^+$, $Na^+$, $Mg^{2+}$, and $Ca^{2+}$ were low for all zeolites.

Alkali Metals, Ammonia

An adsorption study for alkali metals and ammonia was conducted for the zeolite of Example 4. 3 mg of the zeolite was accurately weighed, 0.5 mL of a mixed cation standard solution III (Kanto Chemical Co., Inc., $Na^+$, $NH_4^+$ and $K^+$: 100 mg/mL) and 9.5 mL of ultrapure water were added, and the mixture was stirred at room temperature for 1 hour. This solution was centrifuged, and the cations in the supernatant were measured by ion chromatography to calculate the adsorption ratio. The results are shown in Table 21-2. The zeolite of Example 4, which has a high SAR, hardly adsorbed $Na^+$, $NH_4^+$, and $K^+$.

Alkali Metals, Ammonia (in the presence of TMA)

An adsorption study for alkali metals and ammonia in the presence of TMA was conducted for the zeolites of Examples 34 and 70, and Comparative Example 6. A TMA standard solution was prepared by accurately weighing 80 mg of TMA hydrochloride and adding it into 50 mL of a 0.1N hydrochloric acid aqueous solution prepared by diluting 1N hydrochloric acid, and then shaking well for dissolution. 3 mg of zeolite was accurately weighed, 0.5 mL of a mixed cation standard solution III (Kanto Chemical Co., Inc., $Na^+$, $NH_4^+$ and $K^+$: 100 mg/mL), 9.5 mL of ultrapure water, and 50 $\mu$L of the TMA standard solution were added, and the mixture was stirred at room temperature for 1 hour. This solution was centrifuged, and the cations in the supernatant were measured by ion chromatography to calculate the adsorption ratio. The results are shown in Table 21-3. All compounds adsorbed the entire amount of TMA in the presence of TMA, and the adsorption of $NH_4^+$ and $K^+$ decreased compared to in the absence of TMA (see Table 21-1).

In the process of each cation being adsorbed from water into the zeolite pores, it needs to be dehydrated. The heat of hydration is $Li^+ > Na^+ > K^+ \sim NH_4^+$, and it is considered that the adsorption of $Li^+$ and $Na^+$ was weak because there was not enough stabilization energy to compensate for this heat of hydration. Also, since $Mg^{2+}$ and $Ca^{2+}$ are divalent cations, for them to be stabilized by electrostatic interaction in the zeolite pores, multiple acid sites need to exist within a unit pore. At the SAR of the zeolites used in this study, the proportion of unit pores containing multiple acid sites is low, and it is considered that they were not suitable for the adsorption of divalent cations. Furthermore, this study revealed that the adsorption of TMA is preferred over the adsorption of $NH_4^+$ and $K^+$ (see Tables 21-1 and 21-3).

[Table 21-1]

Table 21-1. Adsorption ratios of alkali metals, alkaline earth metals, and ammonia

| | SAR | Li adsorption ratio | Na adsorption ratio | $NH_4$ adsorption ratio | K adsorption ratio | Mg adsorption ratio | Ca adsorption ratio |
|---|---|---|---|---|---|---|---|
| Comp. Ex. 6 | 24.0 | 1.1% | 0.0% | 40.9% | 40.1% | 0.1% | 3.4% |
| Ex. 34 | 59.6 | 4.9% | 0.2% | 34.4% | 28.5% | 2.6% | 2.4% |
| Ex. 70 | 59.6 | 2.6% | 1.5% | 32.7% | 25.8% | 0.0% | 0.0% |
| Ex. 5 | 129000.0 | 0.0% | 0.0% | 0.0% | 0.0% | 0.0% | 0.0% |

[Table 21-2]

Table 21-2. Adsorption ratios of alkali metals and ammonia

| | SAR | Na adsorption ratio | $NH_4$ adsorption ratio | K adsorption ratio |
|---|---|---|---|---|
| Ex. 4 | 874.0 | 1.5% | 3.3% | 3.1% |

[Table 21-3]

Table 21-3. Adsorption ratios of alkali metals, ammonia, and TMA

| | SAR | Na adsorption ratio | $NH_4$ adsorption ratio | K adsorption ratio | TMA adsorption ratio |
|---|---|---|---|---|---|
| Comp. Ex. 6 | 24.0 | 0.0% | 8.6% | 8.9% | 100.0% |
| Ex. 34 | 59.6 | 0.0% | 2.3% | 0.0% | 100.0% |

(continued)

| Table 21-3. Adsorption ratios of alkali metals, ammonia, and TMA | | | | | |
|---|---|---|---|---|---|
| | SAR | Na adsorption ratio | $NH_4$ adsorption ratio | K adsorption ratio | TMA adsorption ratio |
| Ex. 70 | 59.6 | 0.0% | 0.2% | 0.05 | 100.0% |

Essential Metal Elements

An adsorption study of essential metal elements for human was conducted for the zeolites of Examples 5, 34, and 70, and Comparative Example 6. 3 mg of zeolite was accurately weighed, and 9.25 mL of a 0.1 mol/L nitric acid aqueous solution prepared by diluting 1 mol/L nitric acid aqueous solution 10-fold with ultrapure water, 0.15 mL of a 0.1 mol/L cobalt(II) nitrate aqueous solution, 0.14 mL of a 0.1 mol/L manganese(II) nitrate aqueous solution, 0.16 mL of a 0.1 mol/L zinc(II) nitrate aqueous solution, 0.16 mL of a 0.1 mol/L copper(II) nitrate aqueous solution, and 0.14 mL of a 0.2 mol/L iron(III) nitrate aqueous solution were added, and the mixture was stirred at room temperature for 1 hour. This solution was centrifuged, and high-purity nitric acid (60%) was added to 1000 μL of the supernatant, and the volume was brought to 50 mL by diluting with ultrapure water ($HNO_3$ 2%). The essential metal elements were measured by ICP-AES, and the adsorption ratio was calculated. The results are shown in Table 22. The reagents used in the study are listed in Table 23.

[Table 22]

| Table 22. Adsorption ratios of essential metal elements | | | | | | |
|---|---|---|---|---|---|---|
| | SAR | Co adsorption ratio | Mn adsorption ratio | Zn adsorption ratio | Cu adsorption ratio | Fe adsorption ratio |
| Comp. Ex. 6 | 24.0 | 0.0% | 0.0% | 0.0% | 0.1% | 0.0% |
| Ex. 34 | 59.6 | 0.0% | 0.0% | 0.0% | 0.1% | 0.0% |
| Ex. 70 | 59.6 | 0.0% | 0.0% | 0.0% | 0.7% | 0.0% |
| Ex. 5 | 129000.0 | 0.0% | 0.0% | 0.0% | 0.0% | 0.0% |

[Table 23]

Table 23

| reagent name | product name | product code | supplier |
|---|---|---|---|
| 1 mol/L nitric acid aq. soln. | 1 mol/L nitric acid (1N) | 28583-08 | KANTO CHEMICAL CO.,INC. |
| 0.1 mol/L cobalt (II) nitrate aq. soln. | cobalt standard solution (Co 1000) | 08040-1B | KANTO CHEMICAL CO.,INC. |
| 0.1 mol/L manganese (II) nitrate aq. soln. | manganese standard solution (Mn 1000) | 25824-2B | KANTO CHEMICAL CO.,INC. |
| 0.1 mol/L zinc (II) nitrate aq. soln. | zinc standard solution (Zn 1000) | 48096-2B | KANTO CHEMICAL CO.,INC. |
| 0.1 mol/L copper (II) nitrate aq. soln. | copper standard solution (Cu 1000) | 08046-2B | KANTO CHEMICAL CO.,INC. |
| 0.2 mol/L iron (III) nitrate aq. soln. | iron standard solution (Fe 1000) | 20247-2B | KANTO CHEMICAL CO.,INC. |
| high purity nitric acid (60%) | nitric acid 1.38 | GE00247 | KANTO CHEMICAL CO.,INC. |

Amino Acids

An amino acid adsorption study was conducted for the zeolites of Example 5, Example 34, and Comparative Example 6, using the following two types of amino acid mixture solutions.

Amino Acids Mixture Standard Solution

To accurately weighed 3 mg of zeolite, 1.0 mL of Amino Acids Mixture Standard Solution, Type AN (high range type, FUJIFILM Wako Pure Chemical Corporation), 1.0 mL of Amino Acids Mixture Standard Solution, Type B (high range type, FUJIFILM Wako Pure Chemical Corporation), and 8 mL of phosphate buffer were added. The mixture was stirred at room temperature for 1 hour and centrifuged. The supernatant was ultra-filtered (MWCO 10 kDa), and the filtrate was subjected to amino acid analysis.

Note that the Amino Acids Mixture Standard Solution, Type AN (high range type) is a mixed standard solution of 25 types of amino acids including acidic and neutral amino acids, and the concentration of each component is 2.5 μmol/mL each for L-aspartic acid, L-threonine, L-serine, L-glutamic acid, glycine, L-alanine, L-citrulline, L-valine, L-cystine, L-methionine, L-isoleucine, L-leucine, L-tyrosine, L-phenylalanine, β-alanine, DL-3-aminoisobutyric acid, L-hydroxyproline, and L-proline; 1.25 μmol/mL each for O-phospho-L-serine, taurine, O-phosphoethanolamine, DL-2-aminobutyric acid, and L-cystathionine; 6.25 μmol/mL for sarcosine; and 50 μmol/mL for urea. The Amino Acids Mixture Standard Solution, Type B (high range type) is a mixed standard solution of 12 types of amino acids including 3 types of basic amino acids, and contains 2.5 μmol/mL each of 4-aminobutyric acid, ethanolamine, ammonium chloride, 5-hydroxy-DL-lysine, L-ornithine, L-lysine, 1-methyl-L-histidine, L-histidine, 3-methyl-L-histidine, L-anserine, L-carnosine, and L-arginine.

Unstable Amino Acid Mixture Solution

To accurately weighed 3 mg of zeolite, 10 mL of an unstable amino acid mixture solution (containing 40.8 μg (0.20 μmol) of L-tryptophan, 29.2 μg (0.20 μmol) of L-glutamine, and 26.4 μg (0.20 μmol) of L-asparagine per 1 mL of ultrapure water) was added. The mixture was stirred at room temperature for 1 hour and centrifuged. The supernatant was ultra-filtered (MWCO 10 kDa), and the filtrate was subjected to amino acid analysis. The reagents used are listed in Table 24.

[Table 24]

Table 24

| reagent name | product name | product code | supplier |
|---|---|---|---|
| L-Tryptophan | L-Tryptophan | T8941 | Sigma-Aldrich |
| L-Glutamine | L-Glutamine | G8540 | Sigma-Aldrich |
| L-Asparagine | L-Asparagine | A0884 | Sigma-Aldrich |

The amino acid analysis was performed using a ninhydrin chromogenic assay (post-label method) with a high-speed amino acid analyzer L-8900 (Hitachi High-Tech Corporation). A 0.8 mL volume of the Amino Acid Mixture Standard Solution, Type AN and 0.8 mL of the Amino Acid Mixture Standard Solution, Type B were mixed, and the standard solution was prepared by diluting the mixture with ultrapure water to 10 mL. A calibration curve was obtained using a standard solution. 10 μL of the above filtrate was injected, and the concentrations of each amino acid in the filtrate was determined from the peak area using a one-point calibration curve to calculate the adsorption ratio.

The adsorption ratios for proteinogenic and non-proteinogenic amino acids are shown in Tables 25 and 26, respectively.

For the silicalite of Example 5, none of the proteinogenic amino acids were adsorbed. For the zeolite of Example 34, except for L-asparagine and L-glutamine, no adsorption of proteinogenic amino acids occurred. For the zeolite of Comparative Example 6, adsorption of the basic amino acids L-lysine and L-arginine was observed. It is considered that since Comparative Example 6 has a larger external surface area and a lower SAR compared to Example 34, adsorption of basic amino acids to the external surface acid sites was more likely to occur. Regarding non-proteinogenic amino acids, adsorption of 4-aminobutyric acid and ethanolamine was observed for Example 34. Both are primary amines consisting of a linear alkyl chain, and considering the minimum diameter of the molecule, it is possible for them to enter the zeolite pores, so it is possible that not only external surface adsorption but also internal adsorption occurred. Most other non-proteinogenic amino acids were hardly adsorbed regardless of their molecular size. All are highly water-soluble compounds, and it is considered that the hydrated state was more stable than being inside the hydrophobic zeolite pores. In particular, urea was concerned for competitively inhibiting the adsorption of amines to the zeolite, because it is abundant in the living gastrointestinal tract and is smaller than the pore size. However, urea was not adsorbed by the zeolites in this study. These zeolites showed important selectivity for pharmacological efficacy in vivo.

[Table 25]

Table 25. Adsorption ratios for proteinogenic amino acids

| zeolite (SAR) / amino acid | Comp. Ex. 6 (24.0) | Ex. 34 (59.6) | Ex. 5 (129000.0) |
|---|---|---|---|
| L-Aspartic acid | 0.0 | 0.0 | 0.0 |
| L-Threonine | 0.0 | 0.0 | 0.0 |
| L-Serine | 0.0 | 0.0 | 0.0 |
| L-Glutamic acid | 0.0 | 0.0 | 0.0 |
| Glycine | 0.0 | 0.0 | 0.0 |
| L-Alanine | 0.0 | 0.0 | 0.0 |
| L-Valine | 0.0 | 0.0 | 0.0 |
| L-Cystine | 0.0 | 0.0 | 0.0 |
| L-Methionine | 0.0 | 0.0 | 0.0 |
| L-Isoleucine | 0.0 | 0.0 | 0.0 |
| L-Leucine | 0.0 | 0.0 | 0.0 |
| L-Tyrosine | 0.0 | 0.0 | 0.0 |
| L-Phenylalanine | 0.0 | 0.0 | 0.0 |
| L-Lysine | 2.1 | 0.0 | 0.0 |
| L-Histidine | 0.0 | 0.0 | 0.0 |
| L-Arginine | 10.6 | 0.0 | 0.0 |
| L-Proline | 0.0 | 0.0 | 0.0 |
| L-Asparagine | 17.8 | 7.7 | 0.0 |
| L-Glutamine | 38.5 | 39.2 | 0.0 |
| L-Tryptophan | 0.0 | 0.0 | 0.0 |

(unit: %)

[Table 26]

Table 26. Adsorption ratios for non-proteinogenic amino acids

| zeolite (SAR) / amino acid | Comp. Ex. 6 (24.0) | Ex. 34 (59.6) | Ex. 5 (129000.0) |
|---|---|---|---|
| O-Phospho-L-Serine | 0.0 | 0.0 | 0.0 |
| Taurine | 0.0 | 0.0 | 0.0 |
| O-Phosphoethanolamine | 0.0 | 0.0 | 0.0 |
| Urea | 0.0 | 0.0 | 0.0 |
| Sarcosine | 1.2 | 2.2 | 0.0 |
| L-Citrulline | 0.0 | 0.0 | 0.0 |
| DL-2-Aminobutyric acid | 0.0 | 0.0 | 0.0 |
| β-Alanine | 2.8 | 1.4 | 0.0 |
| DL-3-Aminoisobutyric acid | 0.0 | 0.0 | 0.0 |
| 4-Aminobutyric acid | 14.3 | 9.1 | 0.0 |
| Ethanolamine | 0.0 | 9.1 | 0.0 |
| 5-Hydroxy-DL-Lysine | 0.0 | 0.0 | 0.0 |
| L-Ornithine | 0.0 | 0.0 | 0.0 |
| 1-Methyl-L-Histidine | 0.0 | 0.0 | 0.0 |
| 3-Methyl-L-Histidine | 0.4 | 0.0 | 0.0 |
| L-Anserine | 0.0 | 0.0 | 0.0 |
| L-Carnosine | 0.0 | 0.0 | 0.0 |
| L-Hydroxyproline | 0.0 | 0.0 | 0.0 |

(unit: %)

Vitamins

An adsorption study for vitamins was conducted for the zeolites of Example 34, Example 70, and Comparative Example 6. 3 mg of zeolite was accurately weighed, and 10 mL of ultrapure water was added thereto. To the zeolite suspension was added 100 μL of an aqueous solution of 25 μmol of a vitamin dissolved in 1.0 mL of ultrapure water, and the mixture was stirred at room temperature for 1 hour. This solution was centrifuged, and the vitamin concentration in the supernatant was measured by LC-UV (detection wavelength 210 nm). The results are shown in Table 27. The reagents used are listed in Table 28. Note that each vitamin was not mixed, and the adsorption study and analysis were performed individually for one type at a time.

For the fat-soluble vitamins (VA, $VD_3$, VE, and $VK_1$), except for the adsorption ratio of $VK_1$ which was about 50%, the adsorption ratios of VA, $VD_3$, and VE were 15% or less. Since all fat-soluble vitamins have large molecular sizes and are not considered to be able to enter the MFI-type zeolite pores, this is thought to be due to external surface adsorption. Since Example 34 has a higher SAR than Comparative Example 6, the lipophilicity of the external surface is higher, and it is considered that the lipophilicity of the external surface of Example 70 is even higher due to the surface treatment. It can be seen that these external surface lipophilicities are reflected in the higher adsorption ratios of the fat-soluble vitamins.

Among the water-soluble vitamins ($VB_1$, $VB_3$, $VB_5$, $VB_6$, $VB_{12}$, VC), $VB_1$, $VB_5$, $VB_6$ (3 types), and $VB_{12}$ were hardly adsorbed by any of the zeolites (about 10% at most). On the other hand, the water-soluble vitamin VC was adsorbed by about 10-30%, and $VB_3$ (2 types) was adsorbed by about 40-60%. It is assumed that VC, which has many hydroxyl groups, is surface-adsorbed by multi-point hydrogen bonding with the silanols on the external surface of the zeolite, and it is considered that the adsorption ratio was higher for Comparative Example 6, which has a larger external surface area, than for Example 34, which has a smaller external surface area. Also, since VC is an acidic compound, it is considered that external surface adsorption was more likely to occur on Example 70, which had fewer external surface acid sites due to surface treatment, than on Example 34, which had remaining external surface acid sites. Regarding $VB_3$, it has a pyridine structure, which is a tertiary amine, and is considered to be easily stabilized within the zeolite pores, which have an anionic framework. $VB_6$ also has a pyridine structure, but it has many substituents on the pyridine, and it is thought that adsorption into the pores did not occur due to its size. The other water-soluble vitamins that were difficult to adsorb are thought to have been unable to enter the zeolite pores because their molecular sizes are larger than those of the water-soluble vitamins that were easily adsorbed, and they existed stably in a hydrated state due to their high water solubility. The possibility of applying the zeolites of the examples as carriers for the adsorption and removal, sustained-release formulations, or controlled-release formulations of heterocyclic amines with few or small substituents, such as $VB_3$ (2 types), was shown.

[Table 27]

Table 27. Adsorption ratios of vitamins

| vitamin \ zeolite (SAR) | Comp. Ex. 6 (24.0) | Ex. 34 (59.6) | Ex. 70 (59.6) |
|---|---|---|---|
| VA | 1.2 | 9.1 | 12.2 |
| VB1 | 2.5 | 0.8 | 0.0 |
| VB2 | 4.2 | 1.8 | 8.0 |
| VB3 (nicotinic acid) | 58.8 | 57.6 | 42.8 |
| VB3 (nicotinamide) | 51.4 | 49.4 | 37.0 |
| VB5 | 10.5 | 9.5 | 1.9 |
| VB6 (pyridoxamine) | 4.5 | 1.7 | 0.1 |
| VB6 (pyridoxal) | 3.6 | 0.0 | 0.0 |
| VB6 (pyridoxine) | 2.1 | 0.0 | 0.0 |
| VB7 | 4.4 | 0.0 | 0.0 |
| VB9 | 5.1 | 0.0 | 0.0 |
| VB12 | 1.4 | 3.1 | 3.8 |
| VC | 23.2 | 10.8 | 29.9 |
| VD3 | 0.5 | 8.8 | 14.4 |
| VE | 2.3 | 4.5 | 2.0 |
| VK1 | 50.2 | 49.2 | 47.2 |

(unit: %)

[Table 28]

| Table 28. Reagents used for vitamin adsorption studies | | | |
|---|---|---|---|
| reagent name | product name | product code | supplier |
| VA | Retinol | R7632-100MG | Sigma-Aldrich |
| VB1 | Thiamine Hydrochloride | 201-00852 | FUJIFILM Wako Pure Chemical Corporation |
| VB2 | Riboflavin Standard | 182-01611 | FUJIFILM Wako Pure Chemical Corporation |
| VB3 (nicotinic acid) | Nicotinic acid | N0082 | Tokyo Chemical Industry Co., Ltd. |
| VB3 (nicotinamide) | Nicotinamide | 24317-72 | NACALAI TESQUE, INC. |

(continued)

| Table 28. Reagents used for vitamin adsorption studies | | | |
|---|---|---|---|
| reagent name | product name | product code | supplier |
| VB5 | Calcium (+)-Pantothenate | 031-14161 | FUJIFILM Wako Pure Chemical Corporation |
| VB6 (pyridoxamine) | Pyridoxamine Dihydrochloride Standard | 162-20051 | FUJIFILM Wako Pure Chemical Corporation |
| VB6 (pyridoxal) | Pyridoxal Hydrochloride Standard | 165-20041 | FUJIFILM Wako Pure Chemical Corporation |
| VB6 (pyridoxine) | Pyridoxine Hydrochloride | 165-05401 | FUJIFILM Wako Pure Chemical Corporation |
| VB7 | Biotin | B0463 | Tokyo Chemical Industry Co., Ltd. |
| VB9 | Folic Acid Standard | 062-06161 | FUJIFILM Wako Pure Chemical Corporation |
| VB12 | Vitamin B12 | C0449 | Tokyo Chemical Industry Co., Ltd. |
| VC | L(+)-Ascorbic Acid Standard | 011-16641 | FUJIFILM Wako Pure Chemical Corporation |
| VD3 | Cholecalciferol | C0314 | Tokyo Chemical Industry Co., Ltd. |
| VE | D-α-Tocopherol | T2309 | Tokyo Chemical Industry Co., Ltd. |
| VK1 | Vitamin K1 | 221-00371 | FUJIFILM Wako Pure Chemical Corporation |

Short-Chain Fatty Acids

An adsorption study for short-chain fatty acids was conducted for the zeolites of Example 5, Example 34, Example 70, and Comparative Example 6. 3 mg of zeolite was accurately weighed, and 9.44 mL of ultrapure water was added thereto. Furthermore, 0.143 mL, 0.187 mL, and 0.229 mL of acetic acid, propionic acid, and butyric acid, respectively, were added to achieve a concentration of 2.5 μmol for each component. A stir bar was added to this solution, and the mixture was stirred for 1 hour. After removing the stir bar, the solution was centrifuged. After centrifugation, the solution, diluted 10,000-fold with ultrapure water, was measured by ion chromatography. The reagents used are listed in Table 29.

None of the zeolites adsorbed the short-chain fatty acids. It is considered that the short-chain fatty acids, which dissociate in aqueous solution to produce anions of their conjugate bases, are not adsorbed into the zeolite because stabilization by electrostatic interaction with the zeolite, which consists of an anionic framework, does not occur. Short-chain fatty acids are abundant in the living gastrointestinal tract and are molecules of a size that can enter the zeolite pores, so there was a concern that they might competitively inhibit the adsorption of amines to the zeolite. This study confirmed the important selectivity for the zeolite to show pharmacological efficacy in vivo.

[Table 29]

| Table 29. Reagents used for short-chain fatty acid adsorption studies | | | |
|---|---|---|---|
| reagent name | product name | product code | supplier |
| Acetic Acid | Acetic Acid | 018-20061 | FUJIFILM Wako Pure Chemical Corporation |
| Propionic Acid | Propionic Acid | 169-04723 | FUJIFILM Wako Pure Chemical Corporation |
| Butyric Acid | Butyric Acid | B0754 | Tokyo Chemical Industry Co., Ltd. |

Concomitant Drugs

An adsorption study was conducted using the zeolites of Examples 34, 70, and 79 for drugs that may be co-administered with the inorganic porous material oral composition of Examples (hereinafter referred to as concomitant drugs). 3 mg of zeolite was accurately weighed, and 9.9 mL of ultrapure water was added thereto. To the zeolite suspension, 100 μL of a DMSO solution of a concomitant drug (25 μmol dissolved in 1.0 mL of DMSO) was added, and the mixture was stirred at room temperature for 1 hour. This solution was centrifuged, and the concentration of the concomitant drug in the supernatant was measured by LC-MS/MS. The adsorption ratio (expressed as a percentage of the value obtained by subtracting from 1 the value obtained by dividing the concentration of the concomitant drug in the supernatant by the initial concentration of 250 μmol/L. Negative values were expressed as 0) was calculated. The results are shown in Table 30. The reagents used are listed in Table 31. For carvedilol and miconazole, after suspending the zeolite in ultrapure water, 10 μL of 1N hydrochloric acid was added to increase the solubility of the basic compounds carvedilol and miconazole (the pH of the solution immediately after adding the DMSO solution of the concomitant drug was 6-7). Note that each concomitant drug was not mixed, and the adsorption study and analysis were performed individually for one type at a time.

As a result, almost none of the acidic compounds were adsorbed (adsorption ratio < 4%). Although it is known that p-xylene is adsorbed by MFI zeolite, almost no adsorption from water occurred even for acetaminophen and actarit, which have a similar p-substituted benzene structure and could be adsorbed in terms of molecular size. It is considered that internal adsorption of acidic compounds into the zeolite was disadvantageous for the same reason as for short-chain fatty acids.

The neutral compounds pirfenidone and chloramphenicol were not adsorbed at all, and the adsorption ratio of lactulose was also low. Since neutral compounds have no electrostatic interaction with the surface acid sites, it is considered that their adsorption is less likely to occur compared to basic compounds. Since lactulose has many hydroxyl groups, it is possible that weak external surface adsorption occurred due to multi-point hydrogen bonding with the silanols on the external surface of the zeolite.

For basic compounds, weak adsorption was confirmed for some compounds. Since metformin is a basic compound with a small molecular size, it is thought to have been partially adsorbed into the zeolite. At this time, the metformin adsorption ratio was lower for the surface-treated Examples 72 and 80 than for the non-surface-treated Example 34. It is considered that the surface treatment partially prevented the adsorption of metformin to the acid sites on the external surface. For the other concomitant drugs for which weak adsorption was confirmed (mirtazapine, ondansetron, lincomycin, oseltamivir, carvedilol), since their molecular sizes are considered to exceed the pore diameter of MFI zeolite, it is thought to be due to external surface adsorption rather than internal adsorption.

[Table 30]

Table 30. Adsorption ratio of concomitant drugs

| concomitant drugs | | Molecular weight of the free form | pKa | pKa of conjugate acid | adsorption ratio to zeolite (%) | | |
|---|---|---|---|---|---|---|---|
| | | | | | Ex. 34 | Ex. 70 | Ex. 79 |
| acidic compounds | Actarit | 193.20 | 3.94 | - | 0.8 | 0.0 | 0.0 |
| | Allopurinol | 136.11 | 9.50 | - | 0.0 | 2.4 | 3.6 |
| | Acetaminophen | 151.16 | 9.50 | - | 0.0 | 3.6 | 1.2 |
| neutral compounds | Pirfenidone | 185.22 | - | - | 0.0 | 0.0 | 0.0 |
| | Chloramphenicol | 323.13 | - | - | 0.0 | 0.0 | 0.0 |
| | Lactulose | 342.30 | - | - | 10.4 | 10.4 | 2.0 |

(continued)

| Table 30. Adsorption ratio of concomitant drugs | | | | | | | |
|---|---|---|---|---|---|---|---|
| concomitant drugs | | Molecular weight of the free form | pKa | pKa of conjugate acid | adsorption ratio to zeolite (%) | | |
| | | | | | Ex. 34 | Ex. 70 | Ex. 79 |
| basic compounds | Metronidazole | 171.15 | - | 2.6 | 9.2 | 10.0 | 8.4 |
| | Mirtazapine | 265.35 | - | 3.71 | 27.2 | 17.6 | 21.6 |
| | Miconazole | 416.13 | - | 5.65 | 0.0 | 0.0 | 0.0 |
| | Miglitol | 207.22 | - | 5.9 | 0.0 | 0.8 | 4.8 |
| | Carbamazepine | 236.27 | - | 7 | 0.4 | 0.0 | 1.6 |
| | Ondansetron | 293.36 | - | 7.4 | 26.4 | 24.8 | 30.0 |
| | Lincomycin | 406.54 | - | 7.6 | 17.2 | 14.8 | 16.4 |
| | Oseltamivir | 312.41 | - | 7.75 | 12.8 | 15.6 | 6.8 |
| | Carvedilol | 406.48 | - | 7.8 | 11.2 | 0.0 | 4.4 |
| | Memantine | 179.30 | - | 10.58 | 0.0 | 0.0 | 0.0 |
| | Metformin | 129.16 | - | 12.4 | 30.8 | 14.8 | 16.4 |

[Table 31]

| Table 31. Reagents used for concomitant drug adsorption studies | | | |
|---|---|---|---|
| reagent name | product name | product code | supplier |
| Actarit | Actarit | A2793 | Tokyo Chemical Industry Co., Ltd. |
| Allopurinol | Allopurinol | 011-12501 | FUJIFILM Wako Pure Chemical Corporation |
| Acetaminophen | Acetaminophen Standard | 015-22651 | FUJIFILM Wako Pure Chemical Corporation |
| Pirfenidone | Pirfenidone | P1871 | Tokyo Chemical Industry Co., Ltd. |
| Chloramphenicol | Chloramphenicol Standard | 037-19641 | FUJIFILM Wako Pure Chemical Corporation |
| Lactulose | Lactulose | L0140 | Tokyo Chemical Industry Co., Ltd. |
| Metronidazole | Metronidazole Standard | 139-14931 | FUJIFILM Wako Pure Chemical Corporation |
| Mirtazapine | Mirtazapine | M2151 | Tokyo Chemical Industry Co., Ltd. |
| Miconazole | Miconazole Nitrate | 134-12661 | FUJIFILM Wako Pure Chemical Corporation |
| Miglitol | Miglitol | M2302 | Tokyo Chemical Industry Co., Ltd. |
| Carbamazepine | Carbamazepine Standard | 036-21441 | FUJIFILM Wako Pure Chemical Corporation |

(continued)

| Table 31. Reagents used for concomitant drug adsorption studies | | | |
|---|---|---|---|
| reagent name | product name | product code | supplier |
| Ondansetron | Ondansetron Hydrochloride Dihydrate | 00407 | Tokyo Chemical Industry Co., Ltd. |
| Lincomycin | Lincomycin Hydrochloride Monohydrate Standard | 121-05781 | FUJIFILM Wako Pure Chemical Corporation |
| Oseltamivir | Oseltamivir Phosphate | 153-03441 | FUJIFILM Wako Pure Chemical Corporation |
| Carvedilol | Carvedilol | C2260 | Tokyo Chemical Industry Co., Ltd. |
| Memantine | 3,5-Dimethyl-1-adamantanamine Hydrochloride | D3608 | Tokyo Chemical Industry Co., Ltd. |
| Metformin | 1,1-Dimethylbiguanide Hydrochloride | 320-22391 | FUJIFILM Wako Pure Chemical Corporation |

TMA Precursors

An adsorption study for TMA precursors was conducted for the zeolites of Example 34, Example 70, and Comparative Example 6. 3 mg of zeolite was accurately weighed, and 10 mL of ultrapure water was added thereto. To the zeolite suspension was added 100 μL of an aqueous solution containing 25 μmol of betaine (trimethylglycine) or carnitine dissolved in 1.0 mL of ultrapure water, and the mixture was stirred at room temperature for 1 hour. This solution was centrifuged, and the concentration of betaine or carnitine in the supernatant was measured by LC-CAD (Charged Aerosol Detector). The results are shown in Table 32.

Betaine was adsorbed by all zeolites, and carnitine was adsorbed to all zeolite except for Example 34. Betaine has almost the same molecular size as choline, and it is considered that internal adsorption is occurring. On the other hand, carnitine has a larger molecular size than betaine or choline, and it is considered that internal adsorption is unlikely to occur. For Comparative Example 6, the adsorption amounts of betaine and carnitine were larger than those of Examples 34 and 70. It is considered that since Comparative Example 6 has a larger external surface area and a lower SAR compared to Examples 34 and 70, the adsorption of basic substances to the external surface is more likely to occur. The reagents used are listed in Table 33.

Betaine and carnitine, like choline, are TMA precursors and are also known as nutrients. Since they are decomposed by bacteria in the gastrointestinal tract to produce TMA, adsorbing and releasing these TMA precursors affects the absorption amount of TMA. As with choline, there is a concern that the zeolite may act as a substance for delivering these TMA precursors to the colon, so it is desirable that they are not adsorbed to the zeolite. In this study, it was confirmed that the adsorption of betaine and carnitine was weak for Examples 34 and 70. Since the study was not conducted in the presence of TMA, it is considered that if the study is performed in the presence of TMA, competition will occur, and the adsorption ratios of betaine and carnitine will be even lower.

Note that TMAO, which is also known as a TMA precursor, does not have a basic structure like betaine and carnitine and is highly hydrophilic, so it was considered obvious that it would not be adsorbed to the zeolite in water, and no adsorption study was performed.

[Table 32]

Table 32. Adsorption ratios of TMA precursors

| TMA precursor \ zeolite (SAR) | Comp. Ex. 6 (24.0) | Ex. 34 (59.6) | Ex. 70 (59.6) |
|---|---|---|---|
| Betaine | 38.9 | 11.7 | 10.5 |
| Carnitine | 23.9 | 0.0 | 4.6 |

(unit: %)

[Table 33]

| Table 33. Reagents used for TMA precursor adsorption studies | | | |
|---|---|---|---|
| reagent name | product name | product code | supplier |
| Betaine | Betaine | 023-10862 | FUJIFILM Wako Pure Chemical Corporation |
| Carnitine | L-Carnitine hydrochloride | C0283-1G | Sigma-Aldrich |

Serotonin

An adsorption study for serotonin was conducted for the zeolites of Example 34, Example 70, and Comparative Example 6. 3 mg of zeolite was accurately weighed, and 10 mL of ultrapure water was added thereto. To the zeolite suspension was added 100 μL of an aqueous solution of 25 μmol of serotonin (5-Hydroxytryptamine, FUJIFILM Wako Pure Chemical Corporation) dissolved in 1.0 mL of ultrapure water, and the mixture was stirred at room temperature for 1 hour. This solution was centrifuged, and the serotonin concentration in the supernatant was measured by LC-CAD. The results are shown in Table 34.

A small amount of adsorption was observed only for Comparative Example 6. It is considered that serotonin was surface-adsorbed to Comparative Example 6, which has a large external surface area. Serotonin is a bioactive substance present in the gastrointestinal tract, and there is a concern that if it is adsorbed, it may affect gastrointestinal motility and other biological reactions involving serotonin. However, this study suggested that it is hardly adsorbed by MFI-type zeolite.

[Table 34]

| Table 34. Adsorption ratio of Serotonin | | | |
|---|---|---|---|
| Zeolite (SAR) | Comp. Ex. 6 (24.0) | Ex. 34 (59.6) | Ex. 70 (59.6) |
| Serotonin adsorption ratio | 3.8 | 0.0 | 0.0 |
| (unit: %) | | | |

Reference Examples

The following describes the methods for obtaining and synthesizing the zeolites of the examples and comparative examples used in the evaluation in the above examples, as well as the methods for measuring the physical properties of the zeolites.

[Examples 1-3, Comparative Examples 1-14]

Table 35 lists the origins of the compounds of Examples 1-3 and Comparative Examples 1-14. For Example 1, Example 3, and Comparative Examples 1-14, commercial products were purchased and used as is. For Example 2, the purchased zeolite was converted from the ammonium form to the proton form by calcination at 500°C for 4 hours under air flow.

[Table 35]

Table 35. Purchased compounds

| | framework code | counter cation | SAR | product name (product code) | supplier |
|---|---|---|---|---|---|
| Comp. Ex. 1 | - | - | - | Charcoal, Activated, Powder (037-02115) | FUJIFILM Wako Pure Chemical Corporation |
| Comp. Ex. 2 | - | - | - | Lokelma® | Astrazeneca |
| Comp. Ex. 3 | HEU | Na, K, Ca | 6.0 | Clinoptilolite (SIC2433.0) | GELEST |
| Comp. Ex. 4 | LTA | Na | 2.0 | Molecular sieves 4A powder | Union Showa K. K. |
| Comp. Ex. 5 | LTA | Na | 2.6 | Molecular sieves 4A 1/16 (137-06085) | FUJIFILM Wako Pure Chemical Corporation |
| Ex. 1 | FER | K | 18.0 | HSZ-700 (720KOA) | Tosoh Corporation |
| Comp. Ex. 6 | MFI | H | 24.0 | HSZ-800 (822HOA) | Tosoh Corporation |
| Ex. 2 | MFI | H | 1000.0 | CP-7123 | Zeolyst |
| Ex. 3 | MFI | H | 1600.0 | H-SiV3000 | Union Showa K. K. |
| Comp. Ex. 7 | Beta | $NH_4$ | 25.0 | Zeolite beta, ammonium (45873) | Alfa Aesar |
| Comp. Ex. 8 | Beta | H | 40.0 | HSZ-900 (940HOA) | Tosoh Corporation |
| Comp. Ex. 9 | MOR | $NH_4$ | 20.0 | Zeolite mordenite, ammonium (45877) | Alfa Aesar |
| Comp. Ex. 10 | MOR | H | 30.0 | HSZ-600 (660HOA) | Tosoh Corporation |
| Comp. Ex. 11 | FAU | Na | 6.0 | HSZ-300 (320NAA) | Tosoh Corporation |
| Comp. Ex. 12 | FAU | H | 30.0 | CBV-720 | Zeolyst |
| Comp. Ex. 13 | FAU | H | 60.0 | CBV-760 | Zeolyst |
| Comp. Ex. 14 | FAU | H | 2000.0 | ABSCENTS 2000 | Union Showa K. K. |

Note that each item in Table 35 indicates the following, respectively.

Framework code: The framework code of the zeolite used in the example, which conforms to the code defining the framework structure of zeolites established by the IZA (International Zeolite Association).
Counter cation: The counter cation of the zeolite used in the example.
SAR: The molar ratio of $SiO_2$ to $Al_2O_3$ of the zeolite used in the example.
Product Name (Product Code): The product name of the compound used in the example. The product code is in parentheses.
Supplier: The supplier of the compound used in the example.

[Synthesis Method of Comparative Example 15]

[Hydrothermal Synthesis and Calcination of Organic Structure-Directing Agent]

To a container were sequentially added 4.5 g of sodium hydroxide (NaOH) granules, 3.8 g of potassium hydroxide (KOH) granules, 66.6 g of a 25 mass% aqueous solution of N,N,N-trimethyl-1-adamantylammonium hydroxide (TMAdaOH) from Sachem, Inc. as the Organic Structure-Directing Agent (hereinafter referred to as OSDA), 195 g of desalinated water, 7.1 g of "KYOWAAD 200S" (alumina content 53.5 mass%) from Kyowa Chemical Industry Co., Ltd. as aluminum hydroxide, and 225 g of colloidal silica "Cataloid SI-30" from JGC C&C as silica. The composition and molar ratio of the resulting mixture were $SiO_2$:$Al_2O_3$:NaOH:KOH:TMAdaOH:$H_2O$ = 1.0:0.033:0.1:0.06:0.07:20. After these ingredients were thoroughly mixed, the resulting mixture was placed in a pressure vessel and hydrothermally synthesized at 160°C for 48 hours under a rotation condition of 100 rpm. The resulting gel was subjected to suction filtration, washed with desalinated water, and then dried by heating at 100°C for 8 hours or more (hereinafter, unless otherwise specified, the drying operation was performed under these conditions). The OSDA was removed from the powder by calcining the resulting powder at 550°C for 6 hours under air flow.

[Ion Exchange and Calcination]

The powder from which the OSDA had been removed was dispersed at 10 mass% in a 1 mol/L ammonium nitrate aqueous solution prepared from ammonium nitrate and desalinated water, and ammonium ion exchange was performed at 80°C for 2 hours, followed by suction filtration and washing with desalinated water. Thereafter, the ion exchange and washing were repeated twice more. Furthermore, after drying the ion-exchanged powder, it was calcined at 500°C for 4 hours under air flow to obtain a proton form zeolite.

[Synthesis Method of Comparative Example 16]

To a container were sequentially added 1.0 g of sodium hydroxide granules, 8.7 g of an 18 mass% aqueous solution of 1,1'-(1,4-butanediyl)bis(1-azonia-4-azabicyclo[2.2.2]octane) dihydroxide (Dab-4) from Sachem, Inc. as the OSDA, 8 g of desalinated water, 0.94 g of "KYOWAAD 200S" as aluminum hydroxide, 7.5 g of "Snowtex 40" as silica, and 0.15 g of the CHA-type zeolite synthesized in Comparative Example 15 as seed crystals. The composition and molar ratio of the resulting mixture were $SiO_2$:$Al_2O_3$:NaOH:Dab-4:$H_2O$ = 1.0:0.1:0.5:0.1:22. After these ingredients were thoroughly mixed, the resulting mixture was placed in a pressure vessel and hydrothermally synthesized in an oven at 140°C for 120 hours under static conditions. The resulting gel was subjected to suction filtration, washed with desalinated water, and then dried. The OSDA was removed from the powder by calcining the resulting powder at 550°C for 6 hours under air flow. Furthermore, the [Ion Exchange and Calcination] operation of Comparative Example 15 was performed to obtain a proton form zeolite.

[Synthesis Method of Comparative Example 17]

To a container were sequentially added 0.772 g of sodium hydroxide granules, 2.262 g of a 35 mass% aqueous solution of N,N-dimethyl-3,5-dimethylpiperidinium hydroxide (DMDMPIOH) from Sachem, Inc. as the OSDA, 9.9 g of desalinated water, 0.726 g of "KYOWAAD 200S" as aluminum hydroxide, 7.454 g of "Snowtex 40" as silica, and 0.300 g of the CHA-type zeolite synthesized in Comparative Example 15 as seed crystals. The composition and molar ratio of the resulting mixture were $SiO_2$:$Al_2O_3$:NaOH:DMDMPIOH:$H_2O$ = 1.0:0.013:0.4:0.1:17.5. After these ingredients were thoroughly mixed, the resulting mixture was placed in a pressure vessel and hydrothermally synthesized in an oven at 180°C for 24 hours under a rotation condition of 15 rpm. The resulting gel was subjected to suction filtration, washed with desalinated water, and then dried. The OSDA was removed from the powder by calcining the resulting powder at 600°C for 6 hours under air flow. Furthermore, the [Ion Exchange and Calcination] operation of Comparative Example 15 was performed to obtain a proton form zeolite.

[Synthesis Method of Comparative Example 18]

To a container were sequentially added 1.135 g of sodium hydroxide granules, 4.959 g of hexamethyleneimine (HMI) from Tokyo Chemical Industry Co., Ltd. as the OSDA, 63.2 g of desalinated water, 0.944 g of "KYOWAAD 200S" as aluminum hydroxide, and 14.908 g of "Snowtex 40" as silica. The composition and molar ratio of the resulting mixture were $SiO_2$:$Al_2O_3$:NaOH:HMI:$H_2O$ = 1.0:0.033:0.3:0.5:40. After these ingredients were thoroughly mixed, the resulting mixture was placed in a pressure vessel and hydrothermally synthesized in an oven at 150°C for 144 hours under static conditions. The resulting gel was subjected to suction filtration, washed with desalinated water, and then dried. The OSDA was removed from the powder by calcining the resulting powder at 550°C for 6 hours under air flow. Furthermore, the [Ion Exchange and Calcination] operation of Comparative Example 15 was performed to obtain a proton form zeolite.

[Synthesis Method of Comparative Example 19]

In Comparative Example 19, the synthesis of the zeolite was performed in the same manner as in the synthesis method of Comparative Example 18, except that the composition and molar ratio of the ingredient mixture were changed to $SiO_2$:$Al_2O_3$:NaOH:HMI:$H_2O$ = 1.0:0.02:0.3:0.5:40.

[Synthesis Method of Comparative Example 20]

To a container were sequentially added 7.750 g of a 1 mol/L sodium hydroxide aqueous solution, 6.487 g of a 40 mass% aqueous solution of tetrabutylammonium hydroxide (TBAOH) from Tokyo Chemical Industry Co., Ltd. as the OSDA, 15.9 g of desalinated water, 0.629 g of "KYOWAAD 200S" as aluminum hydroxide, and 14.908 g of "Snowtex 40" as silica. The composition and molar ratio of the resulting mixture were $SiO_2:Al_2O_3:NaOH:TBAOH:H_2O$ = 1.0:0.033:0.1:0.1:20. After these ingredients were thoroughly mixed, the resulting mixture was placed in a pressure vessel and hydrothermally synthesized in an oven at 175°C for 72 hours under a rotation condition of 15 rpm. The resulting gel was subjected to suction filtration, washed with desalinated water, and then dried. The OSDA was removed from the powder by calcining the resulting powder at 550°C for 6 hours under air flow. Furthermore, the [Ion Exchange and Calcination] operation of Comparative Example 15 was performed to obtain a proton form zeolite.

[Synthesis Method of Comparative Example 21]

In Comparative Example 21, the synthesis of the zeolite was performed in the same manner as in Comparative Example 20, except that the composition and molar ratio of the ingredient mixture were changed to $SiO_2:Al_2O_3:NaOH:TBAOH:H_2O$ = 1.0:0.02:0.1:0.1:20.

[Comparative Examples 15-21]

For the zeolites of Comparative Examples 15-21, the framework code and SAR are summarized in Table 36. The zeolite framework was identified by confirming the diffraction pattern of XRPD. The counter cation for all zeolites is a proton.

[Table 36]

Table 36.

| | framework code | SAR |
|---|---|---|
| Comp. Ex. 15 | CHA | 20.0 |
| Comp. Ex. 16 | AFX | 7.0 |
| Comp. Ex. 17 | AEI | 10.0 |
| Comp. Ex. 18 | MWW | 20.9 |
| Comp. Ex. 19 | MWW | 31.4 |
| Comp. Ex. 20 | MEL | 26.5 |
| Comp. Ex. 21 | MEL | 43.0 |

[Synthesis Method of Examples 4-64 and Comparative Example 22]

In Examples 4-34, Examples 37-64, and Comparative Example 22, the synthesis of the zeolite was performed in the same manner as the following procedure, except that the ingredients used, the amounts charged, the hydrothermal synthesis conditions, the stirring method, and the post-treatment were changed as shown in Tables 37-1, 37-2, and 37-3. All zeolites showed the diffraction pattern of MFI-type zeolite in XRPD.

To a container were sequentially added a 1 mol/L sodium hydroxide aqueous solution, 0 to 2 types of Structure-Directing Agents (hereinafter referred to as SDA), desalinated water, an aluminum (Al) source, a silicon (Si) source, and seed crystals. After these ingredients were thoroughly mixed, the resulting mixture was placed in a pressure vessel and hydrothermally synthesized. Thereafter, the resulting gel was subjected to suction filtration, thoroughly washed with desalinated water, and then dried by heating at 100°C for 8 hours or more in an air atmosphere. The resulting zeolite powder was subjected to post-treatment.

[Table 37-1]

Table 37-1. Synthesis conditions of MFI-type zeolite

| | Si source | Si amount | Al source | Ingredient SAR | Na amount | SDA1 | SDA1 amount | SDA2 | SDA2 amount | $H_2O$ amount | seed crystal | seed crystal SAR | seed crystal amount |
|---|---|---|---|---|---|---|---|---|---|---|---|---|---|
| Ex. 4 | TEOS | 0.05 | KYOWAAD 200S | 1000 | 0 | TPAOH | 0.2 | - | - | 100 | - | | 0 |
| Ex. 5 | TEOS | 0.05 | - | ∞ | 0 | TPAOH | 0.2 | - | - | 100 | - | | 0 |
| Ex. 6 | Snowtex 40 | 0.08 | - | ∞ | 0.1 | TPAOH | 0.05 | - | - | 15 | - | | 0 |
| Ex. 7 | TEOS | 0.05 | - | ∞ | 0 | TPAOH | 0.20 | - | - | 100 | - | | 0 |
| Ex. 8 | Snowtex 40 | 0.05 | KYOWAAD 200S | 40 | 0.18 | ED | 0.02 | - | - | 42 | MFI | 40 | 5 |
| Ex. 9 | Snowtex 40 | 0.1 | KYOWAAD 200S | 50 | 0.18 | ED | 0.02 | - | - | 42 | MFI | 40 | 5 |
| Ex.10 | Snowtex 40 | 0.1 | KYOWAAD 200S | 50 | 0.18 | DiEA | 0.02 | - | - | 42 | MFI | 40 | 5 |
| Ex. 11 | Snowtex 40 | 0.1 | KYOWAAD 200S | 50 | 0.18 | TriEA | 0.02 | - | - | 42 | MFI | 40 | 5 |
| Ex. 12 | Snowtex 40 | 0.05 | KYOWAAD 200S | 40 | 0.18 | EtOH | 0.01 | - | - | 42 | MFI | 40 | 5 |
| Ex. 13 | Snowtex 40 | 0.05 | KYOWAAD 200S | 40 | 0.18 | EtOH | 0.02 | - | - | 42 | MFI | 40 | 5 |
| Ex. 14 | Snowtex 40 | 0.05 | KYOWAAD 200S | 50 | 0.18 | EtOH | 0.02 | - | - | 42 | MFI | 40 | 5 |
| Ex. 15 | Snowtex 40 | 0.1 | KYOWAAD 200S | 50 | 0.18 | EtOH | 0.02 | - | - | 42 | MFI | 40 | 5 |
| Ex. 16 | Snowtex 40 | 0.1 | KYOWAAD 200S | 60 | 0.18 | EtOH | 0.02 | - | - | 42 | MFI | 40 | 5 |
| Ex. 17 | Snowtex 40 | 0.1 | KYOWAAD 200S | 70 | 0.18 | EtOH | 0.02 | - | - | 42 | MFI | 40 | 5 |
| Ex. 18 | Snowtex 40 | 0.1 | KYOWAAD 200S | 80 | 0.18 | EtOH | 0.02 | - | - | 42 | MFI | 40 | 5 |

(continued)

Table 37-1. Synthesis conditions of MFI-type zeolite

| | Si source | Si amount | Al source | Ingredient SAR | Na amount | SDA1 | SDA1 amount | SDA2 | SDA2 amount | $H_2O$ amount | seed crystal | seed crystal SAR | seed crystal amount |
|---|---|---|---|---|---|---|---|---|---|---|---|---|---|
| Ex. 19 | Snowtex 40 | 0.1 | KYOWAAD 200S | 100 | 0.18 | EtOH | 0.02 | - | - | 42 | MFI | 40 | 5 |
| Ex. 20 | Snowtex 40 | 0.1 | KYOWAAD 200S | 50 | 0.18 | BuOH | 0.02 | - | - | 42 | MFI | 40 | 5 |
| Ex. 21 | Snowtex 40 | 0.06 | KYOWAAD 200S | 75 | 0.2 | BuOH | 1 | - | - | 42 | MFI | 40 | 5 |
| Ex. 22 | Snowtex 40 | 0.05 | KYOWAAD 200S | 100 | 0.2 | BuOH | 1 | - | - | 40 | MFI | 40 | 5 |
| Ex. 23 | Snowtex 40 | 0.05 | KYOWAAD 200S | 40 | 0.18 | $Na_2CO_3$ | 0.09 | EtOH | 0.02 | 42 | MFI | 40 | 5 |
| Ex. 24 | Snowtex 40 | 0.08 | KYOWAAD 200S | 40 | 0.18 | $Na_2CO_3$ | 0.18 | EtOH | 0.02 | 42 | MFI | 40 | 5 |

Table 37-1-continued. Synthesis conditions of MFI-type zeolite

| | hydrothermal synthesis temperature | hydrothermal synthesis time | stirring rate | vessel | post-treatment |
|---|---|---|---|---|---|
| Ex. 4 | 150 | 5 | 15 | 0.2 | SDA removal |
| Ex. 5 | 150 | 5 | 15 | 0.2 | SDA removal |
| Ex. 6 | 160 | 48 | 15 | 0.2 | SDA removal |
| Ex. 7 | 160 | 5 | 0 | 0.2 | SDA removal |
| Ex. 8 | 175 | 72 | 0 | 0.2 | SDA removal, ion exchange/firing |
| Ex. 9 | 175 | 72 | 15 | 0.2 | SDA removal, ion exchange/firing |
| Ex.10 | 175 | 72 | 15 | 0.2 | SDA removal, ion exchange/firing |
| Ex. 11 | 175 | 72 | 15 | 0.2 | SDA removal, ion exchange/firing |
| Ex. 12 | 175 | 72 | 0 | 0.2 | SDA removal, ion exchange/firing |
| Ex. 13 | 175 | 72 | 0 | 0.2 | SDA removal, ion exchange/firing |
| Ex. 14 | 175 | 72 | 0 | 0.2 | SDA removal, ion exchange/firing |
| Ex. 15 | 175 | 72 | 15 | 0.2 | SDA removal, ion exchange/firing |
| Ex. 16 | 175 | 72 | 15 | 0.2 | SDA removal, ion exchange/firing |
| Ex. 17 | 175 | 72 | 15 | 0.2 | SDA removal, ion exchange/firing |
| Ex. 18 | 175 | 72 | 15 | 0.2 | SDA removal, ion exchange/firing |
| Ex. 19 | 175 | 72 | 15 | 0.2 | SDA removal, ion exchange/firing |
| Ex. 20 | 175 | 72 | 15 | 0.2 | SDA removal, ion exchange/firing |
| Ex. 21 | 175 | 72 | 15 | 0.2 | SDA removal, ion exchange/firing |
| Ex. 22 | 175 | 72 | 15 | 0.2 | SDA removal, ion exchange/firing |
| Ex. 23 | 175 | 72 | 0 | 0.2 | SDA removal, ion exchange/firing |
| Ex. 24 | 175 | 72 | 0 | 0.2 | SDA removal, ion exchange/firing |

Table 37-2. Synthesis conditions of MFI-type zeolite ~continued

| | Si source | Si amount | Al source | Ingredient SAR | Na amount | SDA1 | SDA1 amount | SDA2 | SDA2 amount | $H_2O$ amount | seed crystal | seed crystal SAR | seed crystal amount |
|---|---|---|---|---|---|---|---|---|---|---|---|---|---|
| Ex. 25 | Snowtex 40 | 0.08 | KYOWAAD 200S | 50 | 0.18 | $Na_2CO_3$ | 0.09 | - | - | 42 | MFI | 40 | 5 |
| Ex. 26 | Snowtex 40 | 0.1 | KYOWAAD 200S | 50 | 0.18 | $Na_2CO_3$ | 0.1 | - | - | 42 | MFI | 40 | 2 |
| Ex. 27 | Snowtex 40 | 0.2 | KYOWAAD 200S | 50 | 0.18 | $Na_2CO_3$ | 0.1 | - | - | 21 | MFI | 40 | 5 |
| Ex. 28 | Snowtex 40 | 0.1 | KYOWAAD 200S | 65 | 0.18 | $Na_2CO_3$ | 0.1 | - | - | 42 | MFI | 40 | 5 |
| Ex. 29 | Snowtex 40 | 0.1 | $NaAlO_2$ | 75 | 0.18 | $Na_2CO_3$ | 0.1 | - | - | 42 | MFI | 40 | 5 |
| Ex. 30 | Snowtex 40 | 0.1 | KYOWAAD 200S | 75 | 0.09 | $Na_2CO_3$ | 0.1 | - | - | 42 | MFI | 40 | 5 |
| Ex. 31 | Snowtex 40 | 0.1 | KYOWAAD 200S | 75 | 0.1 | $Na_2CO_3$ | 0.1 | - | - | 42 | MFI | 40 | 5 |
| Ex. 32 | Snowtex 40 | 0.1 | KYOWAAD 200S | 75 | 0.18 | $Na_2CO_3$ | 0.1 | - | - | 42 | - | - | 0 |
| Ex. 33 | Snowtex 40 | 0.1 | KYOWAAD 200S | 75 | 0.18 | $Na_2CO_3$ | 0.1 | - | - | 42 | MFI | 40 | 2 |
| Ex. 34 | Snowtex 40 | 0.1 | KYOWAAD 200S | 75 | 0.18 | $Na_2CO_3$ | 0.1 | - | - | 42 | MFI | 40 | 5 |
| Ex. 35 | Snowtex 40 | 0.1 | KYOWAAD 200S | 75 | 0.18 | $Na_2CO_3$ | 0.1 | - | - | 42 | MFI | 40 | 5 |
| Ex. 37 | Snowtex 40 | 0.1 | KYOWAAD 200S | 75 | 0.18 | $Na_2CO_3$ | 0.2 | - | - | 42 | MFI | 40 | 5 |
| Ex. 38 | Snowtex 40 | 0.1 | KYOWAAD 200S | 75 | 0.18 | $Na_2CO_3$ | 0.5 | - | - | 42 | MFI | 40 | 5 |
| Ex. 39 | Snowtex 40 | 0.1 | KYOWAAD 200S | 75 | 0.8 | $Na_2CO_3$ | 0.1 | - | - | 42 | MFI | 40 | 5 |

(continued)

| | Si source | Si amount | Al source | Ingredient SAR | Na amount | SDA1 | SDA1 amount | SDA2 | SDA2 amount | $H_2O$ amount | seed crystal | seed crystal SAR | seed crystal amount |
|---|---|---|---|---|---|---|---|---|---|---|---|---|---|
| Ex. 40 | Snowtex 40 | 0.1 | Aluminum sulfate | 75 | 0.18 | $Na_2CO_3$ | 0.1 | - | - | 42 | MFI | 40 | 5 |
| Ex. 41 | Snowtex 40 | 0.15 | KYOWAAD 200S | 75 | 0.18 | $Na_2CO_3$ | 0.1 | - | - | 30 | MFI | 40 | 5 |
| Ex. 42 | Snowtex 40 | 0.15 | KYOWAAD 200S | 75 | 0.18 | $Na_2CO_3$ | 0.1 | - | - | 30 | MFI | 40 | 5 |
| Ex. 43 | Snowtex 40 | 0.18 | KYOWAAD 200S | 75 | 0.18 | $Na_2CO_3$ | 0.1 | - | - | 21 | MFI | 40 | 2 |
| Ex. 44 | Snowtex 40 | 0.2 | KYOWAAD 200S | 75 | 0.18 | $Na_2CO_3$ | 0.1 | - | - | 21 | MFI | 40 | 5 |
| Ex. 45 | Snowtex 40 | 0.2 | KYOWAAD 200S | 75 | 0.18 | $Na_2CO_3$ | 0.1 | - | - | 21 | MFI | 40 | 5 |
| Ex. 46 | Snowtex 40 | 0.2 | KYOWAAD 200S | 75 | 0.18 | $Na_2CO_3$ | 0.1 | - | - | 21 | MFI | 40 | 5 |

Table 37-2-continued. Synthesis conditions of MFI-type zeolite ~continued

| | hydrothermal synthesis temperature | hydrothermal synthesis time | stirring rate | vessel | post-treatment |
|---|---|---|---|---|---|
| Ex. 25 | 175 | 72 | 0 | 0.2 | ion exchange/firing |
| Ex. 26 | 170 | 72 | 15 | 0.2 | acid treatment |
| Ex. 27 | 170 | 48 | 15 | 0.2 | acid treatment |
| Ex. 28 | 175 | 72 | 15 | 0.2 | ion exchange/firing |
| Ex. 29 | 175 | 72 | 15 | 0.2 | ion exchange/firing |
| Ex. 30 | 175 | 72 | 15 | 0.2 | ion exchange/firing |
| Ex. 31 | 175 | 72 | 15 | 0.2 | ion exchange/firing |
| Ex. 32 | 175 | 120 | 15 | 0.2 | ion exchange/firing |
| Ex. 33 | 170 | 72 | 15 | 0.2 | acid treatment |
| Ex. 34 | 175 | 72 | 15 | 0.2 | ion exchange/firing |
| Ex. 35 | 175 | 72 | 15 | 0.2 | - |
| Ex. 37 | 175 | 72 | 15 | 0.2 | ion exchange/firing |
| Ex. 38 | 175 | 72 | 15 | 0.2 | ion exchange/firing |
| Ex. 39 | 175 | 72 | 15 | 0.2 | ion exchange/firing |
| Ex. 40 | 175 | 72 | 15 | 0.2 | ion exchange/firing |
| Ex. 41 | 170 | 24 | 15 | 0.2 | acid treatment |
| Ex. 42 | 170 | 48 | 15 | 0.2 | acid treatment |
| Ex. 43 | 170 | 72 | 15 | 0.2 | acid treatment |
| Ex. 44 | 170 | 24 | 15 | 0.2 | acid treatment |
| Ex. 45 | 170 | 48 | 15 | 0.2 | acid treatment |
| Ex. 46 | 175 | 72 | 15 | 0.2 | ion exchange/firing |

Table 37-3. Synthesis conditions of MFI-type zeolite ~continued

| | Si source | Si amount | Al source | Ingredient SAR | Na amount | SDA1 | SDA1 amount | SDA2 | SDA2 amount | $H_2O$ amount | seed crystal | seed crystal SAR | seed crystal amount |
|---|---|---|---|---|---|---|---|---|---|---|---|---|---|
| Ex. 47 | Snowtex 40 | 0.7 | KYOWAAD 200S | 75 | 0.18 | $Na_2CO_3$ | 0.1 | - | - | 30 | MFI | 40 | 5 |
| Ex. 48 | Snowtex 40 | 0.7 | KYOWAAD 200S | 75 | 0.18 | $Na_2CO_3$ | 0.1 | - | - | 42 | MFI | 40 | 5 |
| Ex. 49 | Snowtex 40 | 0.7 | KYOWAAD 200S | 75 | 0.18 | $Na_2CO_3$ | 0.1 | - | - | 42 | MFI | 40 | 5 |
| Ex. 50 | Snowtex 40 | 0.7 | KYOWAAD 200S | 75 | 0.18 | $Na_2CO_3$ | 0.1 | - | - | 42 | MFI | 40 | 5 |
| Ex. 51 | Snowtex 40 | 1 | KYOWAAD 200S | 75 | 0.18 | $Na_2CO_3$ | 0.1 | - | - | 21 | MFI | 40 | 5 |
| Ex. 52 | Snowtex 40 | 1.2 | KYOWAAD 200S | 75 | 0.18 | $Na_2CO_3$ | 0.1 | - | - | 21 | MFI | 40 | 5 |
| Ex. 53 | Admafine | 0.1 | KYOWAAD 200S | 75 | 0.18 | $Na_2CO_3$ | 0.1 | - | - | 42 | MFI | 40 | 5 |
| Ex. 54 | Snowtex 40 | 0.1 | KYOWAAD 200S | 85 | 0.18 | $Na_2CO_3$ | 0.1 | - | - | 42 | MFI | 40 | 5 |
| Ex. 55 | Snowtex 40 | 0.1 | KYOWAAD 200S | 100 | 0.18 | $Na_2CO_3$ | 0.1 | - | - | 42 | MFI | 40 | 5 |
| Ex. 56 | Snowtex 40 | 0.1 | KYOWAAD 200S | 40 | 0.18 | $Na_2SO_4$ | 0.1 | - | - | 42 | MFI | 40 | 5 |
| Ex. 57 | Snowtex 40 | 0.1 | KYOWAAD 200S | 50 | 0.18 | $Na_2SO_4$ | 0.05 | - | - | 42 | MFI | 40 | 5 |
| Ex. 58 | Snowtex 40 | 0.1 | KYOWAAD 200S | 50 | 0.18 | $Na_2SO_4$ | 0.1 | - | - | 42 | MFI | 40 | 5 |
| Ex. 59 | Snowtex 40 | 0.1 | KYOWAAD 200S | 75 | 0.18 | $Na_2SO_4$ | 0.1 | - | - | 42 | MFI | 40 | 5 |
| Comp. Ex. 22 | Snowtex 40 | 0.1 | KYOWAAD 200S | 30 | 0.18 | - | - | - | - | 42 | MFI | 40 | 5 |

(continued)

| | Si source | Si amount | Al source | Ingredient SAR | Na amount | SDA1 | SDA1 amount | SDA2 | SDA2 amount | $H_2O$ amount | seed crystal | seed crystal SAR | seed crystal amount |
|---|---|---|---|---|---|---|---|---|---|---|---|---|---|
| Ex. 60 | Snowtex 40 | 0.1 | KYOWAAD 200S | 60 | 0.18 | - | - | - | - | 42 | MFI | 40 | 5 |
| Ex. 61 | Snowtex 40 | 0.1 | KYOWAAD 200S | 70 | 0.18 | - | - | - | - | 42 | MFI | 40 | 5 |
| Ex. 62 | Snowtex 40 | 0.1 | KYOWAAD 200S | 80 | 0.18 | - | - | - | - | 42 | MFI | 40 | 5 |
| Ex. 63 | Snowtex 40 | 0.1 | KYOWAAD 200S | 90 | 0.18 | - | - | - | - | 42 | MFI | 40 | 5 |
| Ex. 64 | Snowtex 40 | 0.1 | KYOWAAD 200S | 100 | 0.18 | - | - | - | - | 42 | MFI | 40 | 5 |

Table 37-3-continued. Synthesis conditions of MFI-type zeolite ~continued

| | hydrothermal synthesis temperature | hydrothermal synthesis time | stirring rate | vessel | post-treatment |
|---|---|---|---|---|---|
| Ex. 47 | 175 | 72 | 100 | 1 | ion exchange/firing |
| Ex. 48 | 175 | 72 | 100 | 1 | - |
| Ex. 49 | 175 | 72 | 100 | 1 | ion exchange/firing |
| Ex. 50 | 175 | 72 | 100 | 1 | acid treatment |
| Ex. 51 | 160 | 24 | 100 | 1 | acid treatment |
| Ex. 52 | 175 | 72 | 100 | 1 | ion exchange/firing |
| Ex. 53 | 170 | 72 | 15 | 0.2 | acid treatment |
| Ex. 54 | 175 | 72 | 15 | 0.2 | ion exchange/firing |
| Ex. 55 | 175 | 72 | 15 | 0.2 | ion exchange/firing |
| Ex. 56 | 170 | 72 | 15 | 0.2 | acid treatment |
| Ex. 57 | 170 | 72 | 15 | 0.2 | acid treatment |
| Ex. 58 | 170 | 72 | 15 | 0.2 | acid treatment |
| Ex. 59 | 170 | 72 | 15 | 0.2 | acid treatment |
| Comp. Ex. 22 | 175 | 120 | 15 | 0.2 | ion exchange/firing |
| Ex. 60 | 175 | 72 | 15 | 0.2 | ion exchange/firing |
| Ex. 61 | 175 | 72 | 15 | 0.2 | ion exchange/firing |
| Ex. 62 | 175 | 72 | 15 | 0.2 | ion exchange/firing |
| Ex. 63 | 175 | 72 | 15 | 0.2 | ion exchange/firing |
| Ex. 64 | 175 | 72 | 15 | 0.2 | ion exchange/firing |

excluding the seed crystals, added in the example. If no $Al_2O_3$ is included, it is denoted as "∞".

Na Amount: The molar ratio of Na from the Si source and NaOH to Si in the ingredient mixture, excluding the seed crystals, added in the example.
SDA1: The type of the first SDA added in the example.
SDA1 Amount: The molar ratio of SDA1 to Si in the ingredient mixture, excluding the seed crystals, added in the example.
SDA2: The type of the second SDA added in the example.
SDA2 Amount: The molar ratio of SDA2 to Si in the ingredient mixture, excluding the seed crystals, added in the example.
$H_2O$ Amount: The molar ratio of $H_2O$ to Si in the ingredient mixture, excluding the seed crystals, added in the example.
Seed Crystal: The structure of the seed crystal zeolite added in the example.
Seed Crystal SAR: The molar ratio of $SiO_2$ to $Al_2O_3$ of the seed crystal zeolite added in the example.
Seed Crystal Amount: The proportion (mass%) of the seed crystal added relative to the amount obtained by converting all Si in the ingredient mixture, excluding the seed crystals, to $SiO_2$.
Hydrothermal Synthesis Temperature: The temperature (°C) at which hydrothermal synthesis was performed in the example.
Hydrothermal Synthesis Time: The time (hours) for which hydrothermal synthesis was performed in the example.
Stirring Rate: The stirring rate (rpm) at which hydrothermal synthesis was performed in the example (0 means static condition).
Vessel: The capacity (L) of the pressure vessel in which hydrothermal synthesis was performed in the example.
Post-treatment: The post-treatment method performed on the zeolite powder obtained by hydrothermal synthesis in the example. A combination of [SDA Removal], [Ion Exchange and Calcination], or [Acid Treatment] shown below was performed in the order described in the table.

[SDA Removal]

The obtained zeolite powder was calcined at 550°C for 6 hours under air flow to remove the SDA from the powder.

[Ion Exchange and Calcination]

The zeolite powder was dispersed at 10 mass% in a 1 mol/L ammonium nitrate aqueous solution prepared using ammonium nitrate and desalinated water, and ammonium ion exchange was performed at 80°C for 2 hours, followed by suction filtration and washing with desalinated water. Thereafter, the ion exchange and washing were repeated twice more. Furthermore, after drying the ion-exchanged powder, it was calcined at 500°C for 4 hours under air flow to obtain a proton-form zeolite.

[Acid Treatment]

The obtained zeolite powder was added at 10 mass% to a 1 mol/L nitric acid aqueous solution and stirred at room temperature for 1 hour, followed by suction filtration and washing with desalinated water, and then dried to obtain a proton-form zeolite powder.

[Reagents Used in Synthesis Examples]

The reagents used in the synthesis examples of Examples 4-35, Examples 37-64, and Comparative Examples 15-22 are summarized in Table 38.

[Table 38]

Table 38

| reagent name | product name | product code | supplier |
|---|---|---|---|
| Snowtex 40 | Snowtex® 40 | - | Nissan Chemical Corporation |
| TEOS | Tetraethyl orthosilicate | T0100 | Tokyo Chemical Industry Co., Ltd. |
| Admafine | Admafine SO-C1 | - | Admatechs Co.,Ltd. |
| KYOWAAD 200S | KYOWAAD® 200S (alumina content 54.0 wt.%) | - | Kyowa Chemical Industry Co., Ltd. |
| Aluminum sulfate | Aluminum sulfate | 018-09745 | FUJIFILM Wako Pure Chemical Corporation |
| $NaAlO_2$ | Sodium aluminate | 37095-01 | KANTO CHEMICAL CO.,INC. |
| Sodium hydroxide granules | Sodium hydroxide | 000-75165 | KISHIDA CHEMICAL Co., Ltd. |
| Potassium hydroxide granules | Potassium hydroxide | 000-63665 | KISHIDA CHEMICAL Co., Ltd. |
| 1 mol/L sodium hydroxide aq. soln. | 1 mol/L (1N) Sodium hydroxide soln. | 900-01105 | KISHIDA CHEMICAL Co., Ltd.. |
| TPAOH | 40 wt.% Tetrapropylammonium hydroxide aq. soln. | 746 | SACHEM, Inc. |
| EtOH | Ethanol (99.5) | 000-28553 | KISHIDA CHEMICAL Co., Ltd. |
| BuOH | 1-Butanol | 63130-3630 | Junsei Chemical Co.,Ltd. |

(continued)

| reagent name | product name | product code | supplier |
|---|---|---|---|
| ED | Ethylenediamine Anhydrous | E0077 | Tokyo Chemical Industry Co., Ltd. |
| DiEA | 2,2'-Iminodiethanol | 099-03112 | FUJIFILM Wako Pure Chemical Corporation |
| TriEA | 2,2',2"-Nitrilotriethanol | 145-05605 | FUJIFILM Wako Pure Chemical Corporation |
| $Na_2CO_3$ | Sodium carbonate | 199-01585 | FUJIFILM Wako Pure Chemical Corporation |
| $Na_2SO_4$ | Sodium sulfate | 195-03341 | FUJIFILM Wako Pure Chemical Corporation |
| Ammonium nitrate | Ammonium nitrate | 000-03962 | KISHIDA CHEMICAL Co., Ltd. |
| 1 mol/L Nitric acid aq. soln. | 1 mol/L (1N) Nitric acid soln. | 900-00425 | KISHIDA CHEMICAL Co., Ltd. |

Acid Treatment

In Examples 65-67, acid treatment of the zeolite was performed under the reaction conditions shown below, with the nitric acid and temperature shown in Table 39.

[Reaction Conditions]

To 100 mg of the accurately weighed zeolite of Example 50, 5 mL of a 1 mol/L nitric acid aqueous solution (1 mol/L nitric acid (1N)) or concentrated nitric acid (nitric acid 1.38) from Kanto Chemical Co., Inc. was added. The reaction solution was stirred at room temperature or 100°C for 5 hours using a stirrer, and then the zeolite was collected by filtration using a Kiriyama funnel. The residue was washed with ultrapure water and dried under reduced pressure at 40°C for 2 hours.

[Table 39]

| Table 39. Acid treatment conditions (stability tests for framework) | | |
|---|---|---|
| | Nitric acid | Temperature |
| Ex. 65 | 1 mol/L nitric acid aq. soln. | room temperature |
| Ex. 66 | conc. nitric acid | room temperature |
| Ex. 67 | conc. nitric acid | 100°C |

In Example 68, acid treatment of the zeolite was performed under the reaction conditions shown below.

[Reaction Conditions]

To 1 g of the accurately weighed zeolite of Example 48, 10 g of a 1 mol/L nitric acid aqueous solution from Kishida Chemical Co., Ltd. was added. The reaction solution was stirred at room temperature for 1 hour using a stirrer, and then the zeolite was collected by filtration using a Kiriyama funnel. The residue was washed with desalinated water and dried at 100°C for 12 hours in an air atmosphere.

In Example 69, the acid treatment of the zeolite was performed in the same manner as in Example 68, except that the reaction time was changed to 2 hours.

Surface Treatment

In Comparative Example 23 and Examples 70, 72-88, surface treatment of zeolite was performed under one of the following reaction conditions using the "zeolite used" and "surface treatment reagent" shown in Table 40. The surface treatment reagents used in the reaction are listed in Table 41.

[Reaction Conditions: neat (HMDS)]

HMDS was added to the accurately weighed zeolite at a weight ratio of 10 g-HMDS/g-zeolite. This reaction solution was stirred for 5 hours under a nitrogen stream while being heated to 100°C in an oil bath. The obtained powder was collected by filtration with a Kiriyama funnel. The residue was washed with acetone, air-dried in the atmosphere for 8 hours or more, and then dried by heating at 100°C for 8 hours or more in an air atmosphere.

[Reaction Conditions: toluene-1]

Toluene was added to the accurately weighed zeolite at 20 mL/g-zeolite. The surface treatment reagent was added at 0.5 mmol/g-zeolite, stirred at room temperature for 5 hours, and the obtained powder was collected by filtration with a Kiriyama funnel. The residue was washed with ethanol and dried under reduced pressure at 40°C overnight.

[Reaction Conditions: vapor]

In a sealable container, a glass bottle containing 100 mg of the weighed zeolite, an Eppendorf tube with 2 drops of ultrapure water, and an Eppendorf tube with 2 drops of the surface treatment reagent were placed. The container was sealed and heated in an incubator at 100°C for 5 hours. Thereafter, the zeolite was dried under reduced pressure at 40°C for 2 hours.

[Reaction Conditions: neat]

The accurately weighed zeolite was placed in a constant temperature and humidity chamber and moisturized overnight under the conditions of 25°C/75%RH. The surface treatment reagent was added at 20 mL/g-zeolite, stirred at room temperature for 5 hours, and the obtained powder was collected by filtration with a Kiriyama funnel. The residue was washed with ethanol and dried under reduced pressure at 40°C for 2 hours.

[Reaction Conditions: toluene-2]

To 100 mg of the accurately weighed zeolite, 7 mL of toluene was added. Two drops of the surface treatment reagent were added, stirred at room temperature for 5 hours, and the obtained powder was collected by filtration with a Kiriyama funnel. The residue was washed with ethanol and dried under reduced pressure at 40°C for 2 hours.

[Table 40]

Table 40. Surface treatment conditions

| | zeolite used | SAR of zeolite used | surface treatment reagent | reaction condition |
|---|---|---|---|---|
| Comp. Ex. 23 | Comp. Ex. 6 | 24.0 | HMDS | neat (HMDS) |
| Ex. 70 | Ex. 34 | 59.6 | HMDS | neat (HMDS) |
| Ex. 72 | Ex. 50 | 61.7 | Chlorotrimethylsilane | toluene-1 |
| Ex. 73 | | | Dichlorohexylmethylsilane | toluene-1 |
| Ex. 74 | | | Dichloro(methyl)octadecylsilane | toluene-1 |
| Ex. 75 | | | Trichloroethylsilane | toluene-1 |
| Ex. 76 | | | Trichlorooctadecylsilane | toluene-1 |
| Ex. 77 | | | MS-51 | vapor |
| Ex. 78 | | | MS-51 | toluene-2 |
| Ex. 79 | | | MS-51 | neat |
| Ex. 80 | | | MS-56 | vapor |
| Ex. 81 | | | MS-56 | toluene-2 |
| Ex. 82 | | | MS-56 | neat |

(continued)

| Table 40. Surface treatment conditions | | | | |
|---|---|---|---|---|
| | zeolite used | SAR of zeolite used | surface treatment reagent | reaction condition |
| Ex. 83 | | | MS-57 | toluene-2 |
| Ex. 84 | | | MS-57 | neat |
| Ex. 85 | | | MS-58B15 | toluene-2 |
| Ex. 86 | | | MS-58B15 | neat |
| Ex. 87 | | | MS-58B30 | toluene-2 |
| Ex. 88 | | | MS-58B30 | neat |

[Table 41]

| Table 41. Surface treatment reagents | | |
|---|---|---|
| surface treatment reagent | product name | supplier |
| HMDS | hexamethyldisiloxane | KISHIDA CHEMICAL Co., Ltd. |
| Chlorotrimethylsilane | Chlorotrimethylsilane | Tokyo Chemical Industry Co., Ltd. |
| Dichlorohexylmethylsilane | Dichlorohexylmethylsilane | Tokyo Chemical Industry Co., Ltd. |
| Dichloro(methyl)octadecylsilane | Dichloro(methyl)octadecylsilane | Tokyo Chemical Industry Co., Ltd. |
| Trichloroethylsilane | Ethyltrichlorosilane | Tokyo Chemical Industry Co., Ltd. |
| Trichlorooctadecylsilane | Trichlorooctadecylsilane | Tokyo Chemical Industry Co., Ltd. |
| MS-51 | MKC® Silicate MS-51 | Mitsubishi Chemical Corporation |
| MS-56 | MKC® Silicate MS-56 | Mitsubishi Chemical Corporation |
| MS-57 | MKC® Silicate MS-57 | Mitsubishi Chemical Corporation |
| MS-58B15 | MKC® Silicate MS-58B15 | Mitsubishi Chemical Corporation |
| MS-58B30 | MKC® Silicate MS-58B30 | Mitsubishi Chemical Corporation |

Cation Exchange

In Examples 36, 71, and 89, the exchange of counter cations was performed under the reaction conditions shown below, using a 1 mol/L nitrate salt aqueous solution prepared from the corresponding nitrate salt in Table 42 and desalinated water.

[Reaction Conditions]

The zeolite of Example 34 was dispersed at 10 mass% in a 1 mol/L ammonium nitrate aqueous solution, and ammonium ion exchange was performed at 80°C for 2 hours, followed by suction filtration and washing with desalinated water. Thereafter, the ion exchange, filtration, and washing were repeated twice more. The obtained powder was dried to obtain an ammonium form zeolite (Example 89).

The zeolite of Example 89 was dispersed at 10 mass% in a 1 mol/L lithium nitrate or potassium nitrate aqueous solution, and cation exchange was performed at 80°C for 2 hours, followed by suction filtration and washing with desalinated water. Thereafter, the cation exchange, filtration, and washing were repeated twice more. The obtained powder was dried to obtain a lithium- or potassium-form zeolite (Examples 36, 71).

[Table 42]

| Table 42. Nitrate salts used for cation exchange | | | | |
|---|---|---|---|---|
| | nitrate salt | product name | product code | supplier |
| Ex. 89 | Ammonium nitrate | Ammonium nitrate | 000-03965 | KISHIDA CHEMICAL Co., Ltd. |

(continued)

| Table 42. Nitrate salts used for cation exchange | | | | |
|---|---|---|---|---|
| | nitrate salt | product name | product code | supplier |
| Ex. 36 | Lithium nitrate | Lithium nitrate | 129-01245 | FUJIFILM Wako Pure Chemical Corporation |
| Ex. 71 | Potassium nitrate | Potassium nitrate | 000-63835 | KISHIDA CHEMICAL Co., Ltd. |

<Measurement of Physical Properties>

Measurement Method

Analysis of Zeolite Composition

The elemental analysis of silicon, aluminum, and sodium atoms in the zeolite standard samples was conducted as follows.

The accurately weighed standard sample was heated with the addition of an alkaline salt melt, then melted and dissolved in hot water, followed by appropriate dilution with an acidic aqueous solution. The contents (weight %) of silicon, aluminum, and sodium atoms were determined using the acid concentration matching calibration curve method with ICP-AES. Furthermore, the XRF intensities of the analytical elements in the zeolite standard samples were determined, and a calibration curve was prepared using the atomic concentrations obtained from ICP-AES.

The contents (weight %) of silicon, aluminum, and sodium atoms in the zeolite samples were determined by ICP-AES or by XRF using a calibration curve with ICP-AES. For the samples with low aluminum content, the accurately weighed samples were heated with hydrofluoric acid to volatilize and remove the silicon. The residual ash was then dissolved in acid, and the aluminum content (weight %) was calculated using the acid concentration matching calibration curve method with ICP-AES. ICP-AES was performed using a ULTIMA 2C instrument (HORIBA, Ltd.). X-ray fluorescence analysis was performed using a Supermini200 instrument (Rigaku Holdings Corporation).

X-ray Powder Diffraction Measurement

Measurement and data analysis were performed under the following conditions using an X-ray powder diffractometer X'Pert Pro (PANalytical B.V.).

[Measurement Conditions]

X-ray Generator: X-ray tube (anticathode: copper, tube voltage: 45 kV, tube current: 40 mA)
Incident Optics: Focusing mirror
Light-Receiving Optics: High-speed semiconductor array detector (X-Celerator), extended light-receiving arm
Sample Stage: HTS sample stage (vibrating with 4 mm width in the X-axis direction)
Number of Scans: 5 (incident angle changed to -2, -1, 0, 1, and 2° for each)
Measurement Range: $2\theta$ = 3-40°
Scanning Speed: 0.668451°/sec
Step: 0.0167°

[Analysis Method]

Software: High Score Plus
Peak Search
Minimum Significance: 1.00
Minimum Peak Tip (°$2\theta$): 0.01
Maximum Peak Tip (°$2\theta$): 1.00
Peak Base Width ('$2\theta$): 2.00
Profile fit: Default Profile Fit was performed 3 times.

Measurement of BET Specific Surface Area and External Surface Area

(Pre-treatment)

Before measuring the surface area, a measurement cell was filled with 30-80 mg of zeolite powder and degassed by heating under reduced pressure at 350°C for 5 h using MasterPrepTM (Quantachrome Instruments).

(Measurement of Adsorption Amount)

For the pre-treated zeolite powder, an adsorption isotherm was measured. Nitrogen adsorption studies were performed using We prepared a t-plot (standard isotherm: de-Bore equation) and calculated the external surface area from the slope and intercept of the plateau. The measurement was performed using a physisorption analyzer (Autosorb iQ-XR-XR-XR or Autosorb iQ3-MP-XR, Quantachrome) at 77 K with $N_2$ adsorbate in the relative pressure range of $1\times10^{-5}$ to 0.995 . A BET plot was prepared from the obtained adsorption isotherm, and a multi-point BET analysis was performed in a range with good linearity to determine the BET specific surface area. Furthermore, a t-plot (standard isotherm: de-Bore equation) was prepared, and the external surface area was calculated from the slope and intercept of the plateau.

SEM

Platinum was deposited on the surface of the zeolite sample using an ion coater IB-3 (EIKO ENGINEERING) with a thickness of approximately 9 nm. SEM images were recorded using a scanning electron microscope S-4500 (Hitachi High-Tech Corporation) or JSM-6010LV (JEOL Ltd).

Particle Size Distribution

The particle size distribution was measured using a laser diffraction/scattering-type particle size analyzer LA-950V2 (HORIBA, Ltd.), Ltd. The measurements were performed in a flow cell using desalinated water as the dispersion medium, the refractive index of the sample set to 1.450-0.000i, the refractive index of the dispersion medium to 1.333-0.000i, the circulation speed range to 5, and the stirring speed range to 2.

## Claims

1. An oral composition comprising;

   an inorganic porous material,
   wherein the oral composition has an adsorption ratio of primary, secondary, or tertiary amine or ammonia of 60.0% or more, and has an adsorption ratio of choline of 40.0% or less, when each adsorption ratio is measured by the following method:
   to simulated intestinal fluid in which choline and the primary, secondary, or tertiary amine or ammonia are present as an equimolar mixture, the inorganic porous material is added at a concentration of 2 g/L in an amount of 11.8 g of the inorganic porous material per 1 mmol of choline, and after mixing them for 1 hour, each the adsorption ratio is measured.

2. The oral composition according to claim 1, wherein the adsorption ratio of the primary, secondary, or tertiary amine or ammonia to the inorganic porous material is 2.00 times or more than the adsorption ratio of choline.

3. The oral composition according to claim 1, wherein the inorganic porous material is an aluminosilicate or a silicate.

4. The oral composition according to claim 3, wherein the aluminosilicate is a zeolite.

5. The oral composition according to claim 4, wherein the zeolite has a maximum ring number of 10 and a largest free sphere diameter of 3.68 Å or more.

6. The oral composition according to claim 4, wherein the zeolite has an MFI structure or a FER structure.

7. The oral composition according to claim 4, wherein the zeolite has a molar ratio of $SiO_2$ to $Al_2O_3$ of 15.0 or more and 130,000.0 or less.

8. The oral composition according to claim 7, wherein the zeolite has a molar ratio of $SiO_2$ to $Al_2O_3$ of 30.0 or more and 80.0 or less.

9. The oral composition according to claim 4, wherein the external surface area of the zeolite is 33.0 $m^2/g$ or less.

10. The oral composition according to claim 1, wherein the oral composition is a pharmaceutical composition.

11. The oral composition according to claim 10, for use in the prevention, treatment, or alleviation of symptoms of a disease caused by a primary, secondary, or tertiary amine, ammonia, or a metabolite thereof.

12. The oral composition according to claim 11, wherein the primary, secondary, or tertiary amine, ammonia, or the metabolite comprises one or more selected from the group consisting of trimethylamine, dimethylamine, methylamine, histamine, ammonia, and trimethylamine-N-oxide.

13. The oral composition according to claim 11, wherein the primary, secondary, or tertiary amine, ammonia, or the metabolite is trimethylamine or trimethylamine-N-oxide.

14. The oral composition according to claim 11, wherein the disease caused by a primary, secondary, or tertiary amine, ammonia, or the metabolite is selected from the group consisting of trimethylaminuria, cardiovascular disease, glaucoma, atherosclerosis, coronary artery heart disease, heart failure with preserved ejection fraction, ST-elevation myocardial infarction, atrial fibrillation, abdominal aortic aneurysm, ischemic stroke, post-stroke cognitive impairment, mild cognitive impairment, Alzheimer's disease, obesity, progressive chronic kidney disease with type 2 diabetes, cardiovascular complications in chronic kidney disease, diabetic retinopathy, non-alcoholic steatohepatitis, polycystic ovary syndrome, Parkinson's disease, colorectal cancer, irritable bowel syndrome, hyperammonemia, urea cycle disorder, organic acidemia, hepatic encephalopathy, and portosystemic shunt.

15. The oral composition according to claim 14, wherein the disease is caused by trimethylamine, and the disease is trimethylaminuria, cardiovascular disease, or glaucoma.

16. The oral composition according to claim 14, wherein the disease is caused by trimethylamine-N-oxide, and the disease is atherosclerosis, coronary artery heart disease, heart failure with preserved ejection fraction, ST-elevation myocardial infarction, atrial fibrillation, abdominal aortic aneurysm, ischemic stroke, post-stroke cognitive impairment, mild cognitive impairment, Alzheimer's disease, obesity, progressive chronic kidney disease with type 2 diabetes, cardiovascular complications in chronic kidney disease, diabetic retinopathy, non-alcoholic steatohepatitis, polycystic ovary syndrome, Parkinson's disease, or colorectal cancer.

17. The oral composition according to claim 14, wherein the disease is caused by histamine, and the disease is irritable bowel syndrome.

18. The oral composition according to claim 14, wherein the disease is caused by ammonia, and the disease is hyperammonemia, urea cycle disorder, organic acidemia, hepatic encephalopathy, or portosystemic shunt.

19. The oral composition according to claim 10, wherein the oral composition comprises zeolite as an active ingredient, is administered 1 to 5 times per day, and the dose of the zeolite is 200 mg to 42,000 mg per administration.

20. The oral composition according to claim 19, wherein the dose of the zeolite is 500 mg to 2000 mg per administration.

21. A method for producing the oral composition according to claim 4, comprising obtaining the zeolite by hydrothermal synthesis without using an organic structure-directing agent.

22. The method according to claim 21, wherein sodium carbonate and/or sodium sulfate is used as a reagent.

23. The method according to claim 21, comprising converting the zeolite to a proton form with an acid.

24. The method according to claim 23, wherein the acid is sulfuric acid or nitric acid.

25. The method according to claim 21, further comprising performing a surface treatment on the zeolite after the hydrothermal synthesis in a solution containing a Si element source.

**26.** An oral composition comprising;

a zeolite,
wherein the zeolite has an MFI structure and a molar ratio of $SiO_2$ to $Al_2O_3$ of 30.0 or more and 130,000.0 or less, or
the zeolite has a FER structure and a molar ratio of $SiO_2$ to $Al_2O_3$ of 15.0 or more and 130,000.0 or less,
for use in the treatment, prevention, or symptom alleviation of a disease selected from the group consisting of trimethylaminuria, atherosclerosis, irritable bowel syndrome, and hyperammonemia.

**27.** The oral composition according to claim 26, wherein the zeolite has a molar ratio of $SiO_2$ to $Al_2O_3$ of 30.0 or more and 80.0 or less.

**28.** The oral composition according to claim 26, wherein the external surface area of the zeolite is less than 33.0 $m^2/g$.

**29.** The oral composition according to claim 26, wherein the oral composition is a pharmaceutical composition.

**30.** The oral composition according to claim 29, wherein the oral composition is administered 1 to 5 times per day, and the dose of the zeolite is 200 mg to 42,000 mg per administration.

**31.** The oral composition according to claim 30, wherein the dose of the zeolite is 500 mg to 2000 mg per administration.

**32.** A method for producing the oral composition according to claim 26, comprising obtaining the zeolite by hydrothermal synthesis without using an organic structure-directing agent.

**33.** The method according to claim 32, wherein sodium carbonate and/or sodium sulfate is used as a reagent.

**34.** The method according to claim 32, comprising converting the zeolite to a proton form with an acid.

**35.** The method according to claim 34, wherein the acid is sulfuric acid or nitric acid.

**36.** The method according to claim 32, further comprising performing a surface treatment on the zeolite after the hydrothermal synthesis in a solution containing a Si element source.

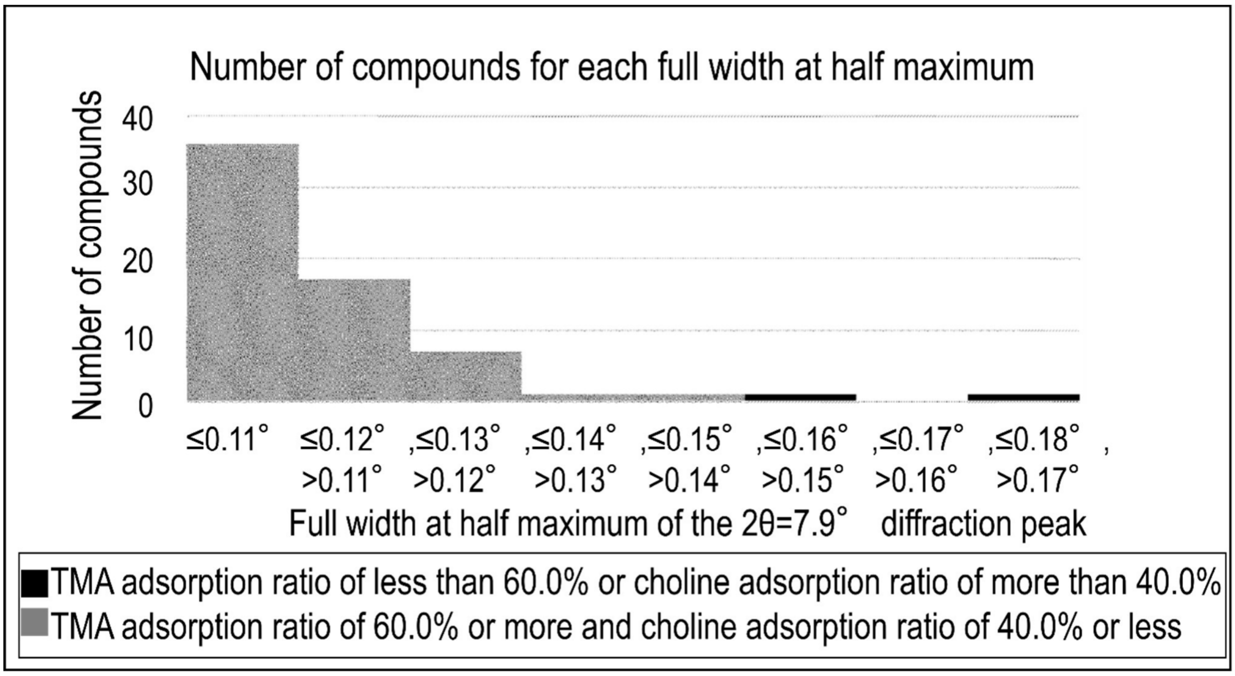
Number of compounds for each full width at half maximum
Number of compounds
40
30
20
10
0
≤0.11°
≤0.12° >0.11°
,≤0.13° >0.12°
,≤0.14° >0.13°
,≤0.15° >0.14°
,≤0.16° >0.15°
,≤0.17° >0.16°
,≤0.18° >0.17°
,
Full width at half maximum of the 2θ=7.9° diffraction peak
TMA adsorption ratio of less than 60.0% or choline adsorption ratio of more than 40.0%
TMA adsorption ratio of 60.0% or more and choline adsorption ratio of 40.0% or less

Fig. 1

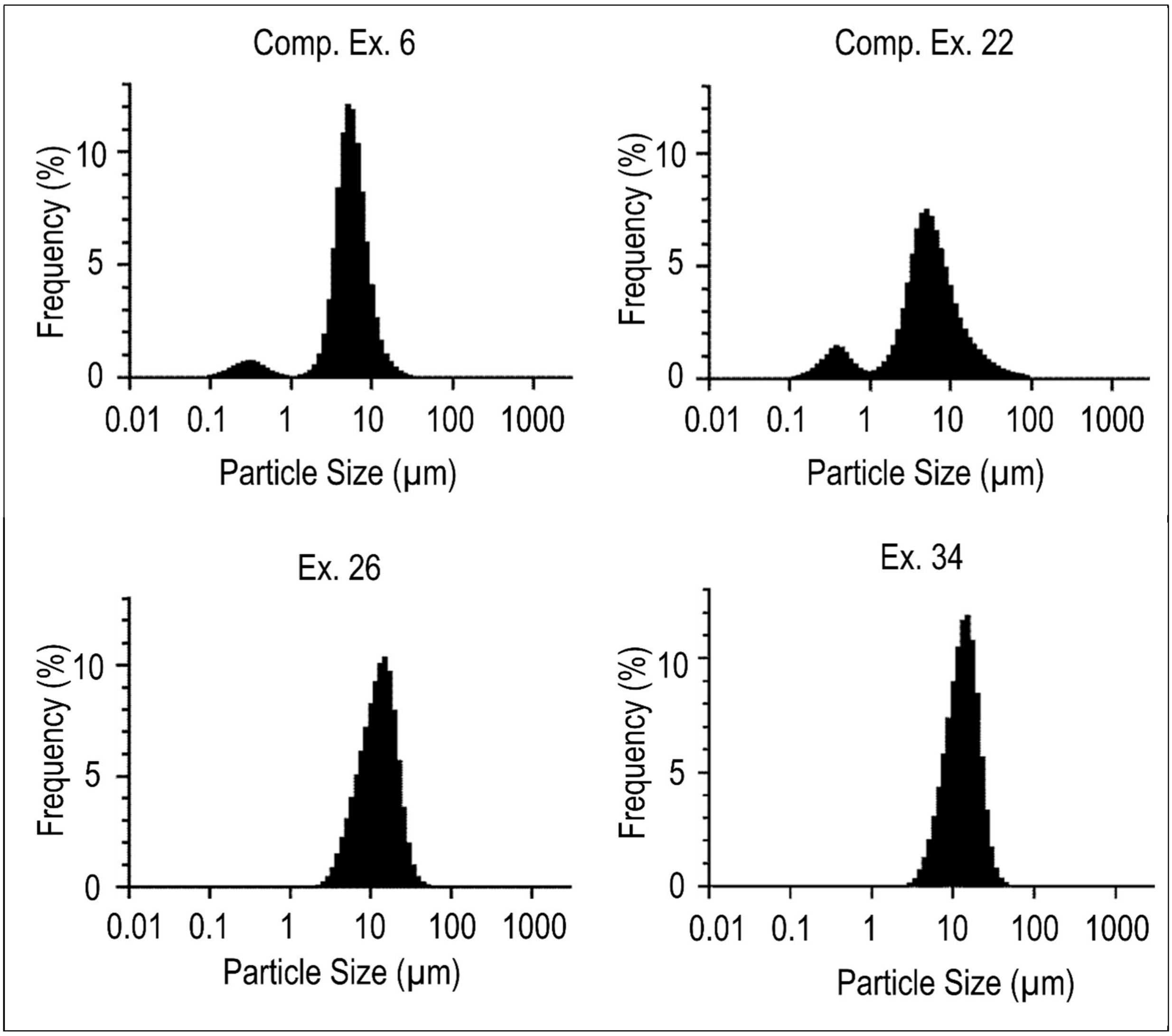
Comp. Ex. 6
Frequency (%)
0
5
10
0.01 0.1 1 10 100 1000
Particle Size (µm)
Comp. Ex. 22
Frequency (%)
0
5
10
0.01 0.1 1 10 100 1000
Particle Size (µm)
Ex. 26
Frequency (%)
0
5
10
0.01 0.1 1 10 100 1000
Particle Size (µm)
Ex. 34
Frequency (%)
0
5
10
0.01 0.1 1 10 100 1000
Particle Size (µm)

Fig. 2

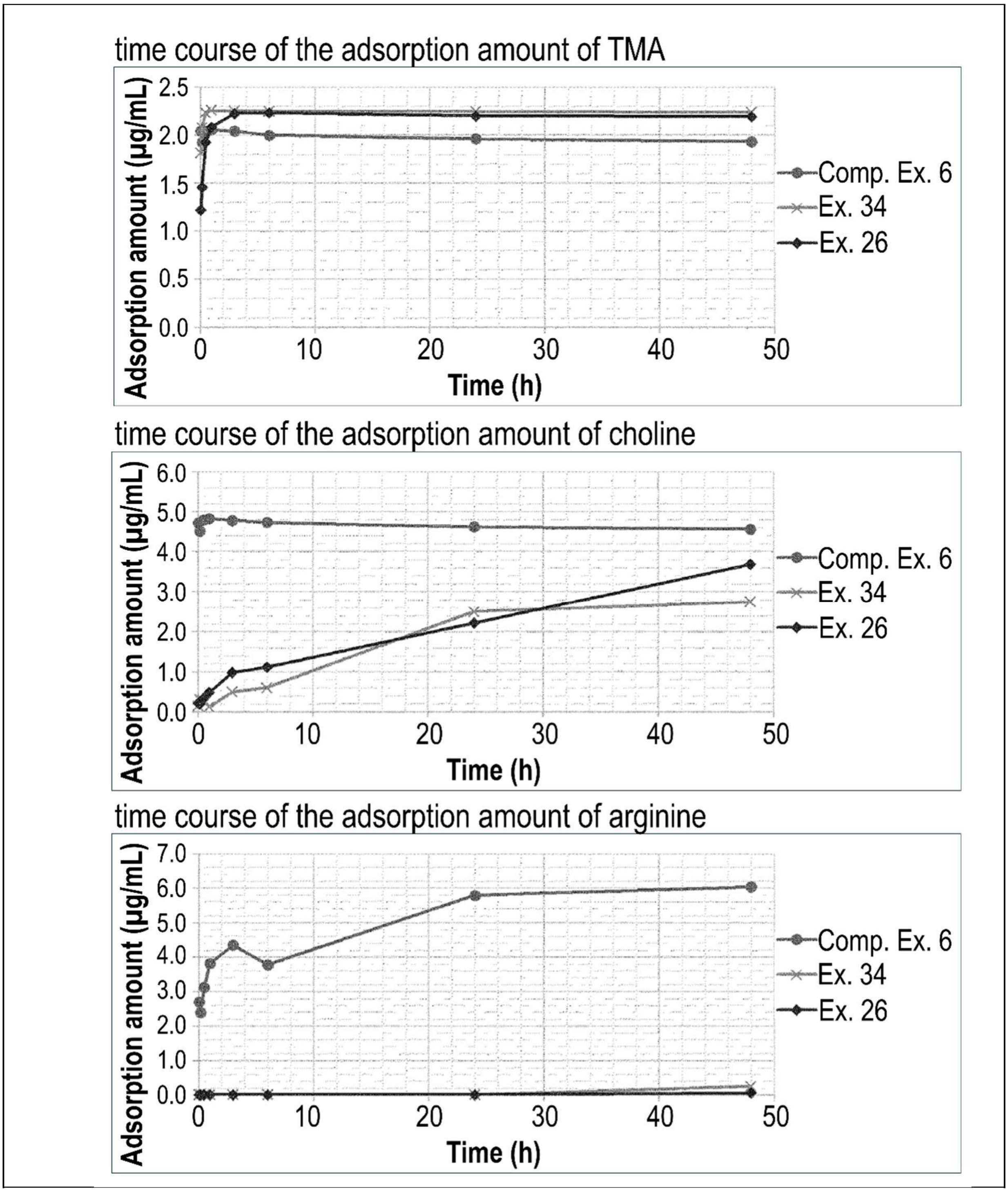
time course of the adsorption amount of TMA
Adsorption amount (µg/mL)
2.5
2.0
1.5
1.0
0.5
0.0
0
10
20
30
40
50
Time (h)
Comp. Ex. 6
Ex. 34
Ex. 26
time course of the adsorption amount of choline
Adsorption amount (µg/mL)
6.0
5.0
4.0
3.0
2.0
1.0
0.0
0
10
20
30
40
50
Time (h)
Comp. Ex. 6
Ex. 34
Ex. 26
time course of the adsorption amount of arginine
Adsorption amount (µg/mL)
7.0
6.0
5.0
4.0
3.0
2.0
1.0
0.0
0
10
20
30
40
50
Time (h)
Comp. Ex. 6
Ex. 34
Ex. 26

Fig. 3

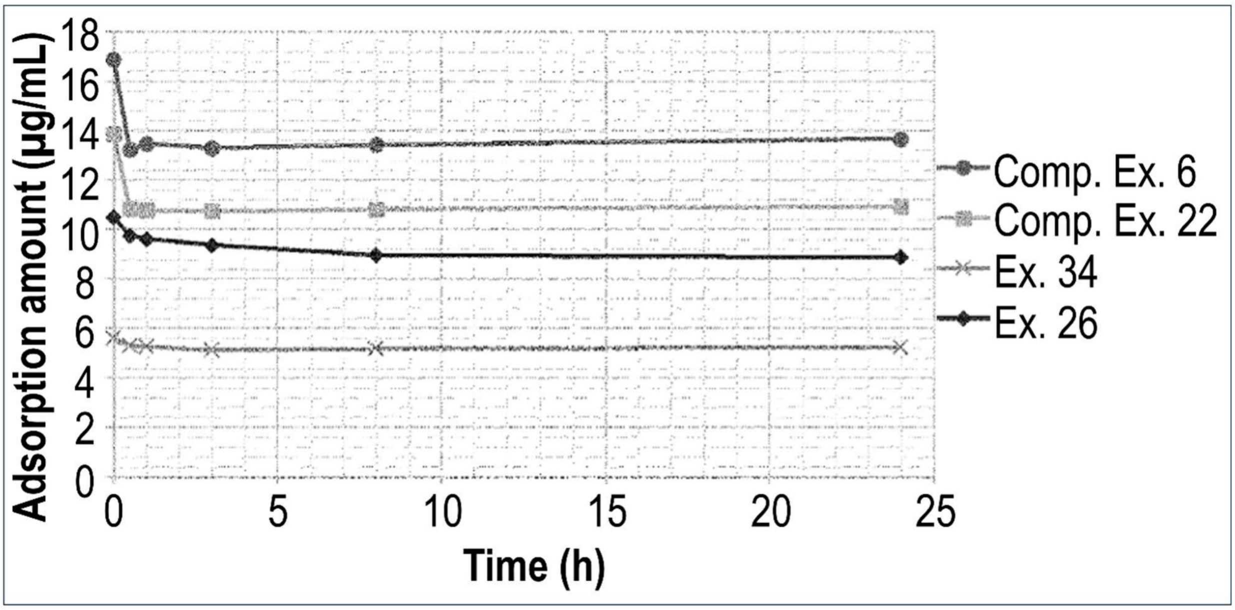
Adsorption amount (µg/mL)
18
16
14
12
10
8
6
4
2
0
0
5
10
15
20
25
Time (h)
Comp. Ex. 6
Comp. Ex. 22
Ex. 34
Ex. 26

Fig. 4

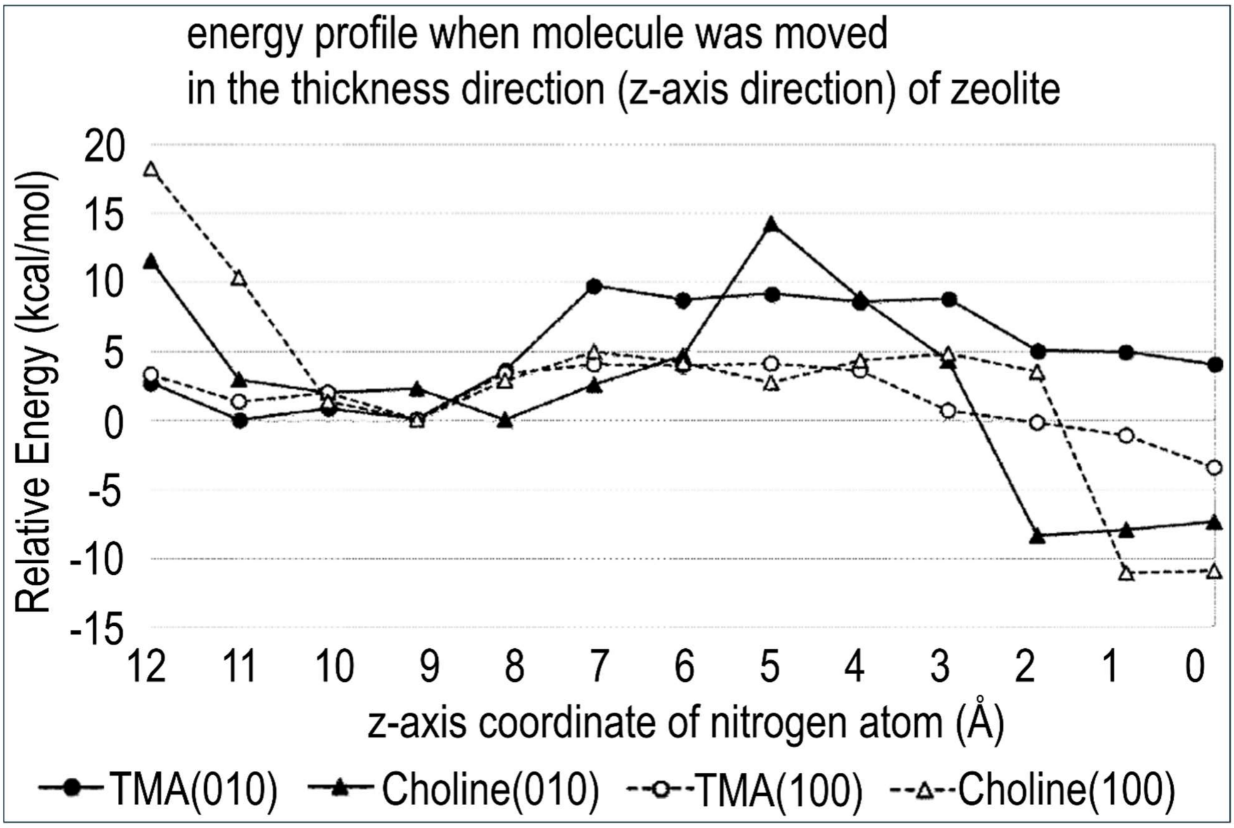
energy profile when molecule was moved
in the thickness direction (z-axis direction) of zeolite
Relative Energy (kcal/mol)
20
15
10
5
0
-5
-10
-15
12
11
10
9
8
7
6
5
4
3
2
1
0
z-axis coordinate of nitrogen atom (Å)
TMA(010)
Choline(010)
TMA(100)
Choline(100)

Fig. 5

TMA/(010) surface model (energy reference point, z=11Å)

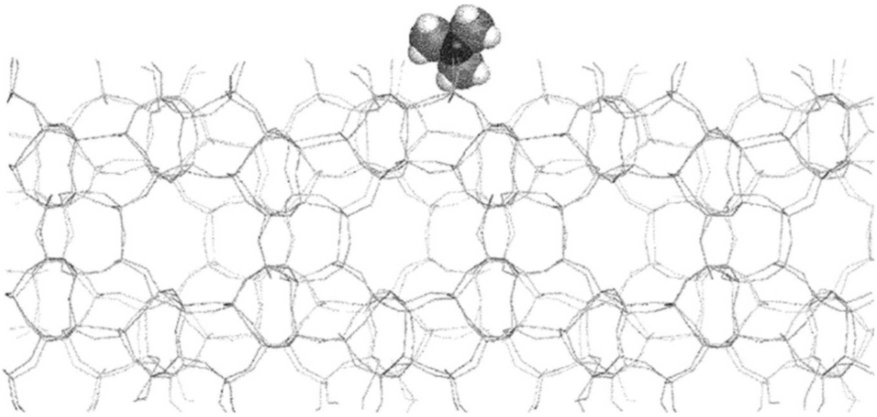

TMA/(010) surface model (the highest point of the energy barrier, z=7Å)

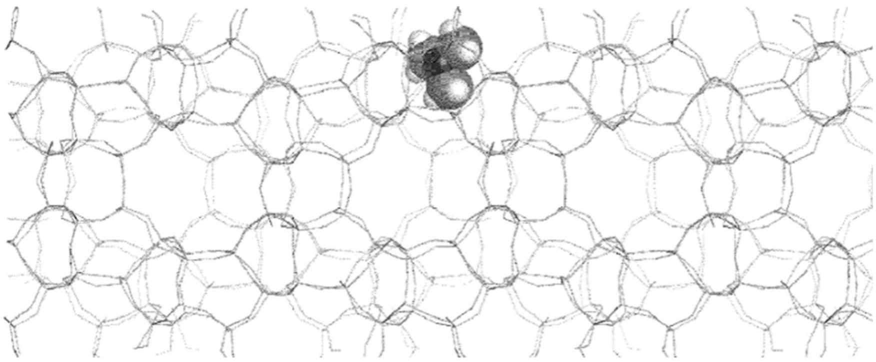

TMA/(010) surface model (internal adsorption, z=0Å)

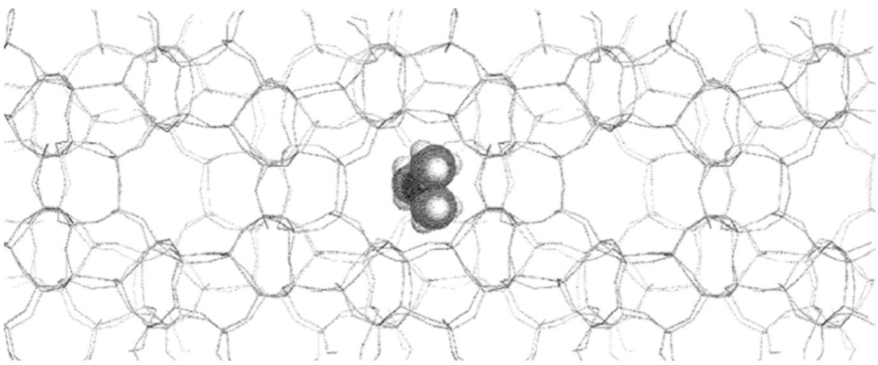

Fig. 6-1

choline/(010) surface model (energy reference point, z=8Å)

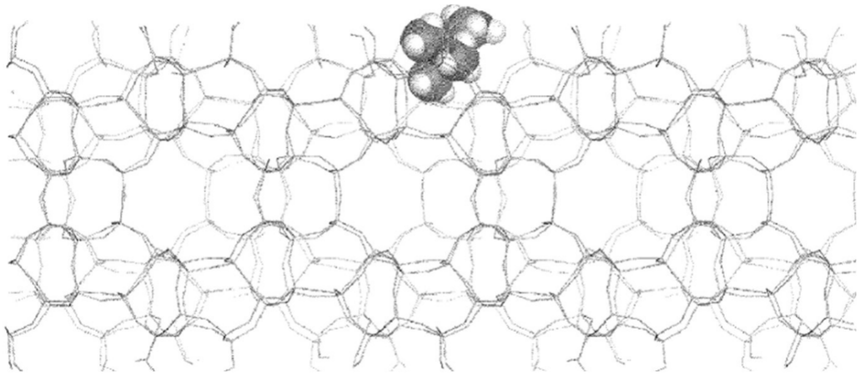

choline/(010) surface model (the highest point of the energy barrier, z=5Å)

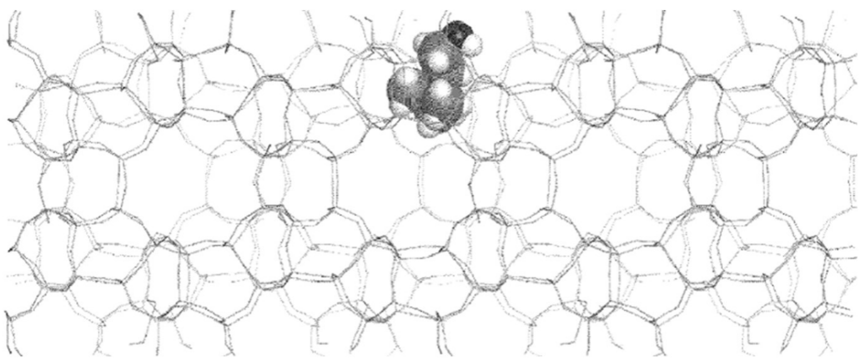

choline/(010) surface model (internal adsorption, z=0Å)

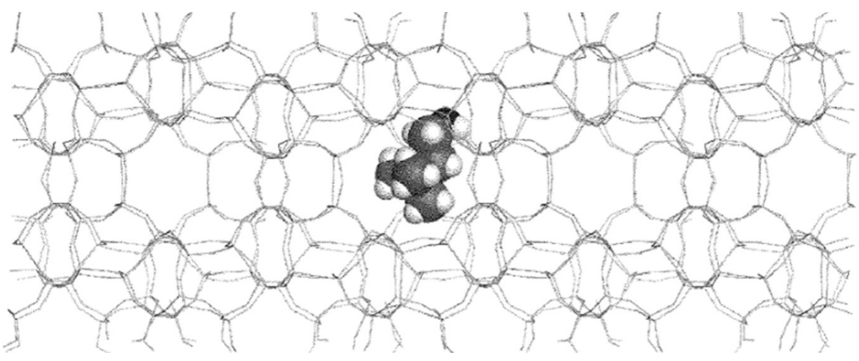

Fig. 6-2

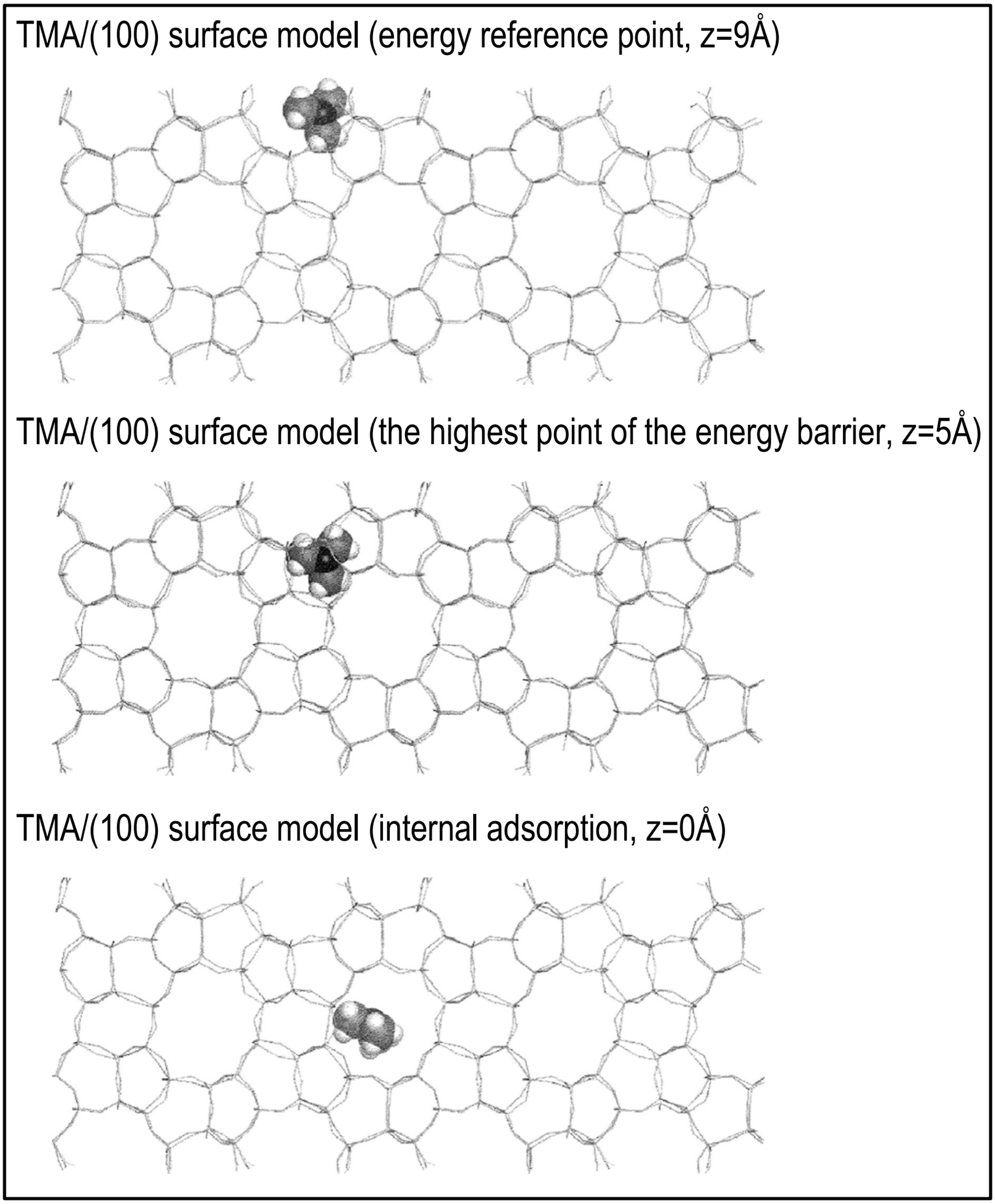
TMA/(100) surface model (energy reference point, z=9Å)
TMA/(100) surface model (the highest point of the energy barrier, z=5Å)
TMA/(100) surface model (internal adsorption, z=0Å)

Fig. 6-3

choline/(100) surface model (energy reference point, z=9Å)

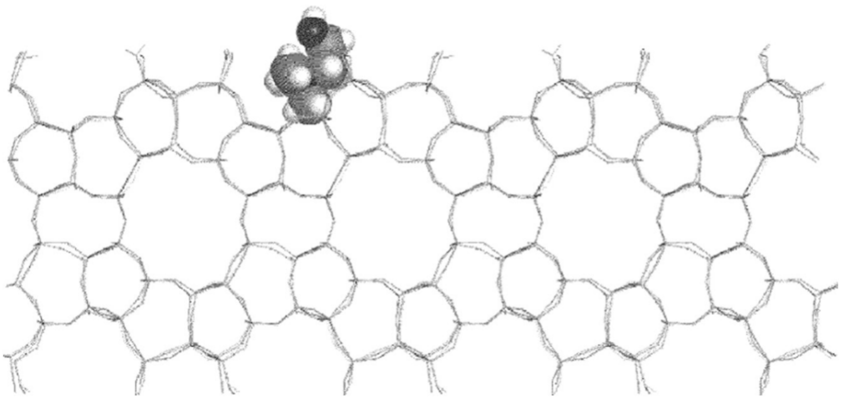

choline/(100) surface model (the highest point of the energy barrier, z=7Å)

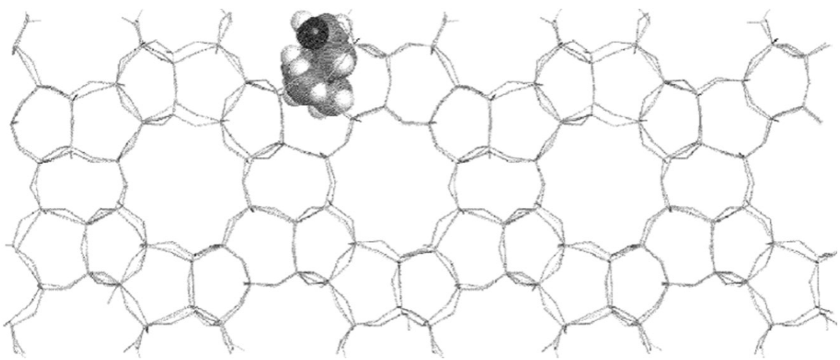

choline/(100) surface model (internal adsorption, z=0Å)

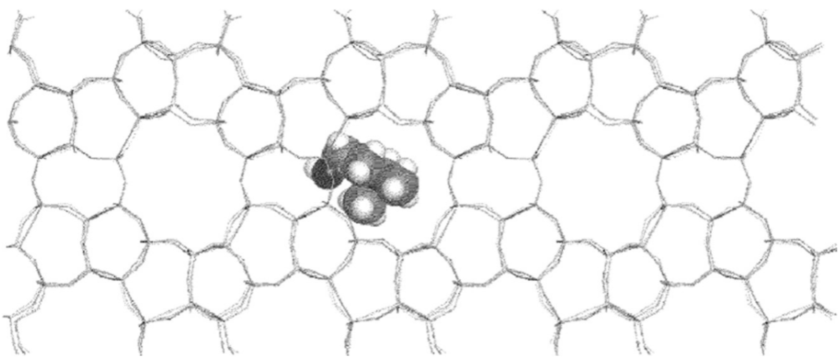

Fig. 6-4

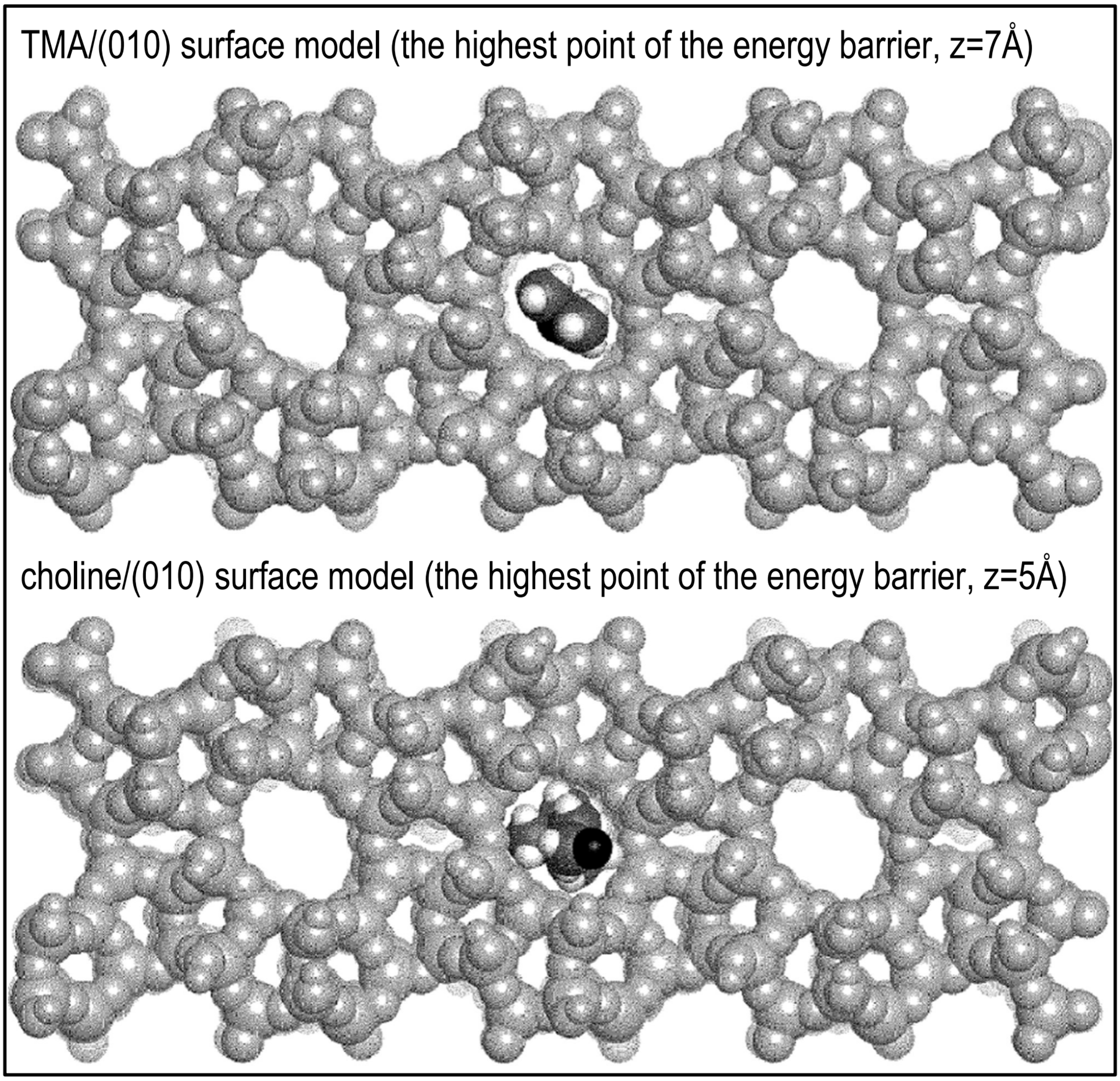
TMA/(010) surface model (the highest point of the energy barrier, z=7Å)
choline/(010) surface model (the highest point of the energy barrier, z=5Å)

Fig. 7

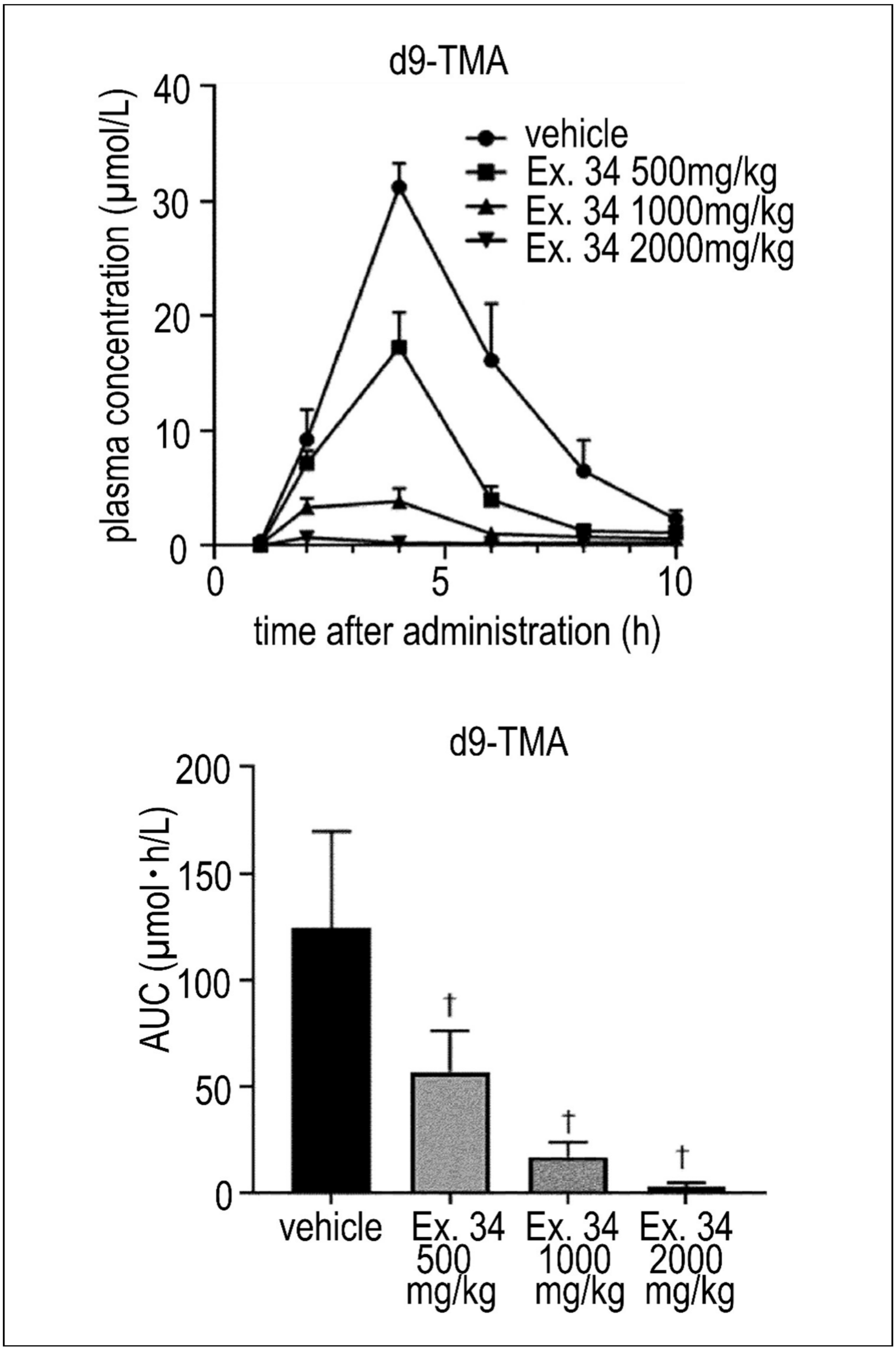
d9-TMA
plasma concentration (μmol/L)
40
30
20
10
0
vehicle
Ex. 34 500mg/kg
Ex. 34 1000mg/kg
Ex. 34 2000mg/kg
0
5
10
time after administration (h)
d9-TMA
AUC (μmol•h/L)
200
150
100
50
0
†
†
†
vehicle
Ex. 34 500 mg/kg
Ex. 34 1000 mg/kg
Ex. 34 2000 mg/kg

Fig. 8

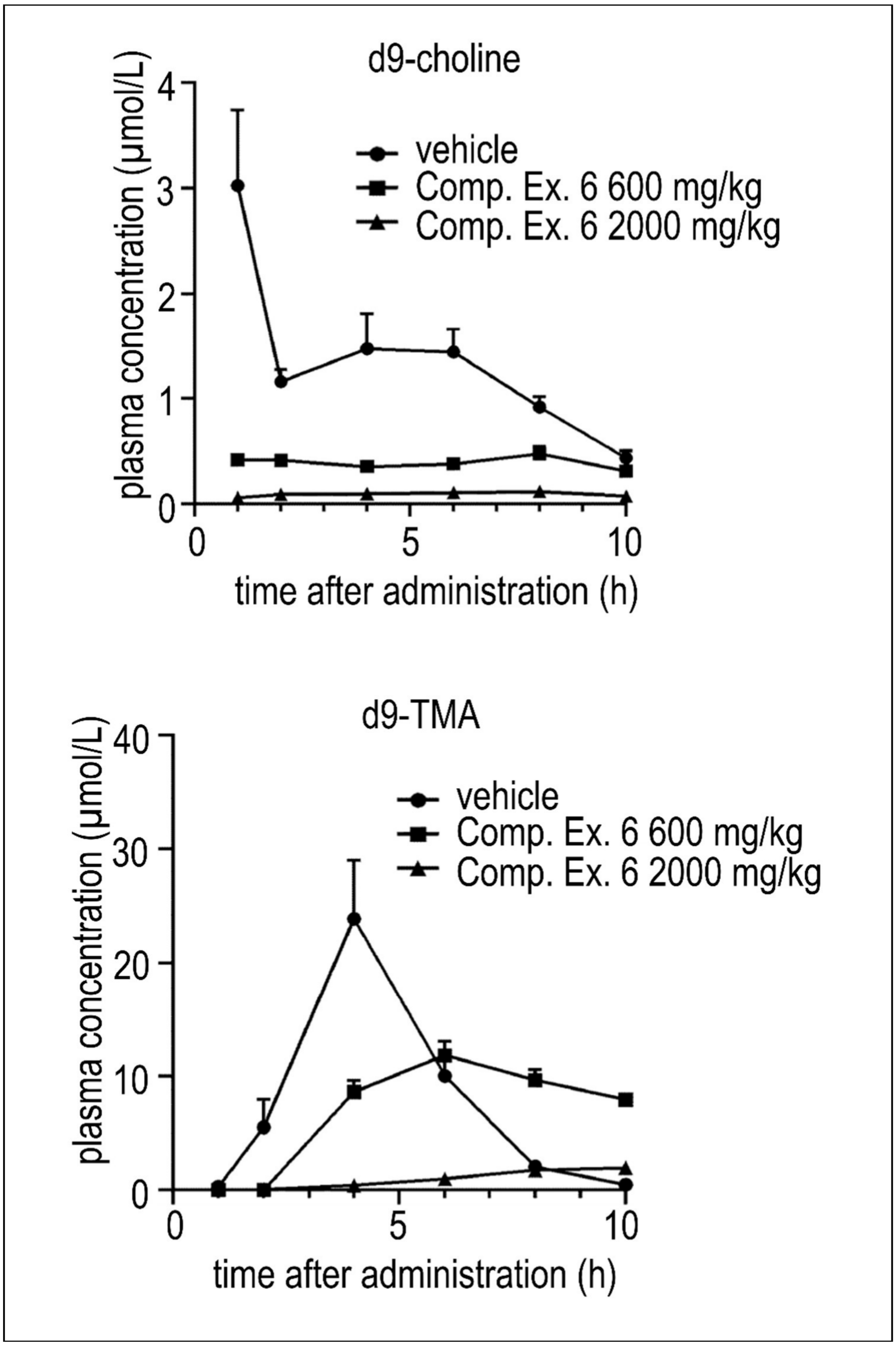
d9-choline
plasma concentration (µmol/L)
0
1
2
3
4
0
5
10
time after administration (h)
vehicle
Comp. Ex. 6 600 mg/kg
Comp. Ex. 6 2000 mg/kg
d9-TMA
plasma concentration (µmol/L)
0
10
20
30
40
0
5
10
time after administration (h)
vehicle
Comp. Ex. 6 600 mg/kg
Comp. Ex. 6 2000 mg/kg

Fig. 9

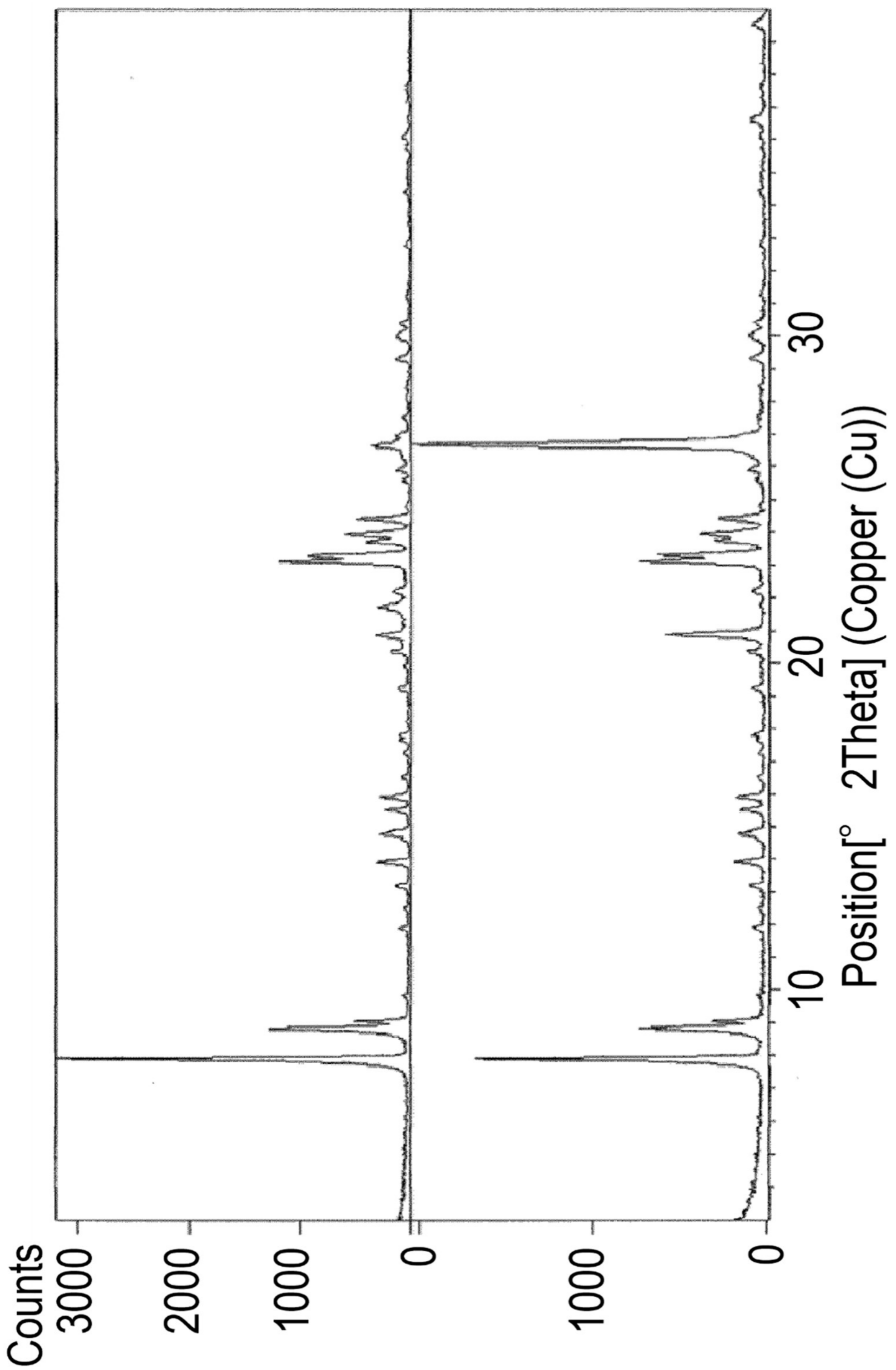
Counts
3000
2000
1000
0
1000
0
10
20
30
Position[° 2Theta] (Copper (Cu))

Fig. 10

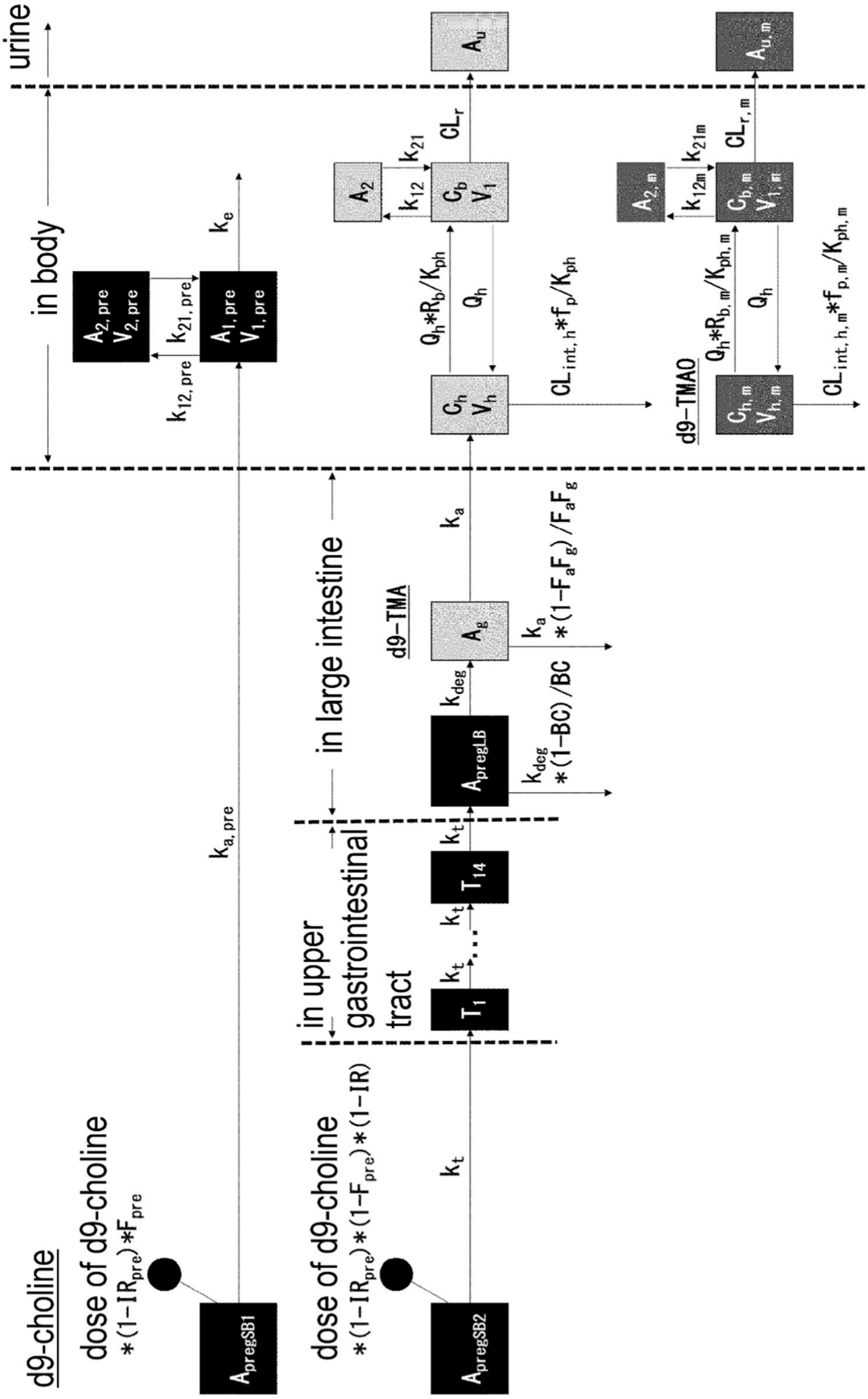

d9-choline
dose of d9-choline
*(1-IRpre)*Fpre
ApregSB1
ka, pre
dose of d9-choline
*(1-IRpre)*(1-Fpre)*(1-IR)
ApregSB2
kt
in upper gastrointestinal tract
T1
kt
kt
T14
kt
in large intestine
ApregLB
kdeg
*(1-BC)/BC
kdeg
d9-TMA
Ag
ka
*(1-FaFg)/FaFg
ka
in body
A2, pre
V2, pre
k12, pre
k21, pre
A1, pre
V1, pre
ke
Ch
Vh
Qh*Rb/Kph
Qh
CLint, h*fp/Kph
A2
k12
k21
Cb
V1
CLr
Au
d9-TMAO
Ch, m
Vh, m
Qh*Rb, m/Kph, m
Qh
CLint, h, m*fp, m/Kph, m
A2, m
k12m
k21m
Cb, m
V1, m
CLr, m
Au, m
urine


Fig. 11

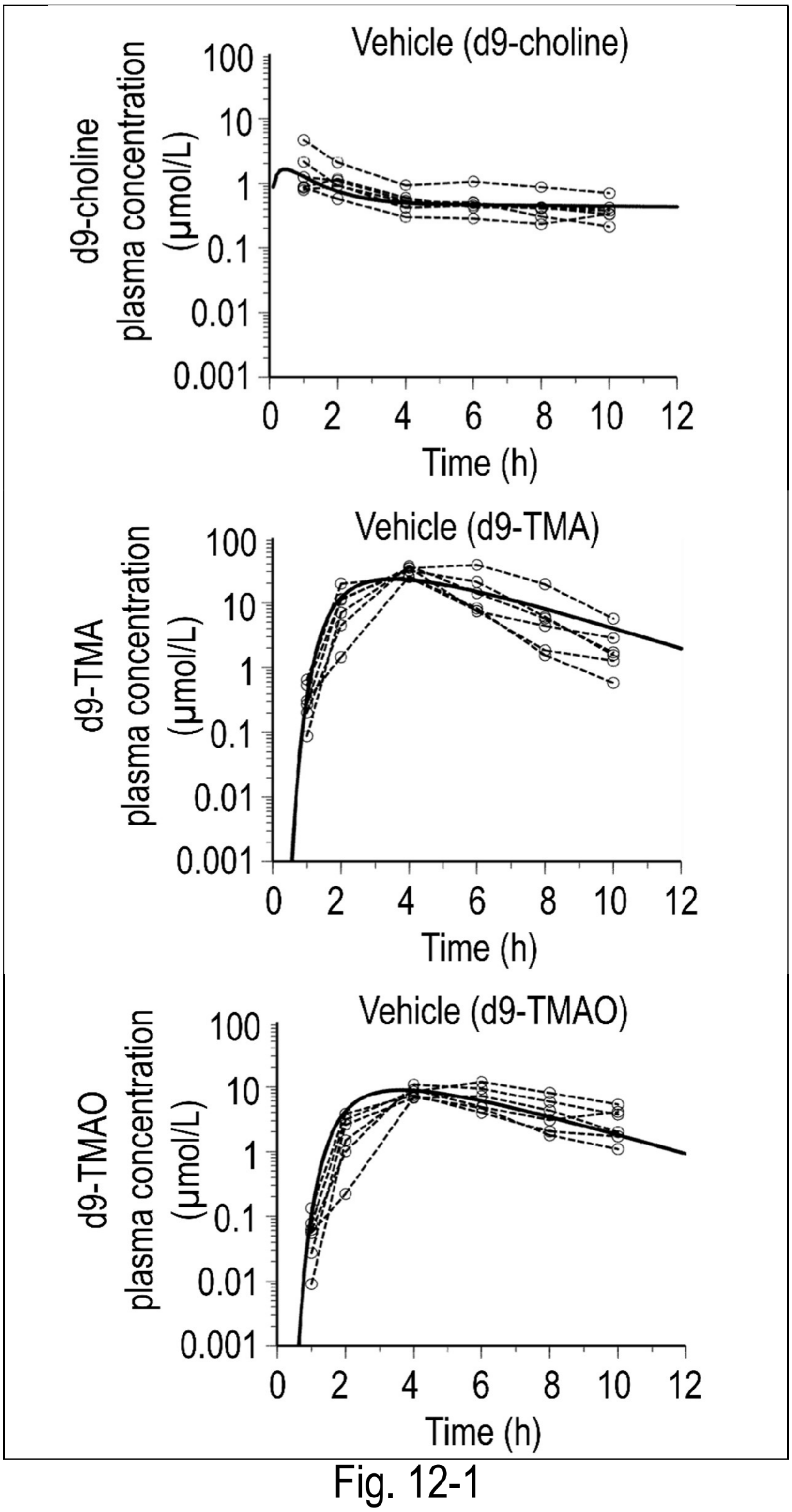
Vehicle (d9-choline)
d9-choline plasma concentration (μmol/L)
100
10
1
0.1
0.01
0.001
0 2 4 6 8 10 12
Time (h)
Vehicle (d9-TMA)
d9-TMA plasma concentration (μmol/L)
100
10
1
0.1
0.01
0.001
0 2 4 6 8 10 12
Time (h)
Vehicle (d9-TMAO)
d9-TMAO plasma concentration (μmol/L)
100
10
1
0.1
0.01
0.001
0 2 4 6 8 10 12
Time (h)

Fig. 12-1

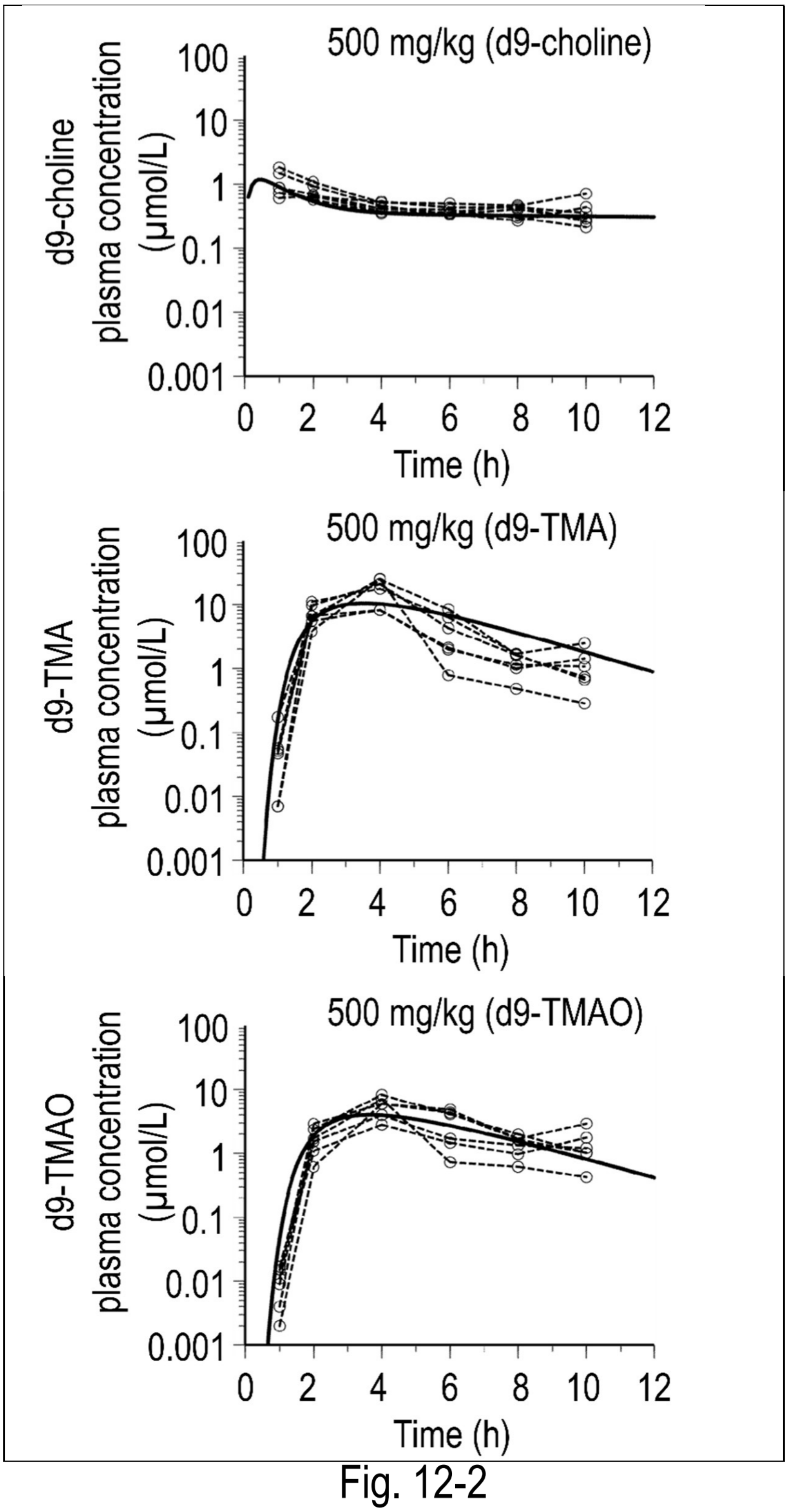
500 mg/kg (d9-choline)
d9-choline plasma concentration (µmol/L)
100
10
1
0.1
0.01
0.001
0
2
4
6
8
10
12
Time (h)
500 mg/kg (d9-TMA)
d9-TMA plasma concentration (µmol/L)
500 mg/kg (d9-TMAO)
d9-TMAO plasma concentration (µmol/L)

Fig. 12-2

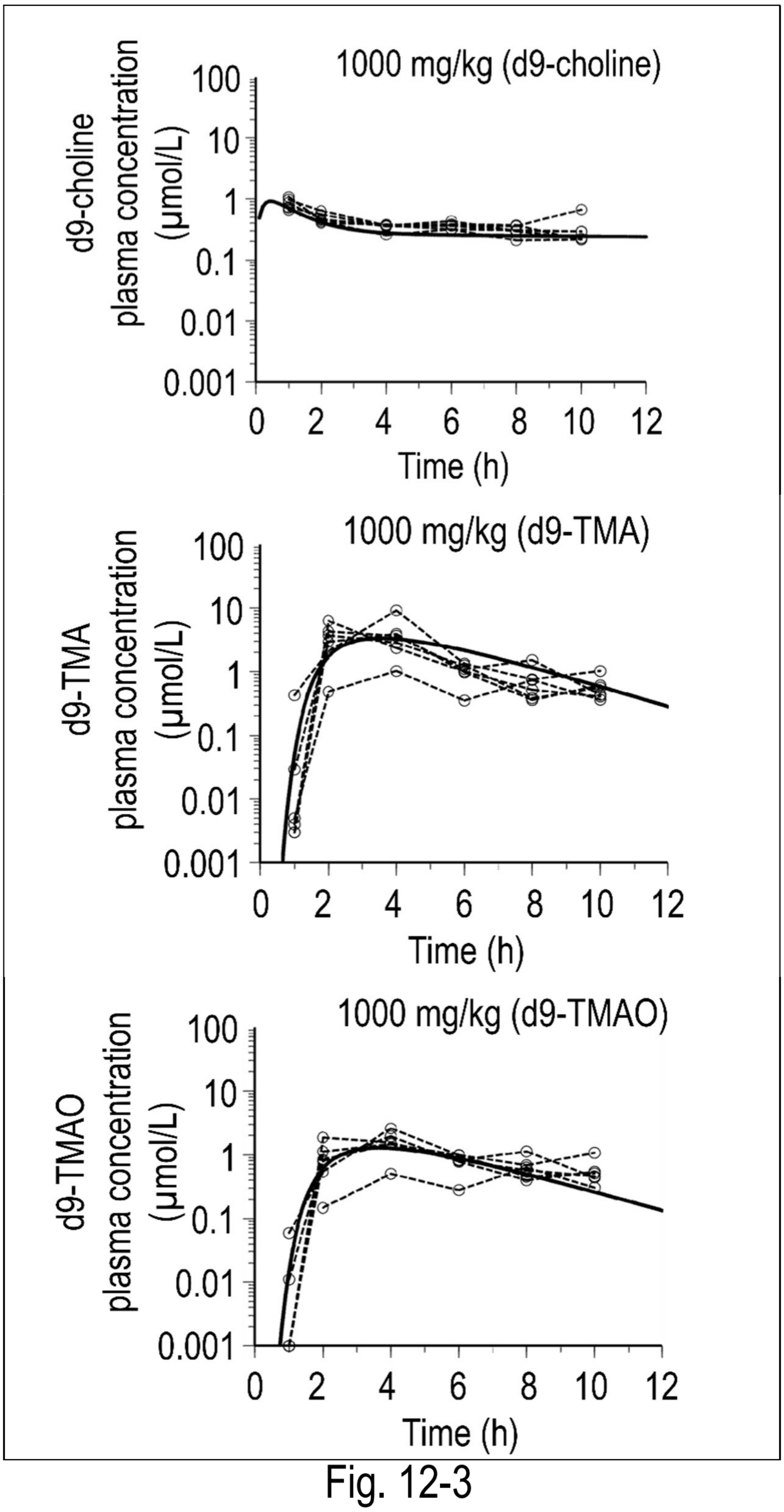
1000 mg/kg (d9-choline)
d9-choline
plasma concentration
(μmol/L)
100
10
1
0.1
0.01
0.001
0
2
4
6
8
10
12
Time (h)
1000 mg/kg (d9-TMA)
d9-TMA
plasma concentration
(μmol/L)
100
10
1
0.1
0.01
0.001
0
2
4
6
8
10
12
Time (h)
1000 mg/kg (d9-TMAO)
d9-TMAO
plasma concentration
(μmol/L)
100
10
1
0.1
0.01
0.001
0
2
4
6
8
10
12
Time (h)

Fig. 12-3

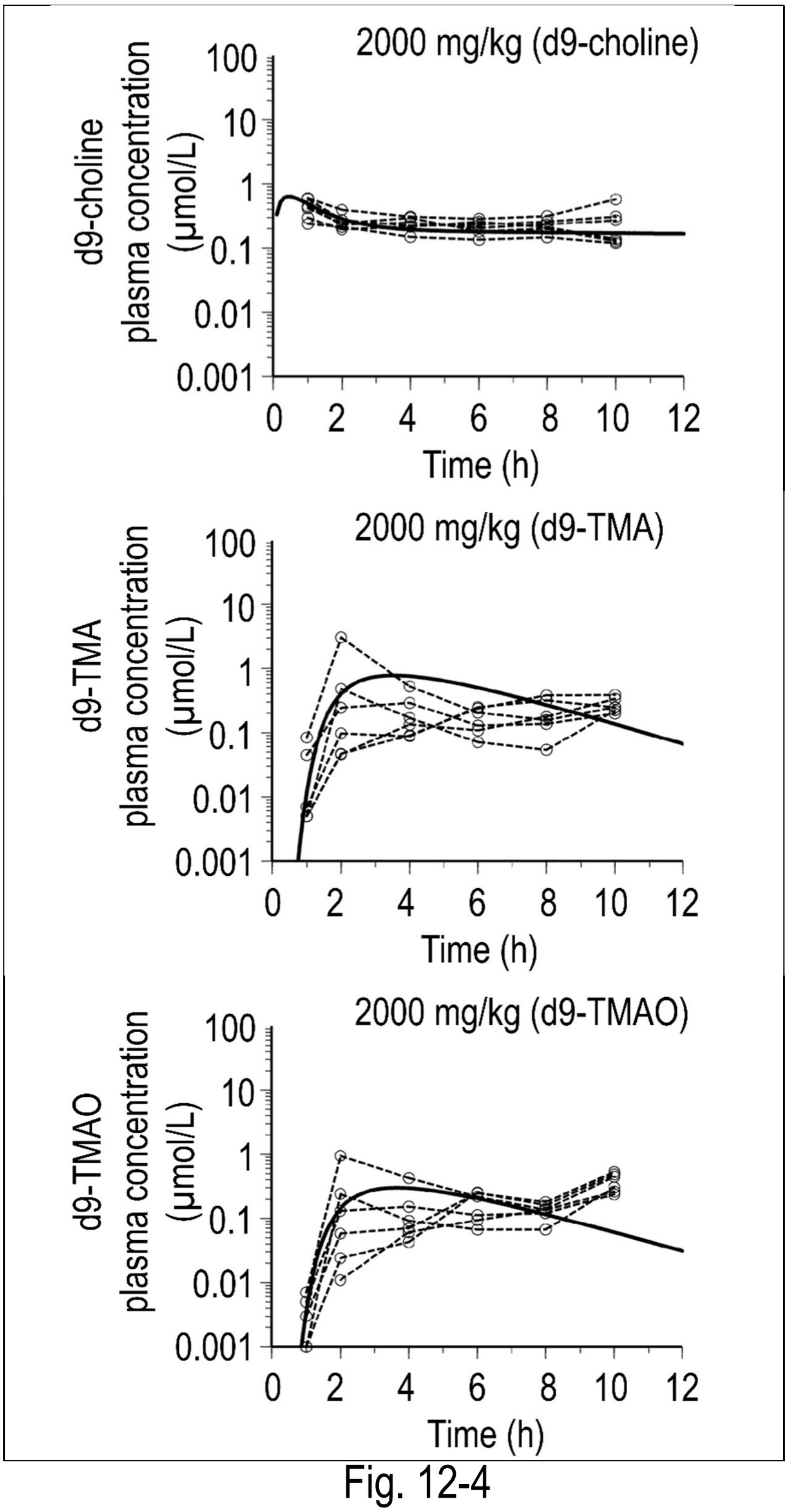
2000 mg/kg (d9-choline)
d9-choline plasma concentration (µmol/L)
100
10
1
0.1
0.01
0.001
0
2
4
6
8
10
12
Time (h)
2000 mg/kg (d9-TMA)
d9-TMA plasma concentration (µmol/L)
100
10
1
0.1
0.01
0.001
0
2
4
6
8
10
12
Time (h)
2000 mg/kg (d9-TMAO)
d9-TMAO plasma concentration (µmol/L)
100
10
1
0.1
0.01
0.001
0
2
4
6
8
10
12
Time (h)

Fig. 12-4

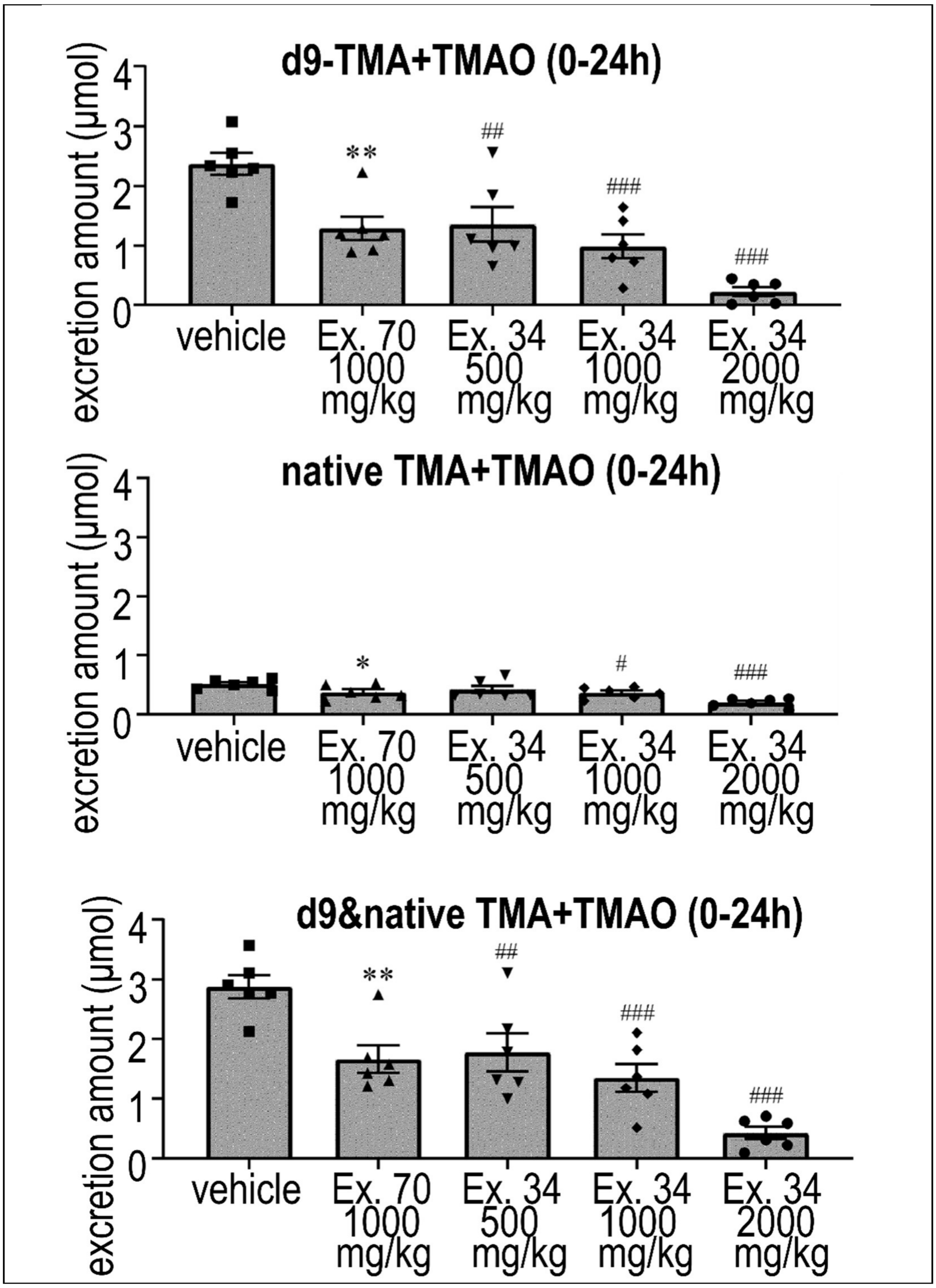
d9-TMA+TMAO (0-24h)
excretion amount (µmol)
4
3
2
1
0
**
##
###
###
vehicle
Ex. 70 1000 mg/kg
Ex. 34 500 mg/kg
Ex. 34 1000 mg/kg
Ex. 34 2000 mg/kg
native TMA+TMAO (0-24h)
excretion amount (µmol)
4
3
2
1
0
*
#
###
vehicle
Ex. 70 1000 mg/kg
Ex. 34 500 mg/kg
Ex. 34 1000 mg/kg
Ex. 34 2000 mg/kg
d9&native TMA+TMAO (0-24h)
excretion amount (µmol)
4
3
2
1
0
**
##
###
###
vehicle
Ex. 70 1000 mg/kg
Ex. 34 500 mg/kg
Ex. 34 1000 mg/kg
Ex. 34 2000 mg/kg

Fig. 13

## INTERNATIONAL SEARCH REPORT

International application No.
**PCT/JP2024/023237**

### A. CLASSIFICATION OF SUBJECT MATTER

***A61K 33/06***(2006.01)i; ***A61P 1/04***(2006.01)i; ***A61P 1/16***(2006.01)i; ***A61P 3/04***(2006.01)i; ***A61P 3/10***(2006.01)i; ***A61P 9/00***(2006.01)i; ***A61P 9/04***(2006.01)i; ***A61P 13/12***(2006.01)i; ***A61P 15/00***(2006.01)i; ***A61P 25/16***(2006.01)i; ***A61P 25/28***(2006.01)i; ***A61P 27/06***(2006.01)i; ***A61P 35/00***(2006.01)i

FI: A61K33/06; A61P1/16; A61P1/04; A61P9/00; A61P9/04; A61P27/06; A61P25/28; A61P25/16; A61P3/04; A61P3/10; A61P13/12; A61P15/00; A61P35/00

According to International Patent Classification (IPC) or to both national classification and IPC

### B. FIELDS SEARCHED

Minimum documentation searched (classification system followed by classification symbols)

A61K33/06; A61P1/04; A61P1/16; A61P3/04; A61P3/10; A61P9/00; A61P9/04; A61P13/12; A61P15/00; A61P25/16; A61P25/28; A61P27/06; A61P35/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2018/139047 A1 (NATIONAL INSTITUTE FOR MATERIALS SCIENCE) 02 August 2018 (2018-08-02)<br>paragraphs [0037], [0048], table 1, fig. 5 | 1-36 |
| Y | ISHIBASHI, M. et al. Application of Synthetic Hydrated Aluminum Silicates as Orally Administered Absorbents of Ammonium Ion. Chem. Pharm. Bull. 1986, vol. 34, no. 2, pp. 806-812<br>abstract, conclusion | 1-36 |
| Y | JP 2019-99451 A (JGC CATALYSTS & CHEMICALS LTD.) 24 June 2019 (2019-06-24)<br>paragraph [0004] | 21-25, 32-36 |
| A | US 2005/0106267 A1 (FRAMEWORK THERAPEUTICS, LLC) 19 May 2005 (2005-05-19)<br>entire text | 36 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2024** | **17 September 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/023237**

**C. DOCUMENTS CONSIDERED TO BE RELEVANT**

| Category* | Citation of document, with indication, where appropriate, of the relevant passages | Relevant to claim No. |
|---|---|---|
| A | US 2018/0200291 A1 (NEUROSCIENCE, INC.) 19 July 2018 (2018-07-19)<br>entire text | 1-36 |
| P, X | 引問脩ほか, トリメチルアミン尿症治療を目的とした世界初のゼオライト経口薬の創薬, ゼオライト研究発表会講演予稿集, November 2023, vol. 39, p. 81, (HIKIMA, Shu et al.), non-official translation (Drug discovery of the world's first oral medicine of zeolite for the treatment of trimethylammonuria. Lecture Preprints of Zeolite Workshops Japan.)<br>abstract | 1-36 |

Form PCT/ISA/210 (second sheet) (July 2022)

**INTERNATIONAL SEARCH REPORT**
**Information on patent family members**

International application No.
**PCT/JP2024/023237**

| Patent document cited in search report | Publication date (day/month/year) | Patent family member(s) | Publication date (day/month/year) |
|---|---|---|---|
| WO 2018/139047 A1 | 02 August 2018 | (Family: none) | |
| JP 2019-99451 A | 24 June 2019 | (Family: none) | |
| US 2005/0106267 A1 | 19 May 2005 | WO 2005/041657 A1<br>EP 1679962 A1 | |
| US 2018/0200291 A1 | 19 July 2018 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20180200291


### Non-patent literature cited in the description


- Comparison of the Solubility and Dissolution of Drugs in Fasted-State Biorelevant Media (FaSSIF and FaSSIF-V2). *Dissolution Technologies*, vol. 20 (3), 44-50
- Trimethylaminuria. *European Journal of Human Genetics*, 2015, vol. 23, 1269
- Cardiovascular disease. *Toxins*, 2019, vol. 11 (9), 490
- Glaucoma. *International Ophthalmology*, 2021, vol. 41, 341-347
- Atherosclerosis. *Nature Medicine*, 2013, vol. 19, 576-585
- Coronary artery heart disease. *BMC Cardiovascular Disorders*, 2020, vol. 20, 7
- Heart failure with preserved ejection fraction. *BMC Cardiovascular Disorders*, 2020, vol. 20, 394
- ST-elevation myocardial infarction. *American Journal of Cardiology*, 15 March 2019, vol. 123 (6), 894-898
- Abdominal aortic aneurysm. *Circulation*, 04 April 2023, vol. 147 (14), 1079-1096
- Atrial fibrillation. *International Journal of Cardiology*, 15 September 2018, vol. 267, 100-106
- Ischemic stroke. *Journal of Biochemical and Molecular Toxicology*, February 2019, vol. 33 (2), 22246
- Post-stroke cognitive impairment. *Neurological Sciences*, 2020, vol. 41, 57-63
- Mild cognitive impairment and Alzheimer's disease. *Alzheimer's Research & Therapy*, 2018, vol. 10, 124
- Obesity. *Obesity Reviews*, 2020, vol. 21 (5), 12993
- Progressive chronic kidney disease with type 2 diabetes. *Journal of Clinical Medicine*, 2017, vol. 6 (9), 86
- Cardiovascular complications in chronic kidney disease. *Kidney International*, October 2017, vol. 92 (4), 809-815
- Diabetic retinopathy. *Acta Diabetologica*, 2021, vol. 58, 221-229
- Non-alcoholic steatohepatitis. *Diabetes & Metabolism*, 2021, vol. 47, 101183
- Polycystic ovary syndrome. *BMC Endocr Disord*, 2020, vol. 20, 3
- Parkinson's disease. *Clinica Chimica Acta*, February 2020, vol. 501, 165-173
- Colorectal cancer. *International Journal of Molecular Sciences*, 2020, vol. 21, 6782
- *Science Translational Medicine*, 2022, vol. 14, 1895
- *Pediatric Nephrology*, 2012, vol. 27, 207-222
- *Dissolution Technologies*, vol. 20 (3), 44-50
- *SCIENCE*, 2003, vol. 300 (5618), 456-460
- *J. Am. Chem. Soc.*, 2023, vol. 145, 9021-9028
- **SHIMIZU, M. et al.** Human plasma concentrations of trimethylamine N-oxide extrapolated using pharmacokinetic modeling based on metabolic profiles of deuterium-labeled trimethylamine in humanized-liver mice. *J. Toxicol. Sci*, 2018, vol. 43, 387-393
- **YAMASHITA, M et al.** Human plasma concentrations of herbicidal carbamate molinate extrapolated from the pharmacokinetics established in in vivo experiments with chimeric mice with humanized liver and physiologically based pharmacokinetic modeling. *Regulatory Toxicology and Pharmacology*, 2014, vol. 70, 214-221
- **AL-WAIZ M** ; **MITCHELL SC** ; **IDLE JR** ; **SMITH RL**. The metabolism of 14C-labelled trimethylamine and its N-oxide in man. *Xenobiotica*, 1987, vol. 17 (5), 551-558
- **AL-WAIZ M** ; **MITCHELL SC**. The fate of trimethylamine in the rat. *Drug Metab and Drug Interact*, 1991, vol. 9 (1), 41-48
- **FENNEMA D** ; **PHILLIPS IR** ; **SHEPHARD EA**. Trimethylamine and Trimethylamine N-Oxide, a Flavin-Containing Monooxygenase 3 (FMO3)-Mediated Host-Microbiome Metabolic Axis Implicated in Health and Disease. *Drug Metab Dis*, 2016, vol. 44, 1839-1850
- **MICHELL SC** ; **SMITH RL**. Trimethylaminuria. *The Fish Malodor Syndrome. Drug Metab Dis*, 2001, vol. 29, 517-521

- **SHIMIZU M** ; **ALLERSTON CK** ; **SHEPHARD EA** ; **YAMAZAKI H** ; **PHILLIPS IR**. Relationships between flavin-containing mono-oxygenase 3 (FMO3) genotype and trimethylaminuria phenotype in a Japanese population. *Br J Clin Pharmacol*, 2013, vol. 77 (5), 839-851